# EUROPÄISCHE PATENTSCHRIFT

(11) **EP 1 534 227 B1**
(45) Veröffentlichungstag und Bekanntmachung des Hinweises auf die Patenterteilung: **27.12.2006**
(21) Anmeldenummer: 03793751.3
(22) Anmeldetag: 23.08.2003
(51) Int. Cl.: A61Q 5/10, A61K 8/49, A61K 8/35

(54) **MITTEL ZUM FÄRBEN VON KERATINHALTIGEN FASERN**
AGENTS USED FOR DYEING KERATINOUS FIBERS
PRODUITS POUR LA TEINTURE DE FIBRES KERATINIQUES

(30) Priorität: 05.09.2002 DE 10241076
(43) Veröffentlichungstag der Anmeldung: 01.06.2005
(73) Patentinhaber: Henkel Kommanditgesellschaft auf Aktien, 40589 Düsseldorf (DE)
(72) Erfinder: GROSS, Wibke, 40549 Düsseldorf (DE); HÖFFKES, Horst, 40595 Düsseldorf (DE); OBERKOBUSCH, Doris, 40591 Düsseldorf (DE); MAUSBERG, Sandra, 40699 Erkrath (DE)
(86) Internationale Anmeldenummer: PCT/EP2003/009366
(87) Internationale Veröffentlichungsnummer: WO 2004/022016

(56) Entgegenhaltungen:
- EP-A- 0 873 745
- WO-A-95/15144
- DE-A- 2 047 431
- DE-A- 10 148 847
- FR-A- 2 787 707
- GB-A- 1 386 269
- US-A- 3 574 200
- US-A- 5 279 616
- US-A- 5 725 607
- HANS BAUMANN ET AL : "Reaktionen der Methylenbasen von Oxazolidinonen und Pyrimidonen" JUSTUS LIEBIGS ANNALEN DER CHEMIE., Bd. 717, 1968, Seiten 124-136, XP002265004 VERLAG CHEMIE GMBH. WEINHEIM., DE ISSN: 0075-4617
- CHEMICAL ABSTRACTS, Bd. 98, Nr. 73809, 1983, Columbus, Ohio, US; abstract no.: 1983:73809, V.A. CHUIGUK ET AL: "1,3-Diaryl-1,2-dihydro-2-oxopyrimidinium salts and methine dyes derived from them"

## Beschreibung

Die Erfindung betrifft ein Mittel zum Färben von keratinhaltigen Fasern, insbesondere menschlichen Haaren, das 1,2-Dihydro-pyrimidinium-Derivate in Kombination mit reaktiven Carbonylverbindungen enthält, die Verwendung dieser Kombination in Mitteln zum Färben von keratinhaltigen Fasern, zur Farbauffrischung bzw. Nuancierung von bereits gefärbten keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy- oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridinderivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Problematisch gestaltet sich nach wie vor eine Bereitstellung von Oxidationshaarfärbungen im Rotbereich mit ausreichenden Echtheitseigenschaften, insbesondere in mit sehr guten Wasch- und Reibechtheiten. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbemittel, enthaltend 1,2-Dihydro-pyrimidinium-Derivate sowie deren Verwendung zum Färben von keratinhaltigen Fasern oder zur Farbauffrischung bzw. Nuancierung von bereits gefärbten keratinhaltigen Fasern sind bislang nicht bekannt.

In der Veröffentlichung H. Baumann et al., *Liebigs Ann. Chem.,* **1968,** 717, 124-136, werden Reaktionen von Pyrimidonen als Methylenbasen beschrieben. Ein Haarfärbemittel, enthaltend 1,2-Dihydro-pyrimidinium-Derivate, oder die Verwendung der offenbarten Hemicyanine zum Färben von keratinhaltigen fasern wird nicht vorgeschlagen.

In der deutschen Patentanmeldung DE-A1-2047431 werden kationische Methinfarbstoffe zur Färbung von anionisch modifizierten Fasern, wie sauer modifizierten Polyestem oder Acrylnitrilpolymerisaten beschrieben. Zur Darstellung der kationischen Methinfarbstoffe werden unter anderem 3,4-Dihydro-3-methyl-4-methylenchinazol-2-on und 1,3,6-Trimethyl-4-methylen-pyrimidin-2-on sowie zwingend Terephthalaldehyd verwendet.

In der deutschen Patentanmeldung DE-A1-2165913 wird ein Verfahren zur Herstellung von Ausbleichbildern unter Verwendung von lichtempfindlichen Farbstoffen vorgeschlagen. Die beanspruchten lichtempfindlichen Farbstoffe gehören zu der Klasse der Pyrimidon- bzw. Thiopyrimidonfarbstoffe.

In der Druckschrift Ukrainskii Khimichsekii Zhurnal, 1982, 48(11), 1220-1223 wird die Synthese von Polymethinfarbstoffen, wie 4-[2-[4-(Dimethylamino)phenyl]ethenyl]-2,3-dihydro-6-methyl-2-oxo-1,3-diphenylpyrimidiniumperchlorat und 4,6-Bis[2-[4-(Dimethylamino)phenyl]ethenyl]-2,3-dihydro-2-oxo-1,3-diphenylpyrimidiniumperchlorat, ausgehend von 2,3-Dihydro-4,6-dimethyl-2-oxo-1,3-diphenylpyrimidinium-Salzen beschrieben.

Die Druckschrift FR-A-2 787 707 betrifft Haarfärbemittel, in denen kationische, zyklische Verbindungen in Kombination mit reaktiven Carbonylverbidungen enthalten sind.

Die Synthese von 2-Phenoxazinylethenyl-substituierten Farbstoffen ausgehend von Methylen-substituierten Stickstoffheterozyklen wie z.B. 1,3,6-Trimethyl-4-methylen-2-oxopyrimidon und Phenoxazinaldehyden kann dem Dokument US-A-3,574,200 entnommen werden.

Aufgabe der vorliegenden Erfindung ist es, Färbemittel für keratinhaltige Fasern, insbesondere menschliche Haare, bereitzustellen, die hinsichtlich der Farbtiefe, der Grauabdeckung und den Echtheitseigenschaften, wie beispielsweise Licht-, Reib- und Waschechtheit sowie Schweiß- und Kaltitvellechtheit, insbesondere im Rotbereich, qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten 1,2-Dihydro-pyrimidinium-Derivate, insbesondere in Kombination mit Verbindungen enthaltend mindestens eine reaktive Carbonylgruppe, sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Es werden insbesondere Ausfärbungen mit verbesserten Echtheitseigenschaften über einen Nuancenbereich von gelb über gelbbraun, orange, braunorange, braun, rot, rotviolett bis hin zu blauviolett und schwarz erhalten. Der Einsatz von oxidierenden Agentien soll jedoch nicht prinzipiell ausgeschlossen werden.

Gegenstand der Erfindung ist ein Mittel zum Färben von keratinhaltigen Fasern, insbesondere menschlichen Haaren, enthaltend mindestens ein 1,2-Dihydrö-pyrimidinium-Derivat gemäß Formel I und/oder dessen Enaminform, 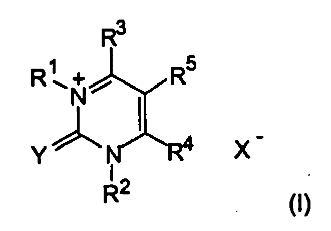 wobei
- R¹ und R² stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₘ-, worin R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe, wobei R^{I} und R^{II} gemeinsam mit dem Stickstoffatom einen 5-, 6- oder 7-gliedrigen Ring bilden können und m steht für eine Zahl 2, 3, 4, 5 oder 6,
- R³ und R⁴ stehen unabhängig voneinander für ein Wasserstoffatom oder eine C,-C₆-Alkylgruppe, wobei mindestens einer der Reste R³ und R⁴ eine C₁-C₆-Alkylgruppe bedeutet,
- R⁵ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}R^{IV}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R⁵ zusammen mit einem der Reste R³ oder R⁴ einen 5- oder 6-gliedrigen aromatischen oder aliphatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe R^{V}W^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
- Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
- X- steht für Halogenid, Benzolsulfonat, p-Toluolsulfonat, C₁-C₄-Alkansulfonat, Trifluormethansulfonat, Perchlorat, 0.5 Sulfat, Hydrogensulfat, Tetrafluoroborat, Hexafluorophosphat oder Tetrachlorozinkat,
und mindestens eine Verbindung mit einer reaktiven Carbonylgruppe (Komponente B).

In einer bevorzugten Ausführungsform steht Y für ein Sauerstoff- oder ein Schwefelatom, besonders bevorzugt für ein Sauerstoffatom.

Bevorzugt steht X- für ein Halogenid, insbesondere Chlorid, Bromid oder lodid, p-Toluolsulfonat, Tetrafluoroborat, Trifluormethansulfonat, Hexafluorophosphat, 0.5 Sulfat, oder Hydrogensulfat. Besonders bevorzugt werden die Anionen Chlorid, Bromid, lodid, Hydrogensulfat oder p-Toluolsulfonat als X- eingesetzt.

R⁵ steht bevorzugt für ein Wasserstoffatom.

Mindestens eine Gruppe R³ oder R⁴ gemäß Formel I steht für eine C₁-C₆-Alkylgruppe. Diese Alkylgruppe trägt an deren α-Kohlenstoffatom bevorzugt mindestens zwei Wasserstoffatome. Besonders bevorzugte Alkylgruppen sind die Methyl-, Ethyl-, Propyl-, n-Butyl-, iso-Butyl, n-Pentyl-, neo-Pentyl-, n-Hexylgruppe. Ganz besonders bevorzugt stehen R³ und R⁴ unabhängig voneinander für Wasserstoff oder eine Methylgruppe, wobei mindestens eine Gruppe R³ oder R⁴ eine Methylgruppe bedeutet.

Vorzugsweise sind die Verbindungen gemäß Formel I ausgewählt aus der Gruppe bestehend aus 1,2-Dihydro-1,3,4,6-tetramethyl-2-oxo-pyrimidinium-chlorid, 1,2-Dihydro-1,3-diethyl-4,6-dimethyl-2-oxo-pyrimidinium-chlorid, 1,2-Dihydro-1,3-dipropyl-4,6-dimethyl-2-oxo-pyrimidinium-chlorid, 1,2-Dihydro-1,3-di(2-hydroxyethyl)-4,6-dimethyl-2-oxo-pyrimidinium-chlorid, 1,2-Dihydro-1,3-diphenyl-4,6-dimethyl-2-oxo-pyrimidinium-chlorid, 1,2-Dihydro-1,3,4,6-tetramethyl-2-oxo-pyrimidinium-hydrogensulfat, 1,2-Dihydro-1,3-diethyl-4,6-dimethyl-2-oxo-pyrimidinium-hydrogensulfat, 1,2-Dihydro-1,3-dipropyl-4,6-dimethyl-2-oxo-pyrimidinium-hydrogensulfat, 1,2-Dihydro-1,3-di(2-hydroxyethyl)-4,8-dimethyl-2-oxo-pyrimidinium-hydrogensulfat, 1,2-Dihydro-l,3-diphenyl-4,6-dimethyl-2-oxo-pyrimidinium-hydrogensulfat, 1,2-Dihydro-1,3,4-trimethyl-2-oxo-pyrimidinium-chlorid, 1,2-Dihydro-1,3,4-trimethyl-2-oxo-pyrimidinium-hydrogensulfat, 1,2-Dihydro-1,3-diethyl-4-methyl-2-oxo-pyrimidinium-chlorid, 1,2-Dihydro-1,3-diethyl-4-rnethyl-2-oxopyrimidinium-hydrogensulfat, 1,2-Dihydro-1,3-dipropyl-4-methyl-2-oxo-pyrimidinium-chlorid, 1,2-Dihydro-1,3-dipropyl-4-methyl-2-oxo-pyrimidinium-hydrogensulfat, 1,2-Dihydro-1,3-di(2-hydroxyethyl)-4-methyl-2-oxo-pyrimidinium-chlorid, 1,2-Dihydro-1,3-di(2-hydroxyethyl)-4-methyl-2-oxo-pyrimidinium-hydrogensulfat, 1,2-Dihydro-1,3-diphenyl-4-methyl-2-oxo-pyrimidinium-chlorid, 1,2-Dihydro-1,3-diphenyl-4-methyl-2-oxopyrimidinium-hydrogensulfat, 1,2-Dihydro-1,3,4,5,6-pentamethyl-2-oxo-pyrimidinium-chlorid, 1,2-Dihydro-1,3,4,6-tetramethyl-2-thioxo-pyrimidinium-chlorid, 1,2-Dihydro-1,3-diethyl-4,6-dimethyl-2-thioxo-pyrimidinium-chlorid, 1,2-Dihydro-1,3-dipropyl-4,6-dimethyl-2-thioxo-pyrimidinium-chlorid, 1,2-Dihydro-1,3-di(2-hydroxyethyl)-4,6-dimethyl-2-thioxopyrimidinium-chlorid, 1,2-Dihydro-1,3-diphenyl-4,6-dimethyl-2-thioxo-pyrimidinium-chlorid, 1,2-Dihydro-1,3,4,6-tetramethyl-2-thioxo-pyrimidinium-hydrogensulfat, 1,2-Dihydro-1,3-dipropyl-4,6-dimethyl-2-thioxo-pyrimidinium-hydrogensulfat, 1,2-Dihydro-1,3-di(2-hydroxyethyl)-4,6-dimethyl-2-thioxo-pyrimidinium-hydrogensulfat, 1,2-Dihydro-1,3-diphenyl-4,6-dimethyl-2-thioxo-pyrimidinium-hydrogensulfat, 1,2-Dihydro-1,3,4-trimethyl-2-thioxo-pyrimidinium-chlorid, 1,2-Dihydro-1,3,4-trimethyl-2-thioxo-pyrimidinium-hydrogensulfat, 1,2-Dihydro-1,3-diethyl-4-methyl-2-thioxo-pyrimidinium-chlorid, 1,2-Dihydro-1,3-diethyl-4-methyl-2-thioxo-pyrimidinium-hydrogensulfat, 1,2-Dihydro-1,3-dipropyl-4-methyl-2-thioxo-pyrimidinium-chlorid, 1,2-Dihydro-1,3-dipropyl-4-methyl-2-thioxo-pyrimidinium-hydrogensulfat, 1,2-Dihydro-1,3-di(2-hydroxyethyl)-4-methyl-2-thioxo-pyrimidinium-chlorid, 1,2-Dihydro-1,3-di(2-hydroxyethyl)-4-methyl-2-thioxopyrimidinium-hydrogensulfat, 1,2-Dihydro-1,3-diphenyl-4-methyl-2-thioxo-pyrimidinium-chlorid, 1,2-Dihydro-1,3-diphenyl-4-methyl-2-thioxo-pyrimidinium-hydrogensulfat, 1,2-Dihydro-3,4-dimethyl-2-oxo-chinazoliniumchlorid, 1,2-Dihydro-3,4-dimethyl-2-oxo-chinazolinium-p-toluolsulfonat, 1,2-Dihydro-3,4-dimethyl-2-thioxo-chinazoliniumchlorid, 1,2-Dihydro-3,4-dimethyl-2-thioxo-chinazolinium-p-toluolsulfonat, 1,2-Dihydro-1,3,4-trimethyl-2-thioxo-chinazolinium-hydrogensulfat, 1,3,4-trimethyl-2-oxo-2,3,b,8,7,8-hexahydrochinazolinium-hydrogensulfat, 1,3,4-Trimethyl-2-thioxo-2,3,5,6,7,8-hexahydrochinazolinium-hydrogensulfat, 1,3,4-Trimethyl-2-oxo-3,5,6,7-tetrahydro-2H-cyclopenta[a]pyrimidinium-hydrogensulfat und 1,3,4-Trimethyl-2-thioxo-3,5,6,7-tetrahydro-2H-cyclopenta[a]pyrimidinium-hydrogensulfat.

Ganz besonders bevorzugt werden die erfindungsgemäßen Verbindungen gemäß Formel I der Komponente A ausgewählt aus 1,2-Dihydro-1,3,4,6-tetramethyl-2-oxopyrimidinium-chlorid, 1,2-Dihydro-1,3,4,6-tetramethyl-2-oxo-pyrimidinium-hydrogensulfat, 1,2-Dihydro-1,3,4-trimethyl-2-oxo-pyrimidinium-chlorid, 1,2-Dihydro-4,6-dimethyl-1,3-dipropyl-2-oxo-pyrimidinium-chlorid, 1,2-Dihydro-1,3,4,6-tetramethyl-2-thioxopyrimidinium-hydrogensulfat und 2-Dihydro-1,3,4,5,6-pentamethyl-2-oxo-pyrimidinium-chlorid.

Im folgenden sollen Beispiele für die als Substituenten im Rahmen dieser Anmeldung genannten Gruppen bzw. Reste erwähnt werden. Beispiele für C₁-C₆-Alkylreste sind die Gruppen Methyl, Ethyl, Propyl, Isopropyl, n-Butyl, sec-Butyl und tert.-Butyl, n-Pentyl und n-Hexyl. Propyl, Ethyl und Methyl sind bevorzugte Alkylreste. Beispiele für entsprechende cyclische Alkylgruppen sind Cyclopentyl und Cyclohexyl. Beispiele für bevorzugte C₂-C₆-Alkenylreste sind Vinyl und -Allyl. Erfindungsgemäß bevorzugte C₁-C₆-Alkoxyreste sind beispielsweise eine Methoxy- oder eine Ethoxygruppe. Die Methoxycarbonyl-, Ethoxycarbonyl-, n-Propoxycarbonyl-, Isopropoxycarbonyl-, n-Butoxycarbonyl-, sec-Butoxycarbonyl- und tert-Butoxycarbonylgruppe sind Beispiele für C₁-C₄-Alkoxycarbonylgruppen; die Methoxycarbonyl- und die Ethoxycarbonylgruppe sind dabei besonders bevorzugt. Weiterhin können als bevorzugte Beispiele für eine C₁-C₆-Hydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyethylgruppe genannt werden. Eine 2-Hydroxyethylgruppe ist besonders bevorzugt. Die Methoxyethyl-, Ethoxyethyl-, Methoxypropyl-, Methoxybutyl-, Ethoxybutyl- und die Methoxyhexylgruppe sind Beispiele für erfindungsgemäße C₁-C₆-Alkoxy-C₁-C₆-alkylgruppen. Beispiele für eine C₂-C₆-Polyhydroxyalkylgruppe sind die 2,3-Dihydroxypropylgruppe, 3,4-Dihydroxybutylgruppe und die 2,4-Dihydroxybutylgruppe. Eine bevorzugte Hydroxy-C₁-C₆-alkoxygruppe ist die 2-Hydroxyethoxygruppe. Bevorzugte Arylgruppen sind Phenyl, Naphthyl und Biphenyl. Beispiele für Halogenatome sind F-, Cl-, Br- oder I-Atome, wobei CI-Atome ganz besonders bevorzugt sind. Bevorzugte C₁-C₆-Aminoalkylgruppen sind die Aminomethyl-, die Aminoethyl und die Aminopropylgruppe. Bevorzugte Aryl-C₁-C₆-alkylgruppen sind Benzyl und 2-Phenylethyl. Die Aminomethyl-, 2-Aminoethyl-, 3-Aminopropyl-, 2-Dimethylaminoethyl-, Diethylaminomethyl-, Dimethylaminomethyl, 2-Methylaminoethyl-, Dimethylamino-, Piperidinomethyl-, Pyrrolidinomethyl, Morpholinomethyl- und die Aminogruppe sind Beispiele für eine Gruppe R'R"N-(CH₂)ₙ-, wobei die Diethylaminomethyl-, Piperidinomethyl, 2-Dimethylaminoethyl-, Dimethylamino- und die Aminogruppe besonders bevorzugt sind. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Beispiele für eine Heterozyklus-C₁₋₄-alkylgruppe sind Pyrrolidino-(C₁₋₄)-alkyl, Piperidino-(C₁₋₄)-alkyl, Morpholino-(C₁₋₄)-alkyl, 2-Furyl-(C₁₋₄)-alkyl, 2-Thienyl-(C₁₋₄)-alkyl, 4-Pyridyl-(C₁₋₄)-alkyl, 3-Pyridyl-(C₁₋₄)-alkyl, 2-Pyridyl-(C₁₋₄)-alkyl, Triazolyl-(C₁₋₄)-alkyl und 1-Imidazolyl-(C₁₋₄)-alkyl. Eine bevorzugte C₁₋₄-Carboxyalkylgruppe ist die 3-Carboxypropylgruppe. Besonders bevorzugte C₂-C₆-Alkenylengruppen sind Vinylen und Propylen. Eine besonders bevorzugte C₄-C₆-Alkadienylengruppe ist die 1,3-Butadien-1,4-diylgruppe. Die Gruppen 1-Carboxypropylen und 1-Carboxyethylen sind bevorzugte Carboxy-(C₁-C₄)-alkylengruppen. Die weiteren verwendeten Begriffe leiten sich erfindungsgemäß von den hier gegebenen Definitionen ab.

Die erfindungsgemäßen 1,2-Dihydro-pyrimidinium-Derivate gemäß Formel I sind CHacide Verbindungen. Sie liegen im chemischen Gleichgewicht mit der Enaminform der 1,2-Dihydro-pyrimidinium-Derivate gemäß Formel I vor. Mit Hilfe einer Base lassen sich aus den Verbindungen gemäß Formel I durch Deprotonierung am α-Kohlenstoffatom der C₁-C₆-Alkylreste R³ bzw. R⁴ die korrespondierenden Enamine gezielt darstellen. Exemplarisch wird diese Deprotonierung nachfolgend illustriert, wobei zur Verdeutlichung R³ als Rest R-CH₂- gewählt wurde. Eine Verbindung gemäß der Formel la ist ein Beispiel für eine erfindungsgemäße Enaminform der 1,2-Dihydro-pyrimidinium-Derivate.

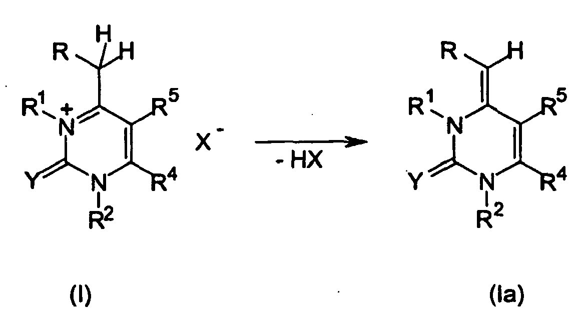

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl- oder Acetylcellulose verwendet werden.

1,2-Dihydro-pyrimidinium-Derivate der Formel I sind zum großen Teil literaturbekannt, im Handel erhältlich oder nach bekannten Syntheseverfahren nach D. Lloyd et al., *J. Chem. Soc. Perkin Trans I,* **1977,** 16, 1862-1869; S. T. Oswald et al., *J. Heterocycl. Chem.,* **1974,** *11(3),* 441-443; H. Baumann et al., *Liebigs Ann. Chem.,* **1968,** 717, 124-136 und V. A. Chuiguk, *Ukr. Khim. Zh. (Russ Ed.),* **1982,** *48(11),* 1220-1223 herstellbar.

Färbungen mit erhöhter Brillanz und verbesserten Echtheitseigenschaften (Lichtechtheit, Waschechtheit, Reibechtheit) über einen weiten Nuancenbereich werden erzielt, wenn die eingesetzten Verbindungen der Formel I erfindungsgemäß gemeinsam mit mindestens einer Substanz mit einer reaktiven Carbonylgruppe (im folgenden Komponente B oder reaktive Carbonylverbindung genannt) in den erfindungsgemäßen Mitteln enthalten sind. Erfindungsgemäße reaktive Carbonylverbindungen besitzen mindestens eine Carbonylgruppe als reaktive Gruppe, welche mit der CH-aciden Verbindung gemäß Formel I unter Ausbildung einer Kohlenstoff-Kohlenstoff-Bindung reagiert. Ferner sind erfindungsgemäß auch solche Verbindungen als Komponente B verwendbar, in denen die reaktive Carbonylgruppe derart derivatisiert ist, daß die Reaktivität des Kohlenstoffatoms der derivatisierten Carbonylgruppe gegenüber den CH-aciden Verbindungen der Formel I stets vorhanden ist. Diese Derivate sind bevorzugt Additionsverbindungen von
a) Aminen und deren Derivate unter Bildung von Iminen oder Oximen als Additionsverbindung
b) von Alkoholen unter Bildung von Acetalen oder Ketalen als Additionsverbindung
an das Kohlenstoffatom der Carbonylgruppe der reaktiven Carbonylverbindung.

Die Komponente B ist bevorzugt ausgewählt aus Verbindungen gemäß Formel II, 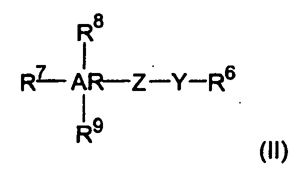 wobei
- AR steht für Benzol, Naphthalin, Pyridin, Pyrimidin, Pyrazin, Pyridazin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
- R⁶ steht für ein Wasserstoffatom, eine C₁-C₆-Alkyl-, C₂-C₆-Acyl-, C₂-C₄-Alkenyl-, C₁-C₄-Perfluoralkyl-, eine ggf. substituierte Aryl- oder Heteroarylgruppe,
- R⁷, R⁸ und R⁹ stehen unabhängig voneinander für ein Wasserstoffatom, ein Halogenatom, eine C₁-C₆-Alkyl-, C₁-C₆-Alkoxy-, C₁-C₆-Aminoalkyl-, C₁-C₆-Hydroxyalkylgruppe, eine C₁-C₆-Alkoxy-C₁-C₆-alkyloxygruppe, eine C₂-C₆-Acylgruppe, eine Acetyl-, Carboxyl-, Carboxylato-, Carbamoyl-, Sulfo-, Sulfato-, Sulfonamid-, Sulfonamido-, C₂-C₆-Alkenyl-, eine Aryl-, eine Aryl-C₁-C₆-alkylgruppe, eine Hydroxy-, eine Nitro-, eine Pyrrolidino-, eine Morpholino-, eine Piperidino-, eine Amino- bzw. Ammonio- oder eine 1-lmidazol(in)iogruppe, wobei die letzten drei Gruppen mit einer oder mehrerer C₁-C₆-Alkyl-, C₁-C₆-Carboxyalkyl-, C₁-C₆-Hydroxyalkyl-, C₂-C₆-Alkenyl-, C₁-C₆-Alkoxy-C₁-C₆-alkyl-, mit ggf. substituierten Benzylgruppen, mit Sulfo-(C₁-C₄)-alkyl- oder Heterozyklus-(C₁-C₄)-alkylgruppen substituiert sein können,
wobei auch zwei der Reste aus R⁷, R⁸, R⁹ und -Z-Y-R⁶ zusammen mit dem Restmolekül einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R⁷, R⁸ und R⁹,
- Z steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vnylenfurylen-, Vinylenthienylengruppe, wobei Z zusammen mit der -Y-R⁶-Gruppe auch einen gegebenenfalls substituierten 5-, 6- oder 7-Ring bilden kann,
- Y steht für eine Gruppe, die ausgewählt ist aus Carbonyl, einer Gruppe gemäß Formel III und einer Gruppe gemäß Formel IV, 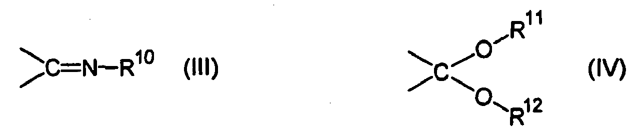 wobei
   - R¹⁰ steht für ein Wasserstoffatom, eine Hydroxygruppe, eine C₁-C₄-Alkoxygruppe, eine C₁-C₆-Alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe,
   - R¹¹ und R¹² stehen unabängig voneinander für eine C₁-C₆-Alkylgruppe, eine Arylgruppe oder bilden zusammen mit dem Strukturelement O-C-O der Formel IV einen 5- oder 6-gliederigen Ring.

Die Komponente B wird besonders bevorzugt ausgewählt aus der Gruppe bestehend aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxyacetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxybenzaldehyd, 4-Hydroxy-2,6-dimethoxy-benzaldehyd, 4-Hydroxy-2-rriethyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxybenzaldehyd, 4-Hydroxy-3,5-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxybenzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1 H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenrylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyctohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenryliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Forrnyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluolsulfonat, -methansulfonat, -perchlorat, -sulfat, -chlorid, -bromid, -iodid, -tetrachlorozinkat, -methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxyisatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

In einer speziellen Ausführungsform kann es günstig sein, die Komponente B derart auszuwählen, daß die Gruppe Y aus Formel II keine Carbonylgruppe ist. In diesem Fall kann es bevorzugt sein, die Komponente B auszuwählen, aus 2-{[(2-Hydroxyethyl)imino]methyl}phenol, 3-{[(2-Hydroxyethyl)imino]methyl}phenol, 4-{[(2-Hydroxyethyl)imino]methyl}phenol, 3-{[(2-Hydroxyethyl)imino]methyl}benzol-1,2-diol, 4-{[(2-Hydroxyethyl)imino]methyl}benzol-1,3-diol, 2-{[(2-Hydroxyethyl)imino]methyl}benzol-1,4-diol, 2-{[(2-Hydroxyethyl)imino]methyl}benzol-1,3-diol, 4-{[(2-Hydroxyethyl)imino]methyl}benzol-1,2-diol, 5-{[(2-Hydroxyethyl)imino]methyl}benzol-1,3-diol, 4-{[(2-Hydroxyethyl)imino]methyl}benzol-1,2,3-triol, 6-{[(2-Hydroxyethyl)imino]methyl}benzol-1,2,4-triol, 3-{[(2-Hydroxyethyl)imino]methyl}benzol-1,2,4-triol, 2-{[(2-Hydroxyethyl)imino]methyl}benzol-1,3,5-triol, 5-{[(2-Hydroxyethyl)imino]-methyl}benzol-1,2,4-triol, 3-{[(2-Hydroxyethyl)imino]methyl}benzole-1,2,4-triol, 2-{[(2-Methoxyphenyl)methylen]amino}ethanol, 2-{[(3-Methoxyphenyl)methylen]amino}ethanol, 2-{[(4-Methoxyphenyl)methylen]amino}ethanol, 2-{[(2-Ethoxyphenyl)methylen]-amino}ethanol, 2-{[(3-Ethoxyphenyl)methylen]amino}ethanol, 2-{[(4-Ethoxyphenyl)-methylen]amino}ethanol, 2-{[(2,3-Dimethoxyphenyl)methylen]amino}ethanol, 2-{[(2,4-Dimethoxyphenyl)methylen]amino}ethanol, 2-{[(2,5-Dimethoxyphenyl)methylen]-amino)ethanol, 2-{[(2,6-Dimethoxyphenyl)methylen]amino}ethanol, 2-{[(3,4-Dimethoxyphenyl)methylen]amino}ethanol, 2-{[(3,5-Dimethoxyphenyl)methylen]-amino}ethanol, 2-{[(2,3,4-Trimethoxyphenyl)methylen]amino}ethanol, 2-{[(2,3,5-Trimethoxyphenyl)methylen]amino}ethanol, 2-{[(2,3,6-Trimethoxyphenyl)methylen]-amino}ethanol, 2-{[(2,4,6-Trimethoxyphenyl)methylen]amino}ethanol, 2-{[(2,4,5-Trimethoxyphenyl)methylen]amino}ethanol, 2-{[(2;3,6-Trimethoxyphenyl)methylen]-amino)ethanol, 4-{[(2-Hydroxyethyl)imino]methyl}-3-methoxyphenol, 4-{[(2-Hydroxyethyl)imino]methyl}-2-methoxyphenol, 5-{[(2-Hydroxyethyl)imino]methyl}-2-methoxyphenol, 2-{[(2-Hydroxyethyl)imino]methyl}-5-methoxyphenol, 3-Ethoxy-4-{[(2-hydroxyethyl)imino]methyl)phenol, 2-Ethoxy-4-{[(2-hydroxyethyl)imino]methyl}phenol, 5-Ethoxy-2-{[(2-hydroxyethyl)imino]methyl}phenol, 2-Ethoicy-5-{[(2-hydroxyethyl)imino]-methyl}phenol, 4-{[(2-Hydroxyethyl)imino]methyl}-2,3-dimethoxyphenol, 4-{[(2-Hydroxyethyl)imino]methyl}-2,5-dimethoxyphenol, 4-{[(2-Hydroxyethyl)imino]methyl}-3,5-dimethoxyphenol, 4-{[(2-Hydroxyethyl)imino]methyl}-2,6-dimethoxyphenol, 2,6-Diethoxy-4-{[(2-hydroxyethyl)imino]methyl}phenol, 3,5-Diethoxy-4-{[(2-hydroxyethyl)imino]-methyl}phenol, 4-{[(2-Hydroxyethyl)imino]methyl}-3-methylphenol, 4-{[(2-Hydroxyethyl)imino]methyl}-2-methylphenol, 4-{[(2-Hydroxyethyl)imino]methyl}-2,3-dimethylphenol, 4-{[(2-Hydroxyethyl)imino]methyl}-2,5-dimethylphenol, 4-{[(2-Hydroxyethyl)imino]methyl}-3,5-dimethylphenol, 4-{[(2-Hydroxyethyl)irnino]methyl}-2,6-dimethylphenol, 2-({[4-(Dibutylamino)phenyl]methylen}amino)ethanol, 2-({[2-Chlor-4-(dimethylamino)phenyl]methylen}amino)ethanol, 2-({[4-(Dimethylamino)-2-methylphenyl]-methylen}amino)ethanol, 2-({[4-(Dimethylamino)-2-methoxyphenyl]methylen}-amino)ethanol, 2-({[2-(Dimethylamino)phenyl]methylen}amino)ethanol, 2-({[4-(Dimethylamino)phenyl]methylen}amino)ethanol, 2-({[4-(Diethylamino)phenyl]methylen}-amino)ethanol, 5-(Dimethylamino)-2-{[(2-hydroxyethyl)imino]methyl}phenol, 5-(Diethylamino)-2-{[(2-hydroxyethyl)imino]methyl}phenol, 2-{[(4-Pyrrolidin-1-ylphenyl)methylen]amino}ethanol, 2-{[(4-Piperidin-1-ylphenyl)methylen]amino}ethanol, 2-{[(4-Morpholin-4-ylphenyl)methylen]amino}ethanol, 2-{[(2-Morpholin-4-ylphenyl)methylen]amino}ethanol, 2-{[(2-Methoxy-1-naphthyl)methylen]amino}ethanol, 2-{[(4-Methoxy-1-naphthyl)methylen]amino}ethanol, 1-{[(2-Hydroxyethyl)imino]methyl}-2-naphthol, 4-{[(2-Hydroxyethyl)imino]methyl}-1-naphthol, 4-{[(2-Hydroxyethyl)imino]methyl}naphthalene-1,3-diol, 4-{[(2-Hydroxyethyl)imino]methyl}-2-methoxy-1-naphthol, 1-{[(2-Hydroxyethyl)imino]methyl}-4-methoxy-2-naphthol, 4-{[(2-Hydroxyethyl)imino]methyl}-1-methoxy-2-naphthol, 2-{[(2,4-Dimethoxy-1-naphthyl)methylen]amino}ethanol, 2-{[(3,4-Dimethoxy-1-naphthyl)methylen]amino}ethanol, 2-({[4-(Dimethylamino)-1-naphthyl]-methylen}amino)ethanol, 2-({3-[4-(Dimethylamino)phenyl]prop-2-enyliden}amino)ethanol, 2-({3-[4-(Diethylamino)phenyl]prop-2-enyliden}amino)ethanol, 4-{3-[(2-Hydroxyethyl)-imino]prop-1-enyl}-2-methoxyphenol, 2-(Diethoxymethyl)phenol, 3-(Diethoxymethyl)-phenol, 4-(Diethoxymethyl)phenol, 3-(Diethoxymethyl)benzol-1,2-diol, 4-(Diethoxymethyl)benzol-1,3-diol, 2-(Diethoxymethyl)benzol-1,4-diol, 2-(Diethoxymethyl)-benzol-1,3-diol, 4-(Diethoxymethyl)benzol-1,2-diol, 5-(Diethoxymethyl)benzol-1,3-diol, 4-(Diethoxymethyl)benzol-1,2,3-triol, 6-(Diethoxymethyl)benzol-1,2,4-triol, 3-(Diethoxymethyl)benzol-1,2,4-triol, 2-(Diethoxymethyl)benzol-1,3,5-triol, 5-(Diethoxymethyl)benzol-1,2,4-triol, 3-(Diethoxymethyl)benzol-1,2,4-triol, 1-(Diethoxymethyl)-2-methoxybenzol, 1-(Diethoxymethyl)-3-methoxybenzol, 1-(Diethoxymethyl)-4-methoxybenzol, 1-(Diethoxymethyl)-2-ethoxybenzol, 1-(Diethoxymethyl)-3-ethoxybenzol, 1-(Diethoxymethyl)-4-ethoxybenzol, 1-(Diethoxymethyl)-2,3-dimethoxybenzol, 1-(Diethoxymethyl)-2,4-dimethoxybenzol, 2-(Diethoxymethyl)-1,4-dimethoxybenzol, 2-(Diethoxymethyl)-1,3-dimethoxybenzol, 4-(Diethoxymethyl)-1,2-dimethoxybenzol, 1-(Diethoxymethyl)-3,5-dimethoxybenzol, 1-(Diethoxymethyl)-2,3,4-trimethoxybenzol, 1-(Diethoxymethyl)-2,3,5-trimethoxybenzol, 2-(Diethoxymethyl)-1,3,4-trimethoxybenzol, 2-(Diethoxymethyl)-1,3,5-trimethoxybenzol, 1-(Diethoxymethyl)-2,4,5-trimethoxybenzol, 2-(Diethoxymethyl)-1,3,4-trimethoxybenzol, 4-(Diethoxymethyl)-3-methoxyphenol, 4-(Diethoxymethyl)-2-methoxyphenol, 5-(Diethoxymethyl)-2-methoxyphenol, 2-(Diethoxymethyl)-5-methoxyphenol, 4-(Diethoxymethyl)-3-ethoxyphenol, 4-(Diethoxymethyl)-2-ethoxyphenol, 2-(Diethoxymethyl)-5-ethoxyphenol, 5-(Diethoxymethyl)-2-ethoxyphenol, 4-(Diethoxymethyl)-2,3-dimethoxyphenol, 4-(Diethoxymethyl)-2,5-dimethoxyphenol, 4-(Diethoxymethyl)-3,5-dimethoxyphenol, 4-(Diethoxymethyl)-2,6-dimethoxyphenol, 4-(Diethoxymethyl)-2,6-diethoxyphenol, 4-(Diethoxymethyl)-3,5-diethoxyphenol, 4-(Diethoxymethyl)-3-methylphenol, 4-(Diethoxymethyl)-2-methylphenol, 4-(Diethoxymethyl)-2,3-dimethylphenol, 4-(Diethoxymethyl)-2,5-dimethylphenol, 4-(Diethoxymethyl)-3,5-dimethylphenol, 4-(Diethoxymethyl)-2,6-dimethylphenol, *N*-[4-(Diethoxymethyl)phenyl]-*N,N*-dibutylamin, *N-*[3-Chlor-4-(diethoxymethyl)phenyl]-*N,N*-dimethylamin, *N*-[4-(Diethoxymethyl)-3-methylphenyl]-*N,N*-dimethylamin, *N*-[4-(Diethoxymethyl)-3-methoxyphenyl]-*N,N*-dimethylamin, *N*-[2-(Diethoxymethyl)phenyl]-*N,N*-dimethylamin, *N*-[4-(Diethoxymethyl)phenyl]-*N,N-*dimethylamin, *N*-[4-(Diethoxymethyl)phenyl]-*N,N*-diethylamin, 2-(Diethoxymethyl)-5-(dimethylamino)phenol, 2-(Diethoxymethyl)-5-(diethylamino)phenol, 1-[4-(Diethoxymethyl)phenyl]pyrrolidin, 1-[4-(Diethoxymethyl)phenyl]piperidin, 4-[4-(Diethoxymethyl)phenyl]morpholin, 4-[2-(Diethoxymethyl)phenyl]morpholin, 1-(Diethoxymethyl)-2-methoxynaphthalin, 1-(Diethoxymethyl)-4-methoxynaphthalin, 1-(Diethoxymethyl)-2-naphthol, 4-(Diethoxymethyl)-1-naphthol, 4-(Diethoxymethyl)-naphthalin-1,3-diol, 4-(Diethoxymethyl)-2-methoxy-1-naphthol, 1-(Diethoxymethyl)-4-methoxy-2-naphthol, 4-(Diethoxymethyl)-1-methoxy-2-naphthol, 1-(Diethoxymethyl)-2,4-dimethoxynaphthalin, 4-(Diethoxymethyl)-1,2-dimethoxynaphthalin, *N*-[4-(Diethoxymethyl)-1-naphthyl]-N,N-dimethylamin, *N*-{4-[3,3-Diethoxyprop-1-enyl]phenyl}-*N,N*-dimethylamin, *N*-{4-[3,3-Diethoxyprop-1-enyl]phenyl}-*N,N*-diethylamin und 4-(3,3-Diethoxyprop-1-enyl)-2-methoxyphenol.

Ganz besonders bevorzugt werden in den erfindungsgemäßen Mitteln Benzaldehyd, Zimtaldhyd und Naphthaldehyd sowie deren Derivate, insbesondere mit einem oder mehreren Hydroxy-, Alkoxy- und Aminosubstituenten, als Komponente B verwendet. Dabei werden wiederum die Verbindungen gemäß Formel V bevorzugt, 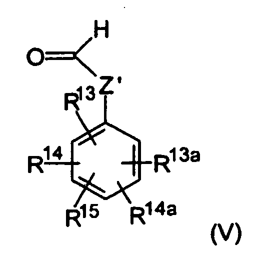 worin
- R¹³, R¹⁴ und R¹⁵ stehen unabhängig voneinander für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe,
- Z' steht für eine direkte Bindung oder eine Vinylengruppe,
- R^{13a} und R^{14a} stehen für Wasserstoff oder bilden zusammen mit dem Restmolekül einen 5- oder 6-gliederigen aromatischen oder aliphatischen Ring.

Ganz besonders bevorzugte Verbindungen der Komponente B werden ausgewählt aus der Gruppe bestehend aus Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxybenzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzatdehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd und Piperonal.

In einer zweiten Ausführungsform kann es zur Erweiterung des Farbspektrums sowie zur Verbesserung der Echtheitseigenschaften vorteilhaft sein, den erfindungsgemäßen Mitteln neben mindestens einer Verbindung gemäß Formel I und mindestens einer reaktiven Carbonylverbindung (Komponente B) mindestens eine weitere Verbindung als Komponente C, ausgewählt aus (a) CH-aciden Verbindungen und (b) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, zuzusetzen.

Die CH-aciden Verbindungen der Komponente C sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluotsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Die primären und sekundären aromatischen Amine der Komponente C sind bevorzugt ausgewählt aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluot, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylarnino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamidophenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diarnino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlorbenzol (HC Red Nr.10), 2-(4-Amino-2-nitroanilino)-bertzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-,carbonsäure, 1-Amino-5-chlor-4-(2-hydroyethylamino)-2-nitrobenzol, aromatische Aniline bzw. Phenole mit einem weiteren aromatischen Rest, wie sie in der Formel VI dargestellt sind 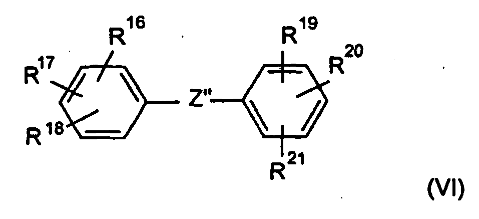 in der
- R¹⁶ für eine Hydroxy- oder eine Aminogruppe, die durch C₁-C₆-Alkyl-, C₁-C₆-Hydroxyalkyl-, C₁-C₆-Alkoxy- oder C₁-C₆-Alkoxy-C₁-C₆-alkylgruppen substituiert sein kann, steht,
- R¹⁷, R¹⁸, R¹⁹, R²⁰ und R²¹ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
- Z" für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel VII

   **-Q'-(CH₂-Q-CH₂-Q")ₒ-** **(VII)**

   in der
   - Q eine direkte Bindung, eine CH₂- oder CHOH-Gruppe bedeutet,
   - Q' und Q" unabhängig voneinander für ein Sauerstoffatom, eine NR²²-Gruppe, worin R²² ein Wasserstoffatom, eine C₁-C₆-Alkylgruppe oder C₁-C₆-Hydroxyalkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
   - o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die stickstoffhaltigen heterocyclischen Verbindungen der Komponente C sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-aminopyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methylpyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Die aromatischen Hydroxyverbindungen der Komponente C sind bevorzugt ausgewählt aus der Gruppe bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure und 3,6-Dihydroxy-2,7-naphthalinsulfonsäure.

Aus den Druckschriften EP-A2-998 908 und JP-A2-2002047153 sind dem Fachmann Haarfärbemittel bekannt, die u.a. als direktziehenden Farbstoff mindestens eine Verbindung mit einem 1,2-Dihydro-pyrimidinium-Rest enthalten. In einer dritten Ausführungsform enthält das Färbemittel mindestens ein Reaktionsprodukt (im folgenden als Reaktionsprodukt RP bezeichnet) aus einem 1,2-Dihydro-pyrimidinium-Derivat der Formel I und einer Verbindung der Komponente B, insbesondere Verbindungen gemäß Formel II, als direktziehenden Farbstoff. Derartige Reaktionsprodukte RP können z. B. durch Erwärmen der beiden Reaktionspartner in wässrigem neutralen bis schwach alkalischen Milieu erhalten werden, wobei die Reaktionsprodukte RP entweder als Feststoff aus der Lösung ausfallen oder durch Eindampfen der Lösung daraus isoliert werden. Ferner besteht die Möglichkeit, die Reaktionsprodukte gemäß Literatur H. Baumann et al, *J. Liebigs Ann. Chem.,* **1968,** *717*, 124-136 oder DE-A1-2165913 darzustellen.

In den erfindungsgemäßen Mitteln können somit Reaktionsprodukte RP gemäß Formel VIII enthalten sein, 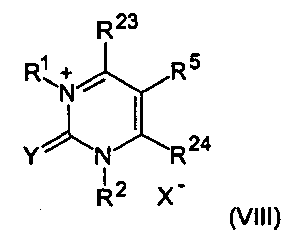 worin
- R¹, R², R⁵, Y und X so definiert sind, wie unter Formel I beschrieben,
- R²³ und R²⁴ unabhängig voneinander stehen für ein Wasserstoffatom, eine C₁-C₆-Alkylgruppe, eine Gruppe gemäß Formel IX, 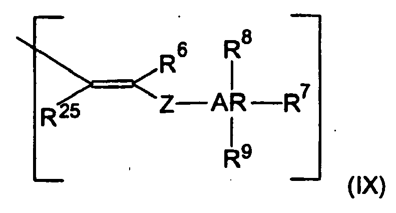 worin
   - R⁶, R⁷, R⁸, R⁹, AR und Z so definiert sind, wie unter Formel II beschrieben,
   - R²⁵ steht für ein Wasserstoffatom oder eine C₁-C₅-Alkylgruppe,
mit der Maßgabe, daß mindestens einer der Reste R²³ und R²⁴ für eine Gruppe gemäß Formel IX steht.

Zur Synthese der Reaktionsprodukte RP können Molverhältnisse der Komponente B zu der Verbindung gemäß Formel I von etwa 1:1 bis etwa 2:1 sinnvoll sein. Ist bei einem 1,2-Dihydro-pyrimidinium-Derivat gemäß Formel I nur ein Substituent R³ oder R⁴ von Wasserstoff verschieden, wird bei der Synthese der Reaktionsprodukte RP etwa 1 Moläquivalent der Komponente B mit einem Moläquivalent 1,2-Dihydro-pyrimidinium-Derivat umgesetzt. In diesem Fall steht entweder R²³ oder R²⁴ in Formel VIII für ein Wasserstoffatom und der jeweils andere Rest für eine Gruppe gemäß Formel IX. Sind beide Substituenten R³ und R⁴ in Formel I von einem Wasserstoffatom verschieden, können diese 1,2-Dihydro-pyrimidinium-Derivate sowohl mit etwa einem, als auch mit etwa zwei Moläquivalenten der Komponente B umgesetzt werden. In letzterem Fall stehen beide Reste R²³ und R²⁴ in den resultierenden. Reaktionsprodukten RP für eine Gruppe gemäß Formel IX.

Es ist besonders bevorzugt, wenn die erfindungsgemäßen Mittel solche Reaktionsprodukte RP gemäß Formel VIII enthalten, in denen AR gemäß Formel IX für Benzol oder Naphthalin steht.

Ferner ist es besonders bevorzugt, wenn die erfindungsgemäßen Mittel solche Reaktionsprodukte RP gemäß Formel VIII enthalten, in denen Z gemäß Formel IX für eine direkte Bindung oder Vinylen steht.

Es ist besonders bevorzugt, wenn die erfindungsgemäßen Mittel solche Reaktionsprodukte RP gemäß Formel VIII enthalten, in denen R²⁵ gemäß Formel IX für ein Wasserstoffatom steht.

Es ist besonders bevorzugt, wenn die erfindungsgemäßen Mittel solche Reaktionsprodukte RP gemäß Formel VIII enthalten, in denen R⁶ gemäß Formel IX für ein Wasserstoffatom steht.

Es ist besonders bevorzugt, wenn die erfindungsgemäßen Mittel solche Reaktionsprodukte RP gemäß Formel VIII enthalten, in denen R⁷, R⁸ und R⁹ gemäß Formel IX stehen unabhängig voneinander für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonamidgruppe, eine Carbamoylgruppe, eine C₂-C₈-Acylgruppe, eine Acetylgruppe, eine Sulfonsäuregruppe, eine Sulfonamidogruppe oder eine Nitrogruppe:

Es ist besonders bevorzugt, wenn die erfindungsgemäßen Mittel solche Reaktionsprodukte RP gemäß Formel VIII enthalten, in denen die Reste R⁷, R⁸ und R⁹ gemäß Formel IX stehen unabhängig voneinander für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₈-Hydroxyalkyloxygruppe, eine Sulfonylgruppe, eine Carboxylgruppe, eine Sulfonamidgruppe, eine Carbamoylgruppe, eine C₂-C₆-Acylgruppe, eine Acetylgruppe, eine Sulfonsäuregruppe, eine Sulfonamidogruppe oder eine Nitrogruppe, mit der Maßgabe, daß, wenn AR für Benzol und einer der Reste R⁷, R⁸ oder R⁹ in para-Position zu Gruppe Z für ein Wasserstoffatom, ein Halogenatom, eine C₁-C₆-Alkoxygruppe oder eine Aminogruppe steht, die beiden anderen Reste unabhängig voneinander weder ein Wasserstoffatom noch ein Halogenatom bedeuten.

Es ist besonders bevorzugt, wenn die erfindungsgemäßen Mittel solche Reaktionsprodukte RP gemäß Formel VIII enthalten, in denen die Reste R⁷, R⁸ und R⁹ unabhängig voneinander stehen für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonamidgruppe, eine Carbamoylgruppe, eine C₂-C₆-Acylgruppe, eine Acetylgruppe, eine Sulfonsäuregruppe, eine Sulfonamidogruppe oder eine Nitrogruppe, mit der Maßgabe, daß, wenn AR für Benzol steht, mindestens einer der Reste R⁷, R⁸ und R⁹ für eine Gruppe steht, die ausgewählt ist aus einer Hydroxygruppe, einer C₁-C₆-Dialkylaminogruppe, einer Di(C₂-C₆-hydroxyalkyl)aminogruppe, einer Di(C₁-C₆-alkoxy-C₁-C₆-alkyl)aminoguppe und einer C₁-C₆-Hydroxyalkyloxygruppe.

Als ganz besonders bevorzugte Reaktionsprodukte RP gemäß Formel VIII ist gemäß dieser Ausführungsform in den erfindungsgemäßen Mitteln mindestens eine Verbindung ausgewählt aus der Gruppe bestehend aus 4-[2-[2-hydroxyphenyl]ethenyl]-2,3-dihydro-1,3,6-trimethyl-2-oxo-pyrimidinium-chlorid, 4-[2-[3-hydroxyphenyl]ethenyl]-2,3-dihydro-1,3,6-trimethyl-2-oxo-pyrimidinium-hydrogensulfat, 4-[2-[4-hydroxyphenyl]ethenyl]-2,3-dihydro-1,3,6-trimethyl-2-oxo-pyrimidinium-chlorid, 4-[2-[2,3-dihydroxyphenyl]ethenyl]-2,3-dihydro-1,3,6-trimethyl-2-oxo-pyrimidinium-chlorid, 4-[2-[2,4-dihydroxyphenyl]ethenyl]-2,3-dihydro-1,3,6-trimethyl-2-oxo-pyrimidinium-chlorid, 4-[2-[2,5-dihydroxyphenyl]ethenyl]-2,3-dihydro-1,3,6-trimethyl-2-oxo-pyrimidinium-hydrogensulfat, 4-[2-[2,6-dihydroxyphenyl]ethenyl]-2,3-dihydro-1,3,6-trimethyl-2-oxopyrimidinium-chlorid, 4-[2-[3,4-dihydroxyphenyl]ethenyl]-2,3-dihydro-1,3,6-trimethyl-2-oxo-pyrimidinium-chlorid, 4-[2-[3,5-dihydroxyphenyl]ethenyl]-2,3-dihydro-1,3,6-trimethyl-2-oxo-pyrimidinium-chlorid, 4-[2-[3,6-dihydroxyphenyl]ethenyl]-2,3-dihydro-1,3,6-trimethyl-2-oxo-pyrimidinium-chlorid, 4-[2-[2,3,4-trihydroxyphenyl]ethenyl]-2,3-dihydro-1,3,6-trimethyl-2-oxo-pyrimidinium-chlorid, 4-[2-[2,3,5-trihydroxyphenyl]ethenyl]-2,3-dihydro-1,3,6-trimethyl-2-oxo-pyrimidinium-hydrogensulfat, 4-[2-[2,3,6-trihydroxyphenyl]ethenyl]-2,3-dihydro-1,3,6-trimethyl-2-oxo-pyrimidinium-chlorid, 4-[2-[3,4,5-trihydroxyphenyl]ethenyl]-2,3-dihydro-1,3,6-trimethyl-2-oxo-pyrimidinium-hydrogensulfat, 4-[2-[3,4,6-trihydroxyphenyl]-ethenyl]-2,3-dihydro-1,3,6-trimethyl-2-oxopyrimidinium-chlorid, 4-[2-[2,4,5-trihydroxy-phenyl]ethenyl]-2,3-dihydro-1,3,6-trimethyl-2-oxo-pyrimidinium-chlorid, 4-[2-[2,4,6-trihydroxyphenyl]ethenyl]-2,3-dihydro-1,3,6-trimethyl-2-oxo-pyrimidinium-hydrogensulfat, 4-[2-[2,3-dimethoxyphenyl]ethenyl]-2,3-dihydro-1,3,6-trimethyl-2-oxo-pyrimidinium-chlorid, 4-[2-[2,4-dimethoxyphenyl]ethenyl]-2,3-dihydro-1,3,6-trimethyl-2-oxo-pyrimidinium-chlorid, 4-[2-[2,5-dimethoxyphenyl]ethenyl]-2,3-dihydro-1,3,6-trimethyl-2-oxo-pyrimidinium-chlorid, 4-[2-[2,6-dimethoxyphenyl]ethenyl]-2,3-dihydro-1,3,6-trimethyl-2-oxo-pyrimidinium-chlorid, 4-[2-[3,4-dimethoxyphenyl]ethenyl]-2,3-dihydro-1,3,6-trimethyl-2-oxo-pyrimidinium-chlorid, 4-[2-[3,5-dimethoxyphenyl]ethenyl]-2,3-dihydro-1,3,6-trimethyl-2-oxo-pyrimidinium-chlorid, 4-[2-[3,6-dimethoxyphenyl]ethenyl]-2,3-dihydro-1,3,6-trimethyl-2-oxopyrimidinium-chlorid, 4-[2-[2,3,4-trimethoxyphenyl]ethenyl]-2,3-dihydro-1,3,6-trimethyl-2-oxo-pyrimidinium-chlorid, 4-[2-[2,3,5-trimethoxyphenyl]ethenyl]-2,3-dihydro-1,3,6-trimethyl-2-oxo-pyrimidinium-chlorid, 4-[2-[2,3,6-trimethoxyphenyl]ethenyl]-2,3-dihydro-1,3,6-trimethyl-2-oxo-pyrimidinium-chlorid, 4-[2-[3,4,5-trimethoxyphenyl]ethenyl]-2,3-dihydro-1,3,6-trimethyl-2-oxo-pyrimidinium-chlorid, 4-[2-[3,4,6-trimethoxyphenyl]ethenyl]-2,3-dihydro-1-,3,6-trimethyl-2-oxo-pyrimidinium-hydrogensulfat, 4-[2-[2,4,6-trimethoxyphenyl]ethenyl]-2,3-dihydro-1,3,6-trimethyl-2-oxo-pyrimidinium-chlorid, 4-[2-[2-hydroxy-3-methoxyphenyl]-ethenyl]-2,3-dihydro-1,3,6-trimethyl-2-oxo-pyrimidinium-chlorid, 4-[2-[2-hydroxy-4-methoxyphenyl]ethenyl]-2,3-dihydro-1,3,6-trimethyl-2-oxopyrimidinium-chlorid, 4-[2-[2-hydroxy-5-methoxyphenyl]ethenyl]-2,3-dihydro-1,3,6-trimethyl-2-oxo-pyrimidinium-chlorid, 4-[2-[2-hydroxy-6-methoxyphenyl]ethenyl]-2,3-dihydro-1,3,6-trimethyl-2-oxo-pyrimidinium-hydrogensulfat, 4-[2-[3-hydroxy-2-methoxyphenyl]ethenyl]-2,3-dihydro-1,3,6-trimethyl-2-oxo-pyrimidinium-chlorid, 4-[2-[3-hydroxy-4-methoxyphenyl]ethenyl]-2,3-dihydro-1,3,6-trimethyl-2-oxo-pyrimidinium-hydrogensulfat, 4-[2-[3-hydroxy-5-methoxyphenyl]ethenyl]-2,3-dihydro-1,3,6-trimethyl-2-oxo-pyrimidinium-chlorid, -4-[2-[3-hydroxy-6-methoxyphenyl]-ethenyl]-2,3-dihydro-1,3,6-trimethyl-2-oxo-pyrimidinium-hydrogensulfat, 4-[2-[4-hydroxy-2-methoxyphenyl]ethenylJ-2,3-dihydro-1,3,6-trimethyl-2-oxo-pyrimidinium-chlorid, 4-[2-[4-hydroxy-3-methoxyphenyl]ethenyl]-2,3-dihydro-1,3,6-trimethyl-2-oxo-pyrimidinium-chlorid, 4-[2-[2,3-dihydroxy-4-methoxyphenyl]ethenyl]-2,3-dihydro-1,3,6-trimethyl-2-oxo-pyrimidinium-chlorid, 4-[2-[2,3-dihydroxy-5-methoxyphenyl]ethenyl]-2,3-dihydro-1,3,6-trimethyl-2-oxopyrimidinium-chlorid, 4-[2-[2,3-dihydroxy-6-methoxyphenyl]ethenyl]-2,3-dihydro-1,3,6-trimethyl-2-oxo-pyrimidiniumchlorid, 4-[2-[2,4-dihydroxy-3-methoxy-phenyl]ethenyl]-2,3-dihydro-1,3,6-trimethyl-2-oxo-pyrimidinium-chlorid, 4-[2-[2,4-dihydroxy-5-methoxyphenyl]ethenylJ-2,3-dihydro-1,3,6-trimethyl-2-oxo-pyrimidinium-chlorid, 4-[2-[2,4-dihydroxy-6-methoxyphenyl]ethenyl]-2,3-dihydro-1,3,6-trimethyl-2-oxo-pyrimidinium-hydrogensulfat, 4-[2-[2,5-dihydroxy-3-methoxyphenyl]ethenyl]-2,3-dihydro-1,3,6-trimethyl-2-oxo-pyrimidinium-hydrogensulfat, 4-[2-[2,5-dihydroxy-4-methoxyphenyl]-ethenyl]-2,3-dihydro-1,3,6-trimethyl-2-oxo-pyrimidinium-chlorid, 4-[2-[2,5-dihydroxy-6-methoxyphenyl]ethenyl]-2,3-dihydro-1,3,6-trimethyl-2-oxo-pyrimidinium-chlorid, 4-[2-[2,6-dihydroxy-3-methoxyphenyl]ethenyl]-2,3-dihydro-1,3,6-trimethyl-2-oxo-pyrimidinium-chlorid, 4-[2-[2,6-dihydroxy-4-methoxyphenyl]ethenyl]-2,3-dihydro-1,3,6-trimethyl-2-oxopyrimidinium-chlorid, 4-[2-[2,6-dihydroxy-5-methoxyphenyl]ethenyl]-2,3-dihydro-1,3,6-trimethyl-2-oxo-pyrimidinium-chlorid, 4-[2-[2,3-dimethoxy-4-hydroxy-phenyl]ethenyl]-2,3-dihydro-1,3,6-trimethyl-2-oxo-pyrimidinium-chlorid, 4-[2-[2,3-dimethoxy-5-hydroxy-phenyl]ethenyl]-2,3-dihydro-1,3,6-trimethyl-2-oxo-pyrimidinium-chlorid, 4-[2-[2,3-dimethoxy-6-hydroxy-phenyl]ethenyl]-2,3-dihydro-1,3,6-trimethyl-2-oxo-pyrimidinium-chlorid, 4-[2-[2,4-dimethoxy-3-hydroxy-phenyl]ethenyl]-2,3-dihydro-1,3,6-trimethyl-2-oxopyrimidinium-chlorid, 4-[2-[2,4-dimethoxy-5-hydroxy-phenyl]ethenyl]-2,3-dihydro-1,3,6-trimethyl-2-oxo-pyrimidinium-chlorid, 4-[2-[2,4-dimethoxy-6-hydroxy-phenyl]ethenyl]-2,3-dihydro-1,3,6-trimethyl-2-oxo-pyrimidinium-chlorid, 4-[2-[2,5-dimethoxy-3-hydroxyphenyl]ethenyl]-2,3-dihydro-1,3,6-trimethyl-2-oxo-pyrimidinium-chlorid, 4-[2-[2,5-dimethoxy-4-hydroxy-phenyl]ethenyl]-2,3-dihydro-1,3,6-trimethyl-2-oxo-pyrimidinium-hydrogensulfat, 4-[2-[2,5-dimethoxy-6-hydroxy-phenyl]ethenyl]-2,3-dihydro-1,3,6-trimethyl-2-oxo-pyrimidinium-chlorid, 4-[2-[2,6-dimethoxy-3-hydroxy-phenyl]ethenyl]-2,3-dihydro-1,3,6-trimethyl-2-oxo-pyrimidinium-hydrogensulfat, 4-[2-[2,6-dimethoxy-4-hydroxy-phenyl]ethenyl]-2,3-dihydro-1,3,6-trimethyl-2-oxo-pyrimidinium-chlorid, 4-[2-[2,6-dimethoxy-5-hydroxy-phenyl]ethenyl]-2,3-dihydro-1,3,6-trimethyl-2-oxo-pyrimidinium-chlorid, 4-[2-[3,4-dimethoxy-3-hydroxy-phenyl]ethenyl]-2,3-dihydro-1,3,6-trimethyl-2-oxopyrimidinium-hydrogensulfat, 4-[2-[3,4-dimethoxy-5-hydroxy-phenyl]ethenyl]-2,3-dihydro-1,3,6-trimethyl-2-oxo-pyrimidinium-chlorid, 4-[2-[3,4-dimethoxy-6-hydroxy-phenyl]-ethenyl]-2,3-dihydro-1,3,6-trimethyl-2-oxo-pyrimidinium-hydrogensulfat, 4-[2-[3,5-dimethoxy-2-hydroxy-phenyl]ethenyl]-2,3-dihydro-1,3,6-trimethyl-2-oxo-pyrimidinium-chlorid, 4-[2-[3,5-dimethoxy-4-hydroxy-phenyl]ethenyl]-2,3-dihydro-1,3,6-trimethyl-2-oxopyrimidinium-chlorid, 4-[2-[3,5-dimethoxy-6-hydroxy-phenyl]ethenyl]-2,3-dihydro-1,3,8-trimethyl-2-oxo-pyrimidinium-chlorid, 4-[2-[3,5-dimethyl-4-hydroxy-phenyl]ethenyl]-2,3-dihydro-1,3,6-trimethyl-2-oxo-pyrimidinium-chlorid, 4-[2-[3,5-dimethyl-2-hydroxy-phenyl]-ethenyl]-2,3-dihydro-1,3,6-trimethyl-2-oxo-pyrimidinium-chlorid, 4-[2-[4-(dimethylamino)-2-hydroxyphenyl]ethenyl]-2,3-dihydro-1,3,6-trimethyl-2-oxo-pyrimidiniumchlorid, 4-[2-[4-(dimethylamino)-3-hydroxyphenyl]ethenyl]-2,3-dihydro-1,3,6-trimethyl-2-oxopyrimidinium-chlorid, 4-[2-[4-(diethylamino)-2-hydroxyphenyl]ethenyl]-2,3-dihydro-1,3,6-trimethyl-2-oxo-pyrimidinium-chlorid, 4-[2-[4-(diethylamino)-3-hydroxyphenyl]ethenyl]-2,3-dihydro-1,3,6-trimethyl-2-oxo-pyrimidinium-chlorid, 4-[2-[4-(diethylamino)-2-methylphenyl]ethenyl]-2,3-dihydro-1,3,6-trimethyl-2-oxo-pyrimidinium-chlorid, 4-[2-[4-(diethylamino)-3-methylphenyl]-ethenylj-2,3-dihydro-1,3,6-trimethyl-2-oxo-pyrimidinium-chlorid, 4-[2-[2-hydroxy-naphth-1-yl]ethenyl]-2,3-dihydro-1,3,6-trimethyl-2-oxopyrimidinium-chlorid, 4-[2-[3-hydroxy-naphth-1-yl]ethenyl]-2,3-dihydro-1,3,6-trimethyl-2-oxo-pyrimidinium-chlorid, 4-[2-[4-hydroxy-naphth-1-yl]ethenyl]-2,3-dihydro-1,3,6-trimethyl-2-oxo-pyrimidinium-chlorid, 4-[2-[2-methoxy-naphth-1-y1]ethenyl]-2,3-dihydro-1,3,6-trimethyl-2-oxopyrimidinium-chlorid, 4-[2-[3-methoxy-naphth-1-yl]ethenyl]-2,3-dihydro-1,3,6-trimethyl-2-oxopyrimidinium-chlorid, 4-[2-[4-methoxy-naphth-1-yl]ethenyl]-2,3-dihydro-1,3,6-trimethyl-2-oxopyrimidinium-chlorid, 4-[2-[4-(dimethylamino)-naphth-1-yl]ethenyl]-2,3-dihydro-1,3,6-trimethyl-2-oxo-pyrimidinium-hydrogensulfat, 4-[2-[4-(diethylamino)-naphth-1-yl]ethenyl]-2,3-dihydro-1,3,6-trimethyl-2-oxo-pyrimidinium-chlorid, 4,6-Bis[2-[2-hydroxyphenyl]ethenyl]-2,3-diyhydro-1,3-dimethyl-2-oxo-pyrimidiniumchlorid, 4,6-Bis[2-[3-hydroxyphenyl]ethenyl]-2,3-diyhydro-1,3-dimethyl-2-oxo-pyrimidinium-chlorid, 4,6-Bis[2-[4-hydroxyphenyl]ethenyl]-2,3-diyhydro-1,3-dimethyl-2-oxo-pyrimidinium-chlorid, 4,6-Bis[2-[2,3-dihydroxyphenyl]ethenyl]-2,3-diyhydro-1,3-dimethyl-2-oxo-pyrimidinium-hydrogensulfat, 4,6-Bis[2-[2,4-dihydroxy-phenyl]ethenyl]-2,3-diyhydro-1,3-dimethyl-2-oxo-pyrimidinium-chlorid, 4,6-Bis[2-[2,5-dihydroxyphenyl]ethenyl]-2,3-diyhydro-1,3-dimethyl-2-oxo-pyrimidinium-chlorid, 4,6-Bis[2-[2,6-dihydroxyphenyl]ethenyl]-2,3-diyhydro-1,3-dimethyl-2-oxo-pyrimidinium-hydrogensulfat, 4,6-Bis[2-[3,4-dihydroxyphenyl]ethenyl]-2,3-diyhydro-1,3-dimethyl-2-oxopyrimidinium-chlorid, 4,6-Bis[2-[3,5-dihydroxyphenyl]ethenyl]-2,3-diyhydro-1,3-dimethyl-2-oxo-pyrimidinium-hydrogensulfat, 4,6-Bis[2-[3,6-dihydroxyphenyl]ethenyl]-2,3-diyhydro-1,3-dimethyl-2-oxo-pyrimidinium-chlorid, 4,6-Bis[2-[2,3,4-trihydroxyphenyl]ethenyl]-2,3-diyhydro-1,3-dimethyl-2-oxo-pyrimidinium-chlorid, 4,6-Bis[2-[2,3,5-trihydroxyphenyl]-ethenyl]-2,3-diyhydro-1,3-dimethyl-2-oxo-pyrimidinium-chlorid, 4,8-Bis[2-[2,3,6-trihydroxy-phenyl]ethenyl]-2,3-diyhydro-1,3-dimethyl-2-oxo-pyrimidinium-chlorid, 4,6-Bis[2-(3,4,5-trihydroxyphenyl]ethenyl]-2,3-diyhydro-1,3-dimethyl-2-oxo-pyrimidinium-chlorid, 4,6-Bis[2-[2,4,6-trihydroxyphenyl]ethenyl]-2,3-diyhydro-1,3-dimethyl-2-oxopyrimidinium-chlorid, 4,6-Bis[2-[2-methoxyphenyl]ethenyl]-2,3-diyhydro-1,3-dimethyl-2-oxo-pyrimidinium-chlorid, 4,6-Bis[2-[3-methoxyphenyl]ethenyl]-2,3-diyhydro-1,3-dimethyl-2-oxo-pyrimidinium-chlorid, 4,6-Bis[2-[4-methoxyphenyl]ethenyl]-2,3-diyhydro-1,3-dimethyl-2-oxo-pyrimidinium-chlorid, 4,6-Bis[2-[2-hydroxy-3-methoxyphenyl]ethenyl]-2,3-diyhydro-1,3-dimethyl-2-oxo-pyrimidinium-chlorid, 4,6-Bis[2-[2-hydroxy-4-methoxyphenyl]ethenyl]-2,3-diyhydro-1,3-dimethyl-2-oxo-pyrimidinium-chlorid, 4,6-Bis[2-[2-hydroxy-5-methoxyphenyl]ethenyl]-2,3-diyhydro-1,3-dimethyl-2-oxo-pyrimidinium-chlorid, 4,6-Bis[2-[2-hydroxy-6-methoxyphenyl]-ethenyl]-2,3-diyhydro-1,3-dimethyl-2-oxopyrimidinium-chlorid, 4,6-Bis[2-[2-hydroxy-3,4-dimethoxyphenyl]ethenyl]-2,3-diyhydro-1,3-dimethyl-2-oxo-pyrimidinium-chlorid, 4,6-Bis[2-[2-hydroxy-3,5-dimethoxyphenyl]ethenyl]-2,3-diyhydro-1,3-dimethyl-2-oxo-pyrimidinium-chlorid, 4,6-Bis[2-[2-hydroxy-3,6-methoxyphenyl]ethenyl]-2,3-diyhydro-1,3-dimethyl-2-oxopyrimidinium-chlorid, 4,6-Bis[2-[3-hydroxy-2,4-dimethoxyphenyl]ethenyl]-2,3-diyhydro-1,3-dimethyl-2-oxo-pyrimidinium-chlorid, 4,6-Bis[2-[3-hydroxy-2,5-dimethoxyphenyl]ethenyl]-2,3-diyhydro-1,3-dimethyl-2-oxo-pyrimidinium-chlorid, 4,6-Bis[2-(3-hydroxy-2,6-dimethoxyphenyl]ethenyl]-2,3-diyhydro-1,3-dimethyl-2-oxopyrimidinium-chlorid, 4,6-Bis[2-[4-hydroxy-2,3-dimethoxyphenyl]ethenyl]-2,3-diyhydro-1,3-dimethyl-2-oxo-pyrimidinium-chlorid, 4,6-Bis[2-[4-hydroxy-2,5-dimethoxyphenyl]etheriyl]-2,3-diyhydro-l,3-dimethyl-2-oxo-pyrimidinium-chlorid, 4,6-Bis[2-[4-hydroxy-2,6-dimethoxyphenyl]ethenyl]-2,3-diyhydro-1,3-dimethyl-2-oxopyrimidinium-chlorid, 4,6-Bis[2-[4-hydroxy-3,5-dimethoxyphenyl]-ethenyl]-2,3-diyhydro-1,3-dimethyl-2-oxo-pyrimidinium-chlorid, 4,6-Bis[2-[4-(diethylamino)phenyl]ethenyl]-2,3-diyhydro-1,3-dimethyl-2-oxo-pyrimidinium-chlorid und 4,6-Bis[2-[4-(diethylamino)-2-hydroxyphenyl]ethenyl]-2,3-diyhydro-1,3-dimethyl-2-oxo-pyrimidinium-chlorid enthalten.

Die voranstehend genannten Verbindungen mit der Formel I, die Verbindungen der Komponente B, Komponente C sowie die Reaktionsprodukte RP werden jeweils vorzugsweise in einer Menge von 0,03 bis 65 mmol, insbesondere von 1 bis 40 mmol, bezogen auf 100 g des gesamten Färbemittels, verwendet.

In einer vierten Ausführungsform können die erfindungsgemäßen Mittel neben mindestens einer Verbindung gemäß Formel I und mindestens einer reaktiven Carbonylverbindung, mindestens eine Entwicklerkomponente und gegebenenfalls mindestens eine Kupplerkomponente als Oxidationsfarbstoffvorprodukte enthalten.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 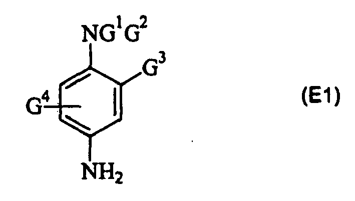 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C,- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁-bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 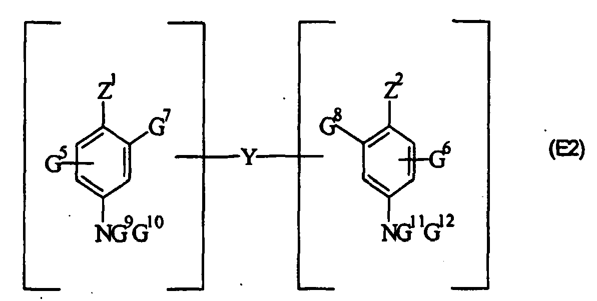 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 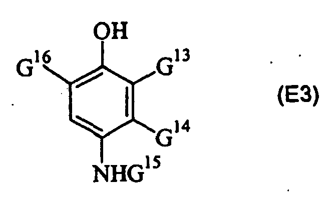 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest, einen Hydroxy-(C₁- bis C₄)-alkylaminorest, einen C₁- bis C₄-Hydroxyalkoxyrest, einen C₁-bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest oder einen C₁-bis C₄-Cyanoalkylrest,
- G¹⁵ steht für Wasserstoff, einen C₁- bis C₄-Alkylrest; einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4.5-Diamino-1-(β-hydroxyethyl)-3-methylpyrazol, 4,5-Diamino-l-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren formen, sofern ein tautomeres Gleichgewicht besteht: 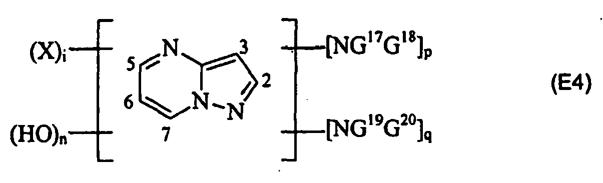 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C,- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁- bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C,- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

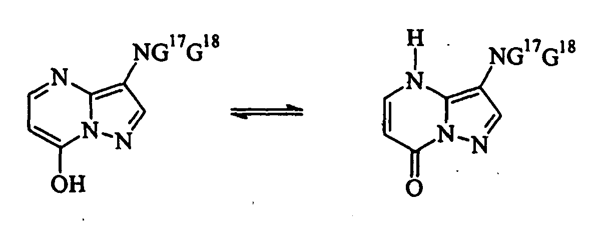

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

Die in den erfindungsgemäßen Mitteln optional enthaltenen Kupplerkomponenten sind bevorzugt ausgewählt aus
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenot, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-S-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Als Vorstufen naturanaloger Farbstoffe können im Rahmen einer fünften Ausführungsform ferner bevorzugt solche Indole und Indoline in den erfindungsgemäßen Mitteln eingesetzt werden, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel Xa, 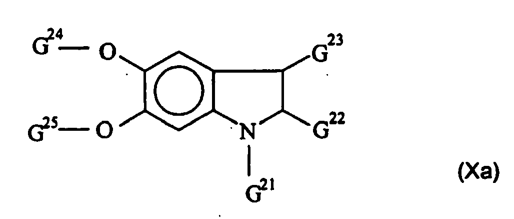 in der unabhängig voneinander
- G²¹ steht für Wasserstoff, eine C₁-C₄-Alkylgruppe oder eine C₁-C₄-Hydroxy-alkylgruppe,
- G²² steht für Wasserstoff oder eine -COOH-Gruppe, wobei die -COOH-Gruppe auch als Salz mit einem physiologisch verträglichen Kation vorliegen kann,
- G²³ steht für Wasserstoff oder eine C₁-C₄-Alkylgruppe,
- G²⁴ steht für Wasserstoff, eine C₁-C₄-Alkylgruppe oder eine Gruppe -CO-G²⁶, in der G²⁶ steht für eine C₁-C₄-Alkylgruppe, und
- G²⁵ steht für eine der unter G²⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel Xb, 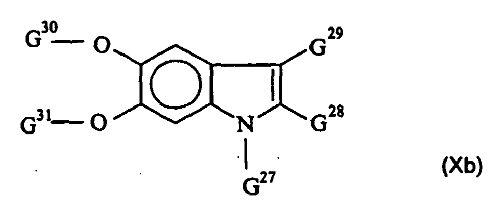 in der unabhängig voneinander
- G²⁷ steht für Wasserstoff, eine C₁-C₄-Alkylgruppe oder eine C₁-C₄-Hydroxyalkylgruppe,
- G²⁸ steht für Wasserstoff oder eine -COOH-Gruppe, wobei die -COOH-Gruppe auch als Salz mit einem physiologisch verträglichen Kation vorliegen kann,
- G²⁹ steht für Wasserstoff oder eine C₁-C₄-Alkylgruppe,
- G³⁰ steht für Wasserstoff, eine C₁-C₄-Alkylgruppe oder eine Gruppe -CO-G³², in der G³² steht für eine C₁-C₄-Alkylgruppe, und
- G³¹ steht für eine der unter G³⁰ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer sechten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, wie Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel bevorzugt einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

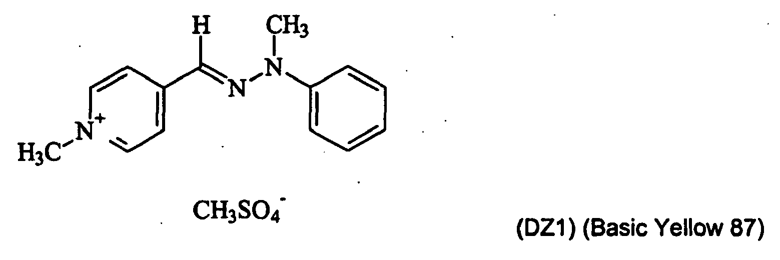

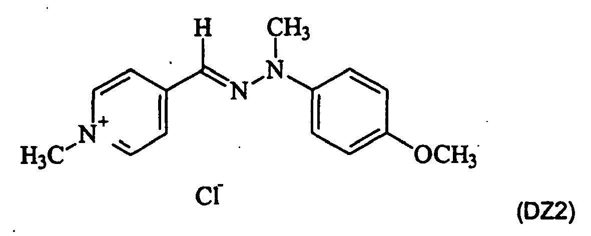

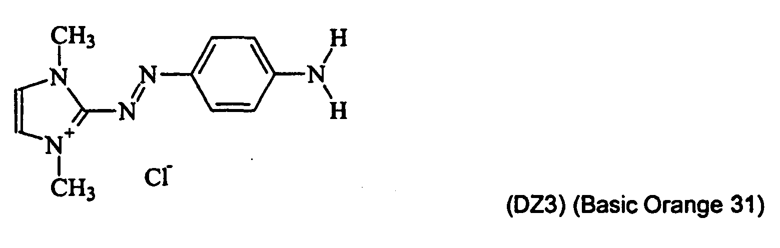

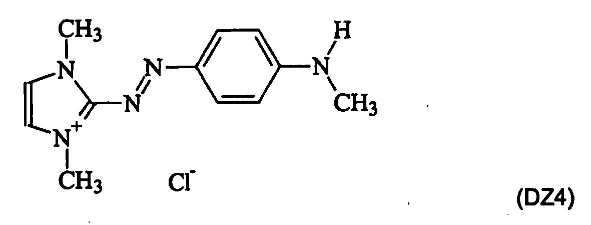

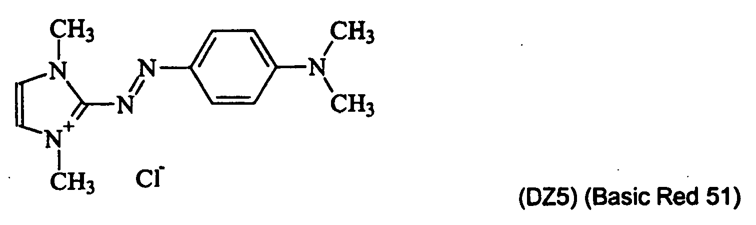

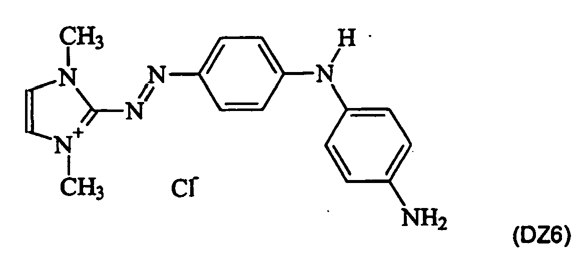

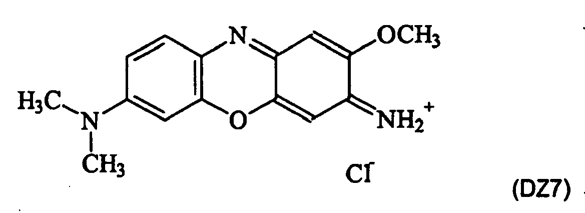

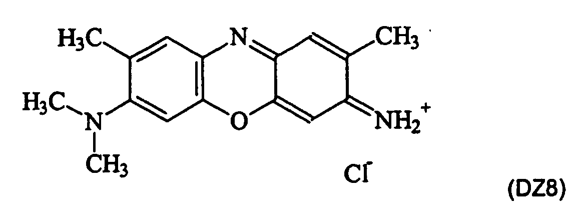

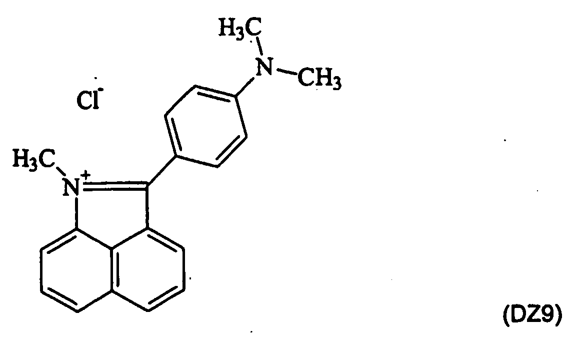

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß besonders bevorzugte direktziehende Farbstoffe.

Die erfindungsgemäßen Mittel gemäß dieser Ausführungsform enthalten die direktziehenden Farbstoffe bevorzugt in einer Menge von 0,01 bis 20 Gew.%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Auf die Anwesenheit von Oxidationsmitteln, z. B. H₂O₂, kann, insbesondere wenn das erfindungsgemäße Mittel keine Oxidationsfarbstoffvorprodukte enthält, verzichtet werden. Wenn das erfindungegemäße Mittel luftoxidable Oxidationsfarbstoffvorprodukte oder Indol bzw. lndolinderivate enthält, kann in einem solchen Fall ohne Probleme auf Oxidationsmittel verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkali- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten.

Weiterhin können die erfindungsgemäßen Färbemittel alle in solchen Zubereitungen bekannten Wirk-, Zusatz- und Hilfsstoffe enthalten. In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Po-Iyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquatemäre Polydimethylsiloxane, Quatemium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise Vinylpyrrolidön/Vinylacrylat-Copolymere, Polyvinylpyrrolidon und Vinylpyrrolidon/Vinylacetat-Copolymere und Polysiloxane,
- kationische Polymere wie quatemisierte Celluloseether, Polysiloxane mit quatemären Gruppen, Dimethyldiallylammoniumchlorid-Polymere, Acrylamid-Dimethyldiallylammoniumchlorid-Copolymere, mit Diethylsulfat quatemierte Dimethylaminoethylmethacrylat-Vinylpyrrolidon-Copölymere, Vinylpyrrolidon-Imidazollniummethochlorid-Copolymere und quatemierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/lsobomylacrylat-Copolymere, Methylvinylether/Maleinsäureanhydrid-Copolymere und Acrylsäure/Ethylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.% und Verdickungsmittel in Konzentrationen von 0,1 bis 25 Gew.% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Diese Färbemittel können zusätzlich
a) mindestens einen weiteren der vorgenannten direktziehenden Farbstoffe und/oder
b) mindestens ein vorgenanntes Oxidationsfarbstoffvorprodukt und/oder mindestens ein vorgenanntes Derivat des Indolins oder Indols sowie
c) gegebenenfalls Oxidationsmittel wie z.B. Wasserstoffperoxid
enthalten.

Ferner können alle Tenside, Farbverstärker, Metallsalze oder Oxidationsmittel sowie alle weiteren Hilfs-, Wirk- und Zusatzstoffe in dem erfindungsgemäßen Mittel des zweiten Gegenstandes der Erfindung enthalten sein, die bereits weiter oben genannt wurden.

Ein zweiter Gegenstand der vorliegenden Erfindung betrifft die Verwendung von mindestens einem 1,2-Dihydro-pyrimidinium-Derivat gemäß Formel I und/oder dessen Enaminform, 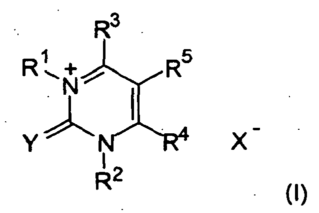 wobei R¹, R², R³, R⁴, R⁵, Y und X- wie oben definiert sind, in Kombination mit mindestens einer reaktiven Carbonylverbindung (Komponente B) als färbende Komponente in Haarfärbemitteln.

In einer bevorzugten Ausführungsform werden die 1,2-Dihydro-pyrimidinium-Derivate gemäß Formel I in Kombination mit mindestens einer reaktiven Carbonylverbindung der Komponente B ausgewählt aus den vorstehend benannten bevorzugten und besonders bevorzugten Vertretern, als färbende Komponente in Haarfärbemitteln verwendet.

Darüber hinaus kann es bevorzugt sein, mindestens ein Reaktionsprodukt RP aus einem 1,2-Dihydro-pyridinium-Derivat gemäß Formel I und einer Verbindung der Komponente B als färbende Komponenten in Haarfärbemitteln zu verwenden.

Ein dritter Gegenstand der vorliegenden Erfindung betrifft ein Verfahren zum Färben von keratinhaltigen Fasern, insbesondere menschlichen Haaren, worin ein Färbemittel, enthaltend mindestens ein 1,2-Dihydro-pyrimidinium-Derivat gemäß Formel I und/oder dessen Enaminform, 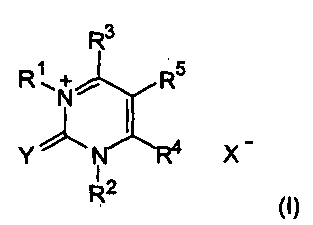 wobei R¹, R², R³, R⁴, R⁵, Y und X- wie oben definiert sind in Kombination mit mindestens einer Verbindung mit einer reaktiven Carbonylgruppe (Komponente B), sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird. Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt.

Dabei können die 1,2-Dihydro-pyrimidinium-Derivate gemäß Formel I und die Verbindungen der Komponente B, insbesondere deren vorstehend benannten bevorzugte und besonders bevorzugte Vertreter, als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Vor der Anwendung des erfindungsgemäßen Mittels in dem erfindungsgemäßen Verfahren kann es wünschenswert sein, die zu färbende keratinhaltige Faser einer Vorbehandlung zu unterziehen. Die zeitliche Abfolge des dazu erforderlichen Vorbehandlungsschritts und der Anwendung des erfindungsgemäßen Mittels muß nicht unmittelbar nacheinander sein, sondern es kann zwischen dem Vorbehandlungsschritt und der Anwendung des erfindungsgemäßen Mittels ein Zeitraum von bis maximal zwei Wochen liegen. Dazu eignen sich mehrere Vorbehandlungsmethoden. Bevorzugt wird die Faser
V1 vor der Anwendung des erfindungsgemäßen Mittels einer Blondierung oder
V2 vor der Anwendung des erfindungsgemäßen Mittels einer oxidativen Färbung
unterzogen.

Im Rahmen der Vorbehandlung V1 wird die keratinhaltige Faser mit einem Blondiermittel behandelt. Das Blondiermittel enthält neben einem Oxidationsmittel, wie üblicherweise Wasserstoffperoxid, bevorzugt mindestens ein als Oxidations- und Bleichverstärker wirksames anorganisches Persalz, wie z.B. ein Peroxodisulfat von Natrium, Kalium oder Ammonium. Färbungen gemäß des erfindungsgemäßen Verfahrens erhalten durch die Vorbehandlung V1 eine besondere Brillanz und Farbtiefe.

Im Rahmen der Vorbehandlung V2 wird ein Mittel enthaltend vorgenannte Oxidationsfarbstoffvorprodukte als Entwickler- und gegebenenfalls Kupplerkomponenten sowie gegebenenfalls vorgenannte Derivate des Indols bzw. Indolins auf die Faser aufgetragen und nach einer Einwirkzeit gegebenenfalls unter Zusatz von vorgenannten geeigneten Oxidationsmitteln auf dem Haar für 5-45 Minuten auf der Kertinfaser belassen. Danach wird das Haar gespült. Durch die anschließende Anwendung des erfindungsgemäßen Mittels kann vorhandenen Oxidationsfärbungen einen neue Farbnuance verliehen werden. Wählt man die Farbnuance des erfindungsgemäßen Mittels in der gleichen Farbnuance der oxidativen Färbung aus, so kann die Färbung vorhandener Oxidationsfärbungen nach dem erfindungsgemäßen Verfahren aufgefrischt werden. Es zeigt sich, daß die Farbauffrischung oder Nuancierung gemäß des erfindungsgemäßen Verfahrens einer Farbauffrischung bzw. Nuancierung allein mit herkömmlichen direktziehenden Farbstoffen in der Farbbrillanz und Farbtiefe überlegen ist.

Enthält das Haarfärbemittel neben den Verbindungen gemäß Formel 1 und gegebenenfalls der Komponente B zusätzlich als Oxidationsmittel Wasserstoffperoxid oder ein wasserstoffperoxidhaltiges Oxidationsmittelgemisch, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationmittel kann unmittelbar vor der Anwendung mit dem Haarfärbemittel gemischt und die Mischung auf das Haar aufgebracht werden. Werden die Verbindungen der Formel I und die Komponente B in einem zweistufigen Verfahren auf das Haar appliziert, ist das Oxidationsmittel in einer der beiden Verfahrensstufen zusammen mit der entsprechenden farbgebenden Komponente anzuwenden. Zu diesem Zweck kann es bevorzugt sein, das Oxidationsmittel mit einer der farbgebenden Komponenten in einem Container zu konfektionieren.

Die 1,2-Dihydro-pyrimidinium-Derivate gemäß Formel I und die Verbindungen der Komponente B können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

Ein vierter Gegenstand, der Erfindung ist die Verwendung von mindestens einem 1,2-Dihydro-pyrimidinium-Derivat gemäß Formel I und/oder dessen Enaminform, 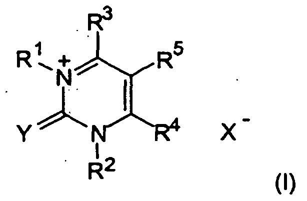 wobei R¹, R², R³, R⁴, R⁵, Y und X- wie oben definiert sind, in Kombination mit reaktiven Carbonylverbindungen (Komponente B), zur Nuancierung von Oxidationsfärbungen von keratinhaltigen Fasern, insbesondere menschlichen Haaren. Bei der Verwendung ist es unerheblich, ob die Nuancierung gleichzeitig während der oxidativen Färbung erfolgt, oder die oxidative Färbung zeitlich vor der Nuancierung liegt.

Ein sechster Gegenstand der Erfindung ist die Verwendung von mindestens einem 1,2-Dihydro-pyrimidinium-Derivat gemäß Formel I und/oder dessen Enaminform, 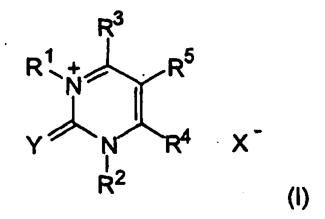 wobei R¹, R², R³, R⁴, R⁵, Y und X⁻ wie oben definiert sind in Kombination mit mindestens einer reaktiven Carbonylverbindung (Komponente B), zur Farbauffrischung von mit oxidativen Färbemitteln gefärbten keratinhaltigen Fasern.

Die Färbungen keratinhaltiger Fasern sind bekanntermaßen Umwelteinflüssen, wie beispielsweise Licht, Reibung oder Waschungen, ausgesetzt und können dadurch an Brillanz und Farbtiefe verlieren. Schlimmstenfalls stellt sich gegebenenfalls eine Nuancenverschiebung der Färbung ein. Solche gealterten Färbungen keratinhaltiger Fasern können, wenn der Anwender es wünscht, durch eine Farbauffrischung wieder annähernd in den farblichen Zustand versetzt werden, wie er sich unmittelbar nach der ursprünglichen Färbung präsentierte. Es ist erfindungsgemäß, für eine solche Farbauffrischung eine Kombination aus mindestens einer Verbindung mit der Formel I und mindestens einer reaktiven Carbonylverbindung zu verwenden.

### Beispiele

### Herstellung einer Färbelösung

Es wurde je eine Aufschlämmung bzw. Lösung von 3 mmol des 1,2-Dihydropyrimidinium-Derivats der Formel I (Komponente A) mit 0,41 g Natriumacetat in 30 ml Wasser bei ca. 50°C hergestellt. Unmittelbar vor der Anwendung werden zu dieser Mischung 3 mmol der Verbindungen der Komponente B hinzugefügt. Die Mischung wurde entweder mit einer 10 %igen wäßrigen Natriumhydroxidlösung auf einen pH-Wert von pH 9 oder mit Salzsäure auf einen pH-Wert von pH 4 bzw. 6 eingestellt (siehe Tabelle 1).

### Ausfärbungen

In die frisch hergestellte Färbelösung wurde bei 30°C 30 Minuten lang eine Strähne zu 90% ergrauten, nicht vorbehandelten Menschenhaares eingebracht. Die Strähne wurde dann 30 Sekunden mit lauwarmem Wasser gespült, mit warmer Luft (30°C bis 40°C) getrocknet und anschließend ausgekämmt.

Die jeweiligen Farbnuancen und Farbtiefen der Beispielausfärbungen sind in der nachfolgenden Tabelle 1 wiedergegeben.

**Verbindungen der Komponente A (zu Tabelle 1):**

| | |
|---|---|
| A1 | 1,2-Dihydro-1,3,4,6-tetramethyl-2-oxo-pyrimidinium-chlorid |
| A2 | 1,2-Dihydro-1,3,4-trimethyl-2-oxo-pyrimidinium-chlorid |
| A3 | 1,2-Dihydro-4,6-dimethyl-1,3-dipropyl-2-oxo-pyrimidinium-chlorid |
| A4 | 1,2-Dihydro-1,3,4,6-tetramethyl-2-thioxo-pyrimidinium-hydrogensulfat |
| A5 | 1,2-Dihydro-1,3,4,6-tetramethyl-2-oxo-pyrimidinium-hydrogensulfat |
| A6 | 1,2-Dihydro-1,3,4,5,6-pentamethyl-2-oxo-pyrimidinium-chlorid |

**Verbindungen der Komponente B (zu Tabelle 1):**

| | |
|---|---|
| B1 | 2,4-Dihydroxybenzaldehyd |
| B2 | Vanillin |
| B3 | Isatin |
| B4 | 3-Indolaldehyd |
| B5 | Coniferylaldehyd |
| B6 | 3,5-Dimethoxy-4-hydroxy-benzaldehyd |
| B7 | 4-Dimethylamino-1-naphthaldehyd |
| B8 | 4-Hydroxybenzaldehyd |
| B9 | 4-Hydroxy-1-naphthaldehyd |
| B10 | 4-Diethylamino-2-hydroxybenzaldehyd |
| B11 | 4-Dimethylamino-benzaldehyd |
| B12 | 4-Hydroxy-2-methoxy-benzaldehyd |
| B13 | 3,4-Dihydroxybenzaldehyd |
| B14 | 4-Dimethylaminozimtaldehyd |
| B15 | 3,5-Dimethyl-4-hydroxybenzaldehyd |
| B16 | 2,3,4-Trihydroxybenzaldehyd |
| B17 | 2,4,6-Trihydroxybenzaldehyd |
| B18 | 3-Hydroxy-4-methoxybenzaldehyd |
| B19 | 2-Hydroxy-4-methoxybenzaldehyd |
| B20 | 2,3-Dimethoxybenzaldehyd |
| B21 | 2,4-Dimethoxybenzaldehyd |
| B22 | 2,3,4-Trimethoxybenzaldehyd |
| B23 | 2,4,5-Trimethoxybenzaldehyd |
| B24 | 3-Brom-4-methoxybenzaldehyd |
| B25 | 3-Methyl-4-methoxybenzaldehyd |
| B26 | 4-Dimethylamino-2-methoxybenzaldehyd |
| B27 | 4-Formyl-1-methylchinotinium-p-toluolsulfonat |
| B28 | Pyrrol-2-carboxaldehyd |
| B29 | 1-Methylpyrrol-2-carboxaldehyd |
| B30 | 2-Furaldehyd |
| B31 | 4-Diethylaminobenzaldehyd |
| B32 | 2,5-Dihydroxybenzaldehyd |
| B33 | 3-Hydroxy-4-nitrobenzaldehyd |
| B34 | 2-Hydroxy-3-methoxy-5-nitrobenzaldehyd |
| B35 | 5-Brom-4-hydroxy-3-methoxybenzaldehyd |
| B36 | 4-Methoxybenzaldehyd |
| B37 | 2-Hydroxy-1-naphthaldehyd |
| B38 | 2-Methoxy-1-naphthaldehyd |
| B39 | 4-Methoxy-1-naphthaldehyd |
| B40 | 2-{[(4-Methoxyphenyl)methylen]amino}ethanol |
| B41 | 4-{[(2-Hydroxyethyl)imino]methyl}-2,6-dimethoxyphenol |
| B42 | 1-(Diethoxymethyl)-4-methoxybenzol |
| B43 | 4-(Diethoxymethyl)-2,6-dimethoxyphenol |

**Tabelle 1**

| **Komponente A** | **Komponente B** | **Farbe** | **pH-Wert** |
|---|---|---|---|
| A1 | B1 | intensiv rotviolett | 9 |
| A1 | B2 | leuchtend violett | 9 |
| A1 | B3 | intensiv orange | 9 |
| A1 | B4 | intensiv orange | 9 |
| A1 | B5 | dunkelgrau | 9 |
| A1 | B6 | intensiv dunkelblau | 9 |
| A1 | B7 | blassgrau | 9 |
| A1 | B8 | intensiv purpur-rot | 9 |
| A1 | B9 | grauschwarz | 9 |
| A1 | B10 | hellrosa | 9 |
| A1 | B11 | intensiv rotviolett | 9 |
| A1 | B15 | leuchtend violett | 9 |
| A1 | B16 | rotbraun | 9 |
| A1 | B17 | blass rosa | 9 |
| A1 | B18 | braun | 9 |
| A1 | B19 | dunkelrot | 9 |
| A1 | B20 | gelbbraun | 9 |
| A1 | B21 | leuchtend orange | 9 |
| A1 | B22 | orangebraun | 9 |
| A1 | B23 | intensiv orangerot | 9 |
| A1 | B24 | gelb | 9 |
| A1 | B25 | braunorange | 9 |
| A1 | B26 | intensiv violett | 9 |
| A1 | B27 | braun | 9 |
| A1 | B28 | rotbraun | 9 |
| A1 | B29 | orange | 9 |
| A1 | B30 | beigebraun | 9 |
| A2 | B1 | rot | 9 |
| A2 | B2 | leuchtend violett | 9 |
| A2 | B3 | orange | 9 |
| A2 | B4 | orange | 9 |
| A2 | B6 | türkisblau | 9 |
| A2 | B8 | pink | 9 |
| A2 | B9 | schwarz | 9 |
| A2 | B12 | leuchtend rosarot | 9 |
| A2 | B13 | intensiv dunkelblau | 9 |
| A2 | B14 | qelb-braun | 9 |
| A2 | B 15 | violett | 9 |
| A3 | B1 | intensiv dunkelrot | 9 |
| A3 | B2 | intensiv violett | 9 |
| A3 | B6 | intensiv dunkelblau | 9 |
| A4 | B1 | rosarot | 9 |
| A4 | B2 | mittelblau | 9 |
| A4 | B6 | dunkelblau/türkis | 9 |
| A4 | B9 | blauschwarz | 6 |
| A4 | B10 | intensiv dunkelrot | 6 |
| A4 | B11 | schwarz | 6 |
| A4 | B15 | leuchtend violett | 6 |
| A4 | B23 | leuchtend rot | 6 |
| A4 | B25 | intensiv violett | 6 |
| A4 | B27 | braun | 6 |
| A4 | B31 | dunkelblau | 6 |
| A4 | B40 | gelborange | 9 |
| A4 | B41 | dunkelblau | 9 |
| A4 | B42 | gelborange | 4 |
| A4 | B43 | dunkelblau | 6 |
| A5 | B9 | dunkelblau | 9 |
| A5 | B32 | grünbraun | 9 |
| A5 | B33 | braunorange | 9 |
| A5 | B34 | schwarz | 9 |
| A5 | B35 | leuchtend violett | 9 |
| A5 | B36 | leuchtend orange | 9 |
| A5 | B37 | dunkelblau | 9 |
| A5 | B38 | graurosa | 9 |
| A5 | B38 | graurosa | 9 |
| A6 | B1 | rosarot | 9 |
| A6 | B2 | intensiv violett | 9 |
| A6 | B6 | intensiv türkisblau | 9 |
| A6 | B8 | rosarot | 9 |
| A6 | B9 | grün | 9 |
| A6 | B12 | rosarot | 9 |
| A6 | B13 | intensiv graublau | 9 |
| A6 | B15 | violett | 9 |

## Patentansprüche

1. Mittel zum Färben von keratinhaltigen Fasern, insbesondere menschlichen Haaren, enthaltend mindestens ein 1,2-Dihydro-pyrimidinium-Derivat gemäß Formel I und/oder dessen Enaminform, 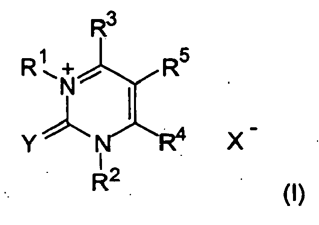 wobei
• R¹ und R² stehen unabhängig voneinander für eine lineare oder cyclische C₁-C₆-Alkylgruppe, eine C₂-C₆-Alkenylgruppe, eine gegebenenfalls substituierte Arylgruppe, eine gegebenenfalls substituierte Heteroarylgruppe, eine Aryl-C₁-C₆-alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe, eine Gruppe R^{I}R^{II}N-(CH₂)ₘ-, worin, R^{I} und R^{II} stehen unabhängig voneinander für ein Wasserstoffatom, eine C₁-C₄-Alkylgruppe, eine C₁-C₄-Hydroxyalkylgruppe oder eine Aryl-C₁-C₆-alkylgruppe,
wobei R^{I} und R^{II} gemeinsam mit dem Stickstoffatom einen 5-, 6- oder 7-gliedrigen Ring bilden können und m steht für eine Zahl 2, 3, 4, 5 oder 6,
• R³ und R⁴ stehen unabhängig voneinander für ein Wasserstoffatom oder eine C₁-C₆-Alkylgruppe, wobei mindestens einer der Reste R³ und R⁴ eine C₁-C₆-Alkylgruppe bedeutet,
• R⁵ steht für ein Wasserstoffatom, eine C₁-C₆-Alkylgruppe, eine C₁-C₆-Hydroxyalkylgruppe, eine C₂-C₆-Polyhydroxyalkylgruppe, eine C₁-C₆-Alkoxygruppe, eine C₁-C₆-Hydroxyalkoxygruppe, eine Gruppe R^{III}R^{IV}N-(CH₂)_{q}-, worin R^{III} und R^{IV} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und q steht für eine Zahl 1, 2, 3, 4, 5 oder 6, wobei der Rest R⁵ zusammen mit einem der Reste R³ oder R⁴ einen 5- oder 6-gliedrigen aromatischen oder aliphatischen Ring bilden kann, der gegebenenfalls mit einem Halogenatom, einer C₁-C₆-Alkylgruppe, einer C₁-C₆-Hydroxyalkylgruppe, einer C₂-C₆-Polyhydroxyalkylgruppe, einer C₁-C₆-Alkoxygruppe, einer C₁-C₆-Hydroxyalkoxygruppe, einer Nitrogruppe, einer Hydroxygruppe, einer Gruppe F^{V}R^{VI}N-(CH₂)ₛ-, worin R^{V} und R^{VI} stehen unabhängig voneinander für ein Wasserstoffatom, eine C₁-C₆-Alkylgruppe, eine C₁-C₆-Hydroxyalkylgruppe oder eine Aryl-C₁-C₆-alkylgruppe und s steht für eine Zahl 0, 1, 2, 3, 4, 5 oder 6 substituiert sein kann,
• Y steht für ein Sauerstoffatom, ein Schwefelatom oder eine Gruppe NR^{VII}, worin R^{VII} steht für ein Wasserstoffatom, eine Arylgruppe, eine Heteroarylgruppe, eine C₁-C₆-Alkylgruppe oder eine C₁-C₆-Arylalkylgruppe,
• X- steht für Halogenid, Benzolsulfonat, p-Toluolsulfonat, C₁-C₄-Alkansulfonat, Trifluormethansulfonat; Perchlorat, 0.5 Sulfat, Hydrogensulfat, Tetrafluoroborat, Hexafluorophosphat oder Tetrachiorozihkat,
und mindestens eine Verbindung mit einer reaktiven Carbonylgruppe (Komponente B).

2. Mittel nach Anspruch 1, **dadurch gekennzeichnet, daß** Y für ein Sauerstoffatom oder ein Schwefelatom steht.

3. Mittel nach einem der Ansprüche 1 oder 2, **dadurch gekennzeichnet, daß** Y für ein Sauerstoffatom steht.

4. Mittel nach einem der Ansprüche 1 bis 3, **dadurch gekennzeichnet, daß** mindestens ein Rest R³ oder R⁴ für eine Methylgruppe steht.

5. Mittel nach einem der Ansprüche 1 bis 4, **dadurch gekennzeichnet, daß** X- steht für Chlorid, Bromid, lodid, Hydrogensulfat oder p-Toluolsulfonat.

6. Mittel nach einem der Ansprüche 1 bis 5, **dadurch gekennzeichnet, daß** die Verbindungen gemäß Formel I ausgewählt sind aus der Gruppe bestehend aus 1,2-Dihydro-1,3,4,6-fetramethyl-2-oxo-pyrimidinium-chlorid, 1,2-Dihydro-1,3-diethyl-4,6-dimethyl-2-oxo-pyrimidinium-chlorid, 1,2-Dihydro-1,3-dipropyl-4,6-dimethyl-2-oxopyrimidinium-chlorid, 1,2-Dihydro-1,3-di(2-hydroxyethyl)-4,6-dimethyl-2-oxopyrimidinium-chlorid, 1,2-Dihydro-1,3-diphenyl-4,6-dimethyl-2-oxo-pyrimidinium-chlorid, 1,2-Dihydro-1,3,4,6-tetramethyl-2-oxo-pyrimidinium-hydrogensulfat, 1,2-Dihydro-1,3-diethyl-4,6-dimethyl-2-oxo-pyrimidinium-hydrogensulfat, 1,2-Dihydro-1,3-dipropyl-4,6-dimethyl-2-oxo-pyrimidinium-hydrogensulfat, 1,2-Dihydro-1,3-di(2-hydroxyethyl)-4,6-dimethyl-2-oxo-pyrimidinium-hydrogensulfat, 1,2-Dihydro-1,3-diphenyl-4,6-dimethyl-2-oxo-pyrimidinium-hydrogensulfat, 1,2-Dihydro-1,3,4-trimethyl-2-oxo-pyrimidinium-chlorid, 1,2-Dihydro-1,3,4-trimethyl-2-oxo-pyrimidinium-hydrogensulfat, 1,2-Dihydro-1,3-diethyl-4-methyl-2-oxo-pyrimidinium-chlorid, 1,2-Dihydro-1,3-diethyl-4-methyl-2-oxo-pyrimidinium-hydrogensulfat, 1,2-Dihydro-1,3-dipropyl-4-methyl-2-oxo-pyrimidinium-chlorid, 1,2-Dihydro-1,3-dipropyl-4-methyl-2-oxo-pyrimidinium-hydrogensulfat, 1,2-Dihydro-1,3-di(2-hydroxyethyl)-4-methyl-2-oxo-pyrimidinium-chlorid, 1,2-Dihydro-1,3-di(2-hydroxyethyl)-4-methyl-2-oxopyrimidinium-hydrogensulfat, 1,2-Dihydro-1,3-diphenyl-4-methyl-2-oxo-pyrimidinium-chlorid, 1,2-Dihydro-1,3-diphenyl-4-methyl-2-oxo-pyrimidinium-hydrogensulfat, 1,2-Dihydro-1,3,4,5,6-pentamethyl-2-oxo-pyrimidinium-chlorid, 1,2-Dihydro-1,3,4,6-tetramethyl-2-thioxo-pyrimidinium-chlorid, 1,2-Dihydro-1,3-diethyl-4,6-dimethyl-2-thioxo-pyrimidinium-chlorid, 1,2-Dihydro-1,3-dipropyl-4,6-dimethyl-2-thioxopyrimidinium-chlorid, 1,2-Dihydro-1,3-di(2-hydroxyethyl)-4,6-dimethyl-2-thioxopyrimidinium-chlorid, 1,2-Dihydro-1,3-diphenyl-4,6-dimethyl-2-thioxo-pyrimidinium-chlorid, 1,2-Dihydro-1,3,4,6-tetramethyl-2-thioxo-pyrimidinium-hydrogensulfat, 1,2-Dihydro-1,3-dipropyl-4,6-dimethyl-2-thioxo-pyrimidinium-hydrogensulfat, 1,2-Dihydro-1,3-di(2-hydroxyethyl)-4,6-dimethyl-2-thioxo-pyrimidinium-hydrogensulfat, 1,2-Dihydro-1,3-diphenyl-4,6-dimethyl-2-thioxo-pyrimidinium-hydrogensulfat, 1,2-Dihydro-1,3,4-thmethyl-2-thioxo-pyrimidinium-chlorid, 1,2-Dihydro-1,3,4-trimethyl-2-thioxo-pyrimidinium-hydrogensulfat, 1,2-Dihydro-1,3-diethyl-4-methyl-2-thioxopyrimidinium-chlorid, 1,2-Dihydro-1,3-diethyl-4-methyl-2-thioxo-pyrimidinium-hydrogensulfat, 1,2-Dihydro-1,3-dipropyl-4-methyl-2-thioxo-pyrimidinium-chlorid, 1,2-Dihydro-1,3-dipropyl-4-methyl-2-thioxo-pyrimidinium-hydrogensulfat, 1,2-Dihydro-1,3-di(2-hydroxyethyl)-4-methyl-2-thioxo-pyrimidinium-chlorid, 1,2-Dihydro-1,3-di(2-hydroxyethyl)-4-methyl-2-thioxo-pyrimidinium-hydrogensulfat, 1,2-Dihydro-1,3-diphenyl-4-methyl-2-thioxo-pyrimidinium-chlorid, 1,2-Dihydro-1,3-diphenyl-4-methyl-2-thioxo-pyrimidinium-hydrogensulfat, 1,2-Dihydro-3,4-dimethyl-2-oxo-chinazoliniumchlorid, 1,2-Dihydro-3,4-dimethyl-2-oxo-chinazolinium-p-toluolsulfonat, 1,2-Dihydro-3,4-dimethyl-2-thioxo-chinazoliniumchlorid, 1,2-Dihydro-3,4-dimethyl-2-thioxo-chinazolinium-p-toluolsulfonat, 1,2-Dihydro-1,3,4-trimethyl-2-thioxo-chinazolinium-hydrogensulfat, 1,3,4-trimethyl-2-oxo-2, 3,5,6,7,8-hexahydrochinazolinium-hydrogensulfat, 1,3,4-Trimethyl-2-thioxo-2,3,5,6,7,8-hexahydrochinazotinium-hydrogensutfat, 1,3,4-Trimethyl-2-oxo-3,5,6,7-tetrahydro-2H-cyclopenta[a]pyrimidinium-hydrogensulfat und 1,3,4-Trimethyl-2-thioxo-3,5,6,7-tetrahydro-2H-cyclopenta[a]pyrimidinium-hydrogensulfat.

7. Mittel nach einem der Ansprüche 1 bis 6, **dadurch gekennzeichnet, daß** die Komponente B ausgewählt ist aus Verbindungen gemäß Formel II 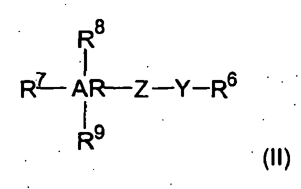 wobei
• AR steht für Benzol, Naphthalin, Pyridin, Pyrimidin, Pyrazin, Pyridazin, Carbazol, Pyrrol, Pyrazol, Furan, Thiophen, 1,2,3-Triazin, 1,3,5-Triazin, Chinolin, Isochinolin, Indol, Indolin, Indolizin, Indan, Imidazol, 1,2,4-Triazol, 1,2,3-Triazol, Tetrazol, Benzimidazol, 1,3-Thiazol, Benzothiazol, Indazol, Benzoxazol, Chinoxalin, Chinazolin, Chinolizin, Cinnolin, Acridin, Julolidin, Acenaphthen, Fluoren, Biphenyl, Diphenylmethan, Benzophenon, Diphenylether, Azobenzol, Chromon, Cumarin, Diphenylamin, Stilben, wobei die N-Heteroaromaten auch quaterniert sein können,
• R⁶ steht für ein Wasserstoffatom, eine C₁-C₆-Alkyl-, C₂-C₆-Acyl-, C₂-C₄-Alkenyl-, C₁-C₄-Perfluoralkyl-, eine ggf. substituierte Aryl- oder Heteroarylgruppe,
• R⁷, R⁸ und R⁹ stehen unabhängig voneinander für ein Wasserstoffatom, ein Halogenatom, eine C₁-C₆-Alkyl-, C₁-C₆-Alkoxy-, C₁-C₆-Aminoalkyl-, C₁-C₆-Hydroxyalkylgruppe, eine C₁-C₆-Alkoxy-C₁-C₆-alkyloxygruppe, eine C₂-C₆-Acylgruppe, eine Acetyl-, eine Carboxyl-, Carboxylato-, Carbamoyl-, Sulfo-, Sulfato-, Sulfonamid-, Sulfonamido-, C₂-C₆-Alkenyl-, eine Aryl-, eine Aryl-C₁-C₆-alkylgruppe, eine Hydroxy-, eine Nitro-, eine Pyrrolidino-, eine Morpholino-, eine Piperidino-, eine Amino- bzw. Ammonio- oder eine 1-Imidazol(in)iogruppe, wobei die letzten drei Gruppen mit einer oder mehrerer C₁-C₆-Alkyl-, C₁-C₆-Carboxyalkyl-, C₁-C₆-Hydroxyalkyl-, C₂-C₆-Alkenyl-, C₁-C₆-Alkoxy-C₁-C₆-alkyl-, mit ggf. substituierten Benzylgruppen, mit Sulfo-(C₁-C₄)-alkyl-, oder Heterozyklus-(C₁-C₄)-alkylgruppen substituiert sein können,
wobei auch zwei der Reste aus R⁷, R⁸, R⁹ und -Z-Y-R⁶ zusammen mit dem Restmolekül einen ankondensierten gegebenenfalls substituierten 5-, 6- oder 7-Ring, der ebenfalls einen ankondensierten aromatischen Ring tragen kann, bilden können, wobei das System AR in Abhängigkeit von der Größe des Ringes weitere Substituenten tragen kann, die unabhängig voneinander für die gleichen Gruppen stehen können wie R⁷, R⁸ und R⁹,
• Z steht für eine direkte Bindung, eine Carbonyl-, eine Carboxy-(C₁-C₄)-alkylen-, eine gegebenenfalls substituierte C₂-C₆-Alkenylen-, C₄-C₆-Alkadienylen-, Furylen-, Thienylen-, Arylen-, Vinylenarylen-, Vinylenfurylen-, Vinylenthienylengruppe, wobei Z zusammen mit der -Y-R⁶-Gruppe auch einen gegebenenfalls substituierten 5-, 6- oder 7-Ring bilden kann,
• Y steht für eine Gruppe, die ausgewählt ist aus Carbonyl, einer Gruppe gemäß Formel III und einer Gruppe gemäß Formel IV, 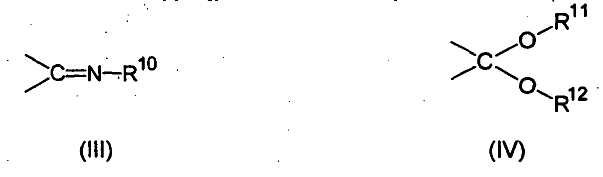 wobei
• R¹⁰ steht für ein Wasserstoffatom, eine Hydroxygruppe, eine C₁-C₄-Alkoxygruppe, eine C₁-C₆-Alkylgruppe, eine C₁-C₆-Hydroxyalkylgruppe, eine C₂-C₆-Polyhydroxyalkylgruppe, eine C₁-C₆-Alkoxy-C₁-C₆-alkylgruppe,
• R¹¹ und R¹² stehen unabängig voneinander für eine C₁-C₆-Alkylgruppe, eine Arylgruppe oder bilden zusammen mit dem Strukturelement O-C-O der Formel IV einen 5- oder 6-gliederigen Ring.

8. Mittel nach einem der Ansprüche 1 bis 7, **dadurch gekennzeichnet, daß** die Komponente B ausgewählt ist aus der Gruppe bestehend aus Acetophenon, Propiophenon, - 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetopherion, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinöacetophenon, 4-lmidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Amiriobenzophenon, 4,4'-Dihydroxybenzophenon, 2,4-Dihydroxy-benzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxybenzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-behzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyl, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzatdehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzatdehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon, Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-l-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Fonnyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethytohinolinium, 5-Formyl-1-benzylchinolinium-, 6-Formyl-1-benzylchinolinium-, 7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazoliurn-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-benzolsulfonat, -p-toluoisulfonat, - methansulfonat; -perchlorat, -sulfat, -chlorid, -bromid, -iodid, -tetrachlorozinkat, - methylsulfat-, trifluormethansulfonat, -tetrafluoroborat, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

9. Mittel nach einem der Ansprüche 1 bis 8, **dadurch gekennzeichnet, daß** die Komponente B ausgewählt ist aus der Gruppe bestehend aus Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxybenzaldehyd, 3-Hydroxy-4-methoxy-benzaldehyd, 2-Hydroxy-4-methoxybenzaldehyd, 2-Ethoxy-4-hydroxy-benzaldehyd, 3-Ethoxy-4-hydroxy-benzaldehyd,-4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3;4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd und Piperonal.

10. Mittel nach einem der Ansprüche 1 bis 9, **dadurch gekennzeichnet, daß** es mindestens eine Verbindung als Komponente C, ausgewählt aus (a) CH-aciden Verbindungen und (b) Verbindungen mit primärer oder sekundärer Amino- oder Hydroxygruppe, ausgewählt aus aromatischen Hydroxyverbindungen, primären oder sekundären aromatischen Aminen und stickstoffhaltigen heterozyklischen Verbindungen, enthält.

11. Mittel nach Anspruch 10, **dadurch gekennzeichnet, daß** die CH-aciden Verbindungen der Komponente C ausgewählt sind aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methyl-naphtho[1,2-d]thiazotium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methylchinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-berizothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

12. Mittel nach Anspruch 10, **dadurch gekennzeichnet, daß** die primären und sekundären aromatischen Amine der komponente C ausgewählt sind aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin,- N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethano), 2,4-Diaminopherioxyethanol, 2-(2,5-Diaminophenoxy)-ettianol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sutfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitröbenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-S-chlor-4-(2-hydroyethylamino)-2-nitrobenzol, aromatische Aniline bzw. Phenole mit einem weiteren aromatischen Rest, wie sie in der Formel VI dargestellt sind 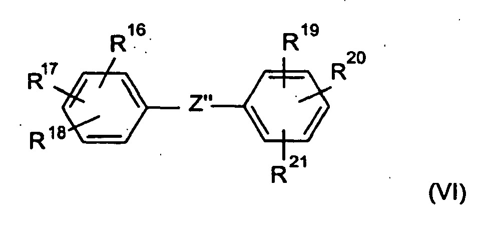 in der
• R¹⁶ für eine Hydroxy- oder eine Aminogruppe, die durch C₁-C₆-Alkyl-, C₁-C₆-Hydroxyalkyl-, C₁-C₆-Alkoxy- oder C₁-C₆-Alkoxy-C₁-C₆-alkylgruppen substituiert sein kann, steht,
• R¹⁷, R¹⁸, R¹⁹, R²⁰ und R²¹ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
• Z" für eine direkte Bindung, eine gesättigte oder ungesättigte, ggf. durch Hydroxygruppen substituierte Kohlenstoffkette mit 1 bis 4 Kohleristoffatomen, eine Carbonyl-, Sulfonyl- oder Iminogruppe, ein Sauerstoff- oder Schwefelatom, oder eine Gruppe mit der Formel VII
**-Q'-(CH₂-Q-CH₂-Q")ₒ- (VII)**
in der
• Q eine direkte Bindung, eine CH₂- oder CHOH-Gruppe bedeutet,
• Q' und Q" unabhängig voneinander für ein Sauerstoffatom, eine NR²²-Gruppe, worin R²² ein Wasserstoffatom, eine C₁-C₆-Alkylgruppe oder C₁-C₆-Hydroxyalkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
• o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan

13. Mittel nach Anspruch 10, **dadurch gekennzeichnet, daß** die aromatischen Hydroxyverbindungen der Komponente C ausgewählt sind aus der Gruppe bestehend aus 2-, 4-, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-, 3-, 4-Methoxy-, 3-Dimethylamino-, 2-(2-Hydroxyethyl)-, 3,4-Methylendioxyphenol, 2,4-, 3,4-Dihydroxybenzoesäure, -phenylessigsäure, Gallussäure, 2,4,6-Trihydroxybenzoesäure, -acetophenon, 2-, 4-Chlorresorcin, 1-Naphthol, 1,5-, 2,3-, 2,7-Dihydroxynaphthalin, 6-Dimethylamino-4-hydroxy-2-naphthalinsulfonsäure und 3,6-Dihydroxy-2,7-naphthalinsulfonsäure.

14. Mittel nach Anspruch 10, **dadurch gekennzeichnet, daß** die stickstoffhaltigen heterocyclischen Verbindungen der Komponente C ausgewählt sind aus der Gruppe bestehend aus 2-Aminopyridin, 3-Aminopyridin, 4-Aminopyridin, 2-Amino-3-hydroxypyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6-Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochihaldin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin, 4-Hydroxyindolin und Hydroxypyrimidin-Derivate und die physiologisch verträglichen Salze der vorgenannten Verbindungen.

15. Mittel nach einem der Ansprüche 1 bis 14, **dadurch gekennzeichnet, daß** die Verbindungen der Formel I, die Verbindungen der Komponente B und die Verbindungen der Komponente C jeweils in einer Menge von 0,03 bis 65 mmol, insbesondere von 1 bis 40 mmol, bezogen auf 100 g des gesamten Färbemittels, enthalten sind.

16. Mittel nach einem der Ansprüche 1 bis 15, **dadurch gekennzeichnet, daß** es mindestens ein Reaktionsprodukt RP gemäß Formel VIII, 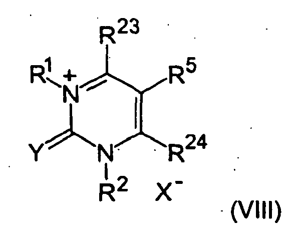 worin
• R¹, R², R⁵, Y und X⁻ gemäß Anspruch 1 definiert sind,
• R²³ und R²⁴ unabhängig voneinander stehen für ein Wasserstoffatom, eine C₁-C₆-Alkylgruppe, eine Gruppe gemäß Formel IX, 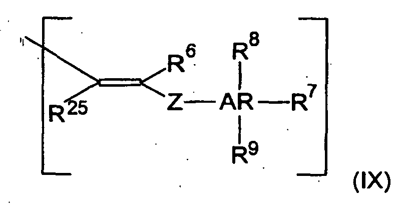 worin
• R⁶, R⁷, R⁸, R⁹, AR und Z gemäß Anspruch 7 definiert ist,
• R²⁵ steht für ein Wasserstoffatom oder eine C₁-C₅-Alkylgruppe,
mit der Maßgabe, daß mindestens einer der Reste R²³ und R²⁴ für eine Gruppe gemäß Formel IX steht.

17. Mittel nach Anspruch 16, **dadurch gekennzeichnet, daß** AR gemäß Formel IX für Benzol oder Naphthalin steht.

18. Mittel nach einem der Ansprüche 16 oder 17, **dadurch gekennzeichnet, daß** Z gemäß Formel IX für eine direkte Bindung oder Vinylen steht.

19. Mittel nach einem der Ansprüche 16 bis 18, **dadurch gekennzeichnet, daß** R²⁵ gemäß Formel IX für ein Wasserstoffatom steht.

20. Mittel nach einem der Ansprüche 16 bis 19, **dadurch gekennzeichnet, daß** R⁷, R⁸ und R⁹ gemäß Formel IX unabhängig voneinander für ein Wasserstoffatom, ein Halogenatom, eine C₁-C₆-Alkylgruppe, eine Hydroxygruppe, eine C₁-C₆-Alkoxygruppe, eine Aminogruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonamidgruppe, eine Carbamoylgruppe, eine C₂-C₆-Acylgruppe, eine Acetylgruppe, eine Sulfonsäuregruppe, eine Sulfonamidogruppe oder eine Nitrogruppe stehen.

21. Mittel nach einem der Ansprüche 16 bis 20, **dadurch gekennzeichnet, daß** das Reaktionsprodukt RP in einer Menge von 0,03 bis 65 mmol, insbesondere von 1 bis 40 mmol, bezogen auf 100 g des gesamten Färbemittels, enthalten ist.

22. Mittel nach einem der Ansprüche 1 bis 21, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

23. Mittel nach einem der Ansprüche 1 bis 22, **dadurch gekennzeichnet, daß** es mindestens einen direktziehenden Farbstoff, vorzugsweise in einer Menge von 0,01 bis 20 Gew.-%, bezogen auf das gesamte Färbemittel, enthält.

24. Mittel nach einem der Ansprüche 1 bis 23, **dadurch gekennzeichnet, daß** es mindestens eine Entwicklerkomponente und gegebenenfalls mindestens eine Kupplerkomponente als Oxidationsfarbstoffvorprodukt enthält.

25. Mittel nach einem der Ansprüche 1 bis 24, **dadurch gekennzeichnet, daß** Ammonium- oder Metallsalze ausgewählt aus der Gruppe der Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, zugegeben werden.

26. Mittel nach einem der Ansprüche 1 bis 25, **dadurch gekennzeichnet, daß** es Oxidationsmittel, insbesondere H₂O₂, in einer Menge von 0,01 bis 6 Gew.-%, bezogen auf die Anwendungslösung, enthält.

27. Mittel nach einem der Ansprüche 1 bis 26, **dadurch gekennzeichnet, daß** es anionische, zwitterionische oder nichtionische Tenside enthält.

28. Verwendung von mindestens einer Verbindung gemäß Formel I gemäß Anspruch 1, in Kombination mit mindestens einer Verbindung der Komponente B gemäß Anspruch 1, als eine färbende Komponente in Haarfärbemitteln.

29. Verfahren zum Färben von keratinhaltigen Fasern, insbesondere menschlichen Haaren, worin ein Färbemittel, gemäß Anspruch 1, sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird.

30. Verfahren gemäß Anspruch 29, **dadurch gekennzeichnet, daß** in einem Zweischrittverfahren die Komponente B vor oder nach Applikation der Verbindung gemäß Formel I auf die keratinhaltigen Fasern aufgebracht, die auf dem Haar erhaltene Mischung einige Zeit, üblicherweise ca. 15-30 Minuten, auf der Faser belassen und anschließend wieder ausgespült oder mit einem Shampoo ausgewaschen wird.

31. Verfahren nach einem der Ansprüche 29 oder 30, **dadurch gekennzeichnet, daß** die keratinhaltigen Fasern, bevor ein Färbemittel gemäß Anspruch 1 zur Anwendung kommt, im Rahmen einer Vorbehandlung mit einem Blondiermittel blondiert oder mit einem Oxidationsfärbemittel gefärbt wurden.

32. Verwendung von mindestens einem 1,2-Dihydro-pyrimidinium-Derivat gemäß Formel I und/oder dessen Enaminform, 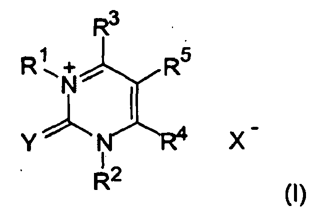 wobei R¹, R², R³, R⁴, R⁵, Y und X⁻ wie in Anspruch 1 definiert sind in Kombination mit reaktiven Carbonylverbindungen (Komponente B), zur Nuancierung von Oxidationsfärbungen von keratinhaltigen Fasern, insbesondere menschlichen Haaren.

33. Verwendung von mindestens einem 1,2-Dihydro-pyrimidinium-Derivat gemäß Formel I und/oder dessen Enaminform, 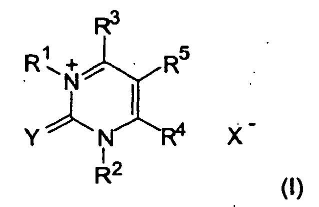 wobei R¹, R², R³, R⁴, R⁶, Y und X- wie in Anspruch 1 definiert sind in Kombination mit mindestens einer reaktiven Carbonylverbindung (Komponente B), zur Farbauffrischung von mit oxidativen Färbemitteln gefärbten keratinhaltigen Fasern.

## Claims

1. Agent for dyeing keratinous fibres, in particular human hair, comprising at least one 1,2-dihydropyrimidinium derivative according to formula I and/or its enamine form, 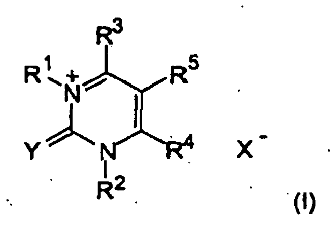 where
• R¹ and R², independently of one another, are a linear or cyclic C₁-C₆-alkyl group, a C₂-C₆-alkenyl group, an optionally substituted aryl group, an optionally substituted heteroaryl group, an aryl-C₁-C₆-alkyl group, a C₁-C₆-hydroxyalkyl group, a C₂-C₆-polyhydroxyalkyl group, a C₁-C₆-alkoxy-C₁-C₆-alkyl group, a group R^{I}R^{II}N-(CH₂)ₘ-, in which R^{I} and R^{II}, independently of one another, are a hydrogen atom, a C₁-C₄-alkyl group, a C₁-C₄-hydroxyalkyl group or an aryl-C₁-C₆-alkyl group, where R^{I} and R^{II}, together with the nitrogen atom, can form a 5-, 6- or 7-membered ring and m is a number 2, 3, 4, 5 or 6,
• R³ and R⁴, independently of one another, are a hydrogen atom or a C₁-C₆-alkyl group, where at least one of the radicals R³ and R⁴ is a C₁-C₆-alkyl group,
• R⁵ is a hydrogen atom, a C₁-C₆-alkyl group, a C₁-C₆-hydroxyalkyl group, a C₂-C₆-polyhydroxyalkyl group, a C₁-C₆-alkoxy group, a C₁-C₆-hydroxyalkoxy group, a group R^{III}R^{IV}N-(CH₂)_{q}-, in which R^{III} and R^{IV}, independently of one another, are a hydrogen atom, a C₁-C₆-alkyl group, a C₁-C₆-hydroxyalkyl group or an aryl-C₁-C₆-alkyl group and q is a number 1, 2, 3, 4, 5 or 6, where the radical R⁵, together with one of the radicals R³ or R⁴, can form a 5- or 6-membered aromatic or aliphatic ring, which may optionally be substituted by a halogen atom, a C₁-C₆-alkyl group, a C₁-C₆-hydroxyalkyl group, a C₂-C₆-polyhydroxyalkyl group, a C₁-C₆-alkoxy group, a C₁-C₆-hydroxyalkoxy group, a nitro group, a hydroxy group, a group R^{V}R^{VI}N-(CH₂)ₛ-, in which R^{V} and R^{VI}, independently of one another, are a hydrogen atom, a C₁-C₆-alkyl group, a C₁-C₆-hydroxyalkyl group or an aryl-C₁-C₆-alkyl group and s is a number 0, 1, 2, 3, 4, 5 or 6,
• Y is an oxygen atom, a sulphur atom or a group NR^{VII}, in which R^{VII} is a hydrogen atom, an aryl group, a heteroaryl group, a C₁-C₆-alkyl group or a C₁-C₆-arylalkyl group,
• X⁻ is halide, benzenesulphonate, p-toluenesulphonate, C₁-C₄-alkanesulphonate, trifluoromethanesulphonate, perchlorate, 0.5 sulphate, hydrogensulphate, tetrafluoroborate, hexafluorophosphate or tetrachlorozincate,
and at least one compound with a reactive carbonyl group (component B).

2. Agent according to Claim 1, **characterized in that** Y is an oxygen atom or a sulphur atom.

3. Agent according to one of Claims 1 or 2, **characterized in that** Y is an oxygen atom.

4. Agent according to one of Claims 1 to 3, **characterized in that** at least one radical R³ or R⁴ is a methyl group.

5. Agent according to one of Claims 1 to 4, **characterized in that** X⁻ is chloride, bromide, iodide, hydrogensulphate or p-toluenesulphonate.

6. Agent according to one of Claims 1 to 5, **characterized in that** the compounds according to formula I are chosen from the group consisting of 1,2-dihydro-1,3,4,6-tetramethyl-2-oxopyrimidinium chloride, 1,2-dihydro-1,3-diethyl-4,6-dimethyl-2-oxopyrimidinium chloride, 1,2-dihydro-1,3-dipropyl-4,6-dimethyl-2-oxopyrimidinium chloride, 1,2-dihydro-1,3-di(2-hydroxyethyl)-4,6-dimethyl-2-oxopyrimidinium chloride, 1,2-dihydro-1,3-diphenyl-4,6-dimethyl-2-oxopyrimidinium chloride, 1,2-dihydro-1,3,4,6-tetramethyl-2-oxopyrimidinium hydrogensulphate, 1,2-dihydro-1,3-diethyl-4,6-dimethyl-2-oxopyrimidinium hydrogensulphate, 1,2-dihydro-1,3-dipropyl-4,6-dimethyl-2-oxopyrimidinium hydrogensulphate, 1,2-dihydro-1,3-di(2-hydroxyethyl)-4,6-dimethyl-2-oxopyrimidinium hydrogensulphate, 1,2-dihydro-1,3-diphenyl-4,6-dimethyl-2-oxopyrimidinium hydrogensulphate, 1,2-dihydro-1,3,4-trimethyl-2-oxopyrimidinium chloride, 1,2-dihydro-1,3,4-trimethyl-2-oxopyrimidinium hydrogensulphate, 1,2-dihydro-1,3-diethyl-4-methyl-2-oxopyrimidinium chloride, 1,2-dihydro-1,3-diethyl-4-methyl-2-oxopyrimidinium hydrogensulphate, 1,2-dihydro-1,3-dipropyl-4-methyl-2-oxopyrimidinium chloride, 1,2-dihydro-1,3-dipropyl-4-methyl-2-oxopyrimidinium hydrogensulphate, 1,2-dihydro-1,3-di(2-hydroxyethyl)-4-methyl-2-oxopyrimidinium chloride, 1,2-dihydro-1,3-di(2-hydroxyethyl)-4-methyl-2-oxopyrimidinium hydrogensulphate, 1,2-dihydro-1,3-diphenyl-4-methyl-2-oxopyrimidinium chloride, 1,2-dihydro-1,3-diphenyl-4-methyl-2-oxopyrimidinium hydrogensulphate, 1,2-dihydro-1,3,4,5,6-pentamethyl-2-oxopyrimidinium chloride, 1,2-dihydro-1,3,4,6-tetramethyl-2-thioxopyrimidinium chloride, 1,2-dihydro-1,3-diethyl-4,6-dimethyl-2-thioxopyrimidinium chloride, 1,2-dihydro-1,3-dipropyl-4,6-dimethyl-2-thioxopyrimidinium chloride, 1,2-dihydro-1,3-di(2-hydroxyethyl)-4,6-dimethyl-2-thioxopyrimidinium chloride, 1,2-dihydro-1,3-diphenyl-4,6-dimethyl-2-thioxopyrimidinium chloride, 1,2-dihydro-1,3,4,6-tetramethyl-2-thioxopyrimidinium hydrogensulphate, 1,2-dihydro-1,3-dipropyl-4,6-dimethyl-2-thioxopyrimidinium hydrogensulphate, 1,2-dihydro-1,3-di(2-hydroxyethyl)-4,6-dimethyl-2-thioxopyrimidinium hydrogensulphate, 1,2-dihydro-1,3-diphenyl-4,6-dimethyl-2-thioxopyrimidinium hydrogensulphate, 1,2-dihydro-1,3,4-trimethyl-2-thioxopyrimidinium chloride, 1,2-dihydro-1,3,4-trimethyl-2-thioxopyrimidinium hydrogensulphate, 1,2-dihydro-1,3-diethyl-4-methyl-2-thioxopyrimidinium chloride, 1,2-dihydro-1,3-diethyl-4-methyl-2-thioxopyrimidinium hydrogensulphate, 1,2-dihydro-1,3-dipropyl-4-methyl-2-thioxopyrimidinium chloride, 1,2-dihydro-1,3-dipropyl-4-methyl-2-thioxopyrimidinium hydrogensulphate, 1,2-dihydro-1,3-di(2-hydroxyethyl)-4-methyl-2-thioxopyrimidinium chloride, 1,2-dihydro-1,3-di(2-hydroxyethyl)-4-methyl-2-thioxopyrimidinium hydrogensulphate, 1,2-dihydro-1,3-diphenyl-4-methyl-2-thioxopyrimidinium chloride, 1,2-dihydro-1,3-diphenyl-4-methyl-2-thioxopyrimidinium hydrogensulphate, 1,2-dihydro-3,4-dimethyl-2-oxoquinazolinium chloride, 1,2-dihydro-3,4-dimethyl-2-oxoquinazolinium p-toluenesulphonate, 1,2-dihydro-3,4-dimethyl-2-thioxoquinazolinium chloride, 1,2-dihydro-3,4-dimethyl-2-thioxoquinazolinium p-toluenesulphonate, 1,2-dihydro-1,3,4-trimethyl-2-thioxoquinazolinium hydrogensulphate, 1,3,4-trimethyl-2-oxo-2,3,5,6,7,8-hexahydroquinazolinium hydrogensulphate, 1,3,4-trimethyl-2-thioxo-2,3,5,6,7,8-hexahydroquinazolinium hydrogensulphate, 1,3,4-trimethyl-2-oxo-3,5,6,7-tetrahydro-2H-cyclopenta[a]pyrimidinium hydrogensulphate and 1,3,4-trimethyl-2-thioxo-3,5,6,7-tetrahydro-2H-cyclopenta[a]pyrimidinium hydrogensulphate.

7. Agent according to one of Claims 1 to 6, **characterized in that** component B is chosen from compounds according to formula II 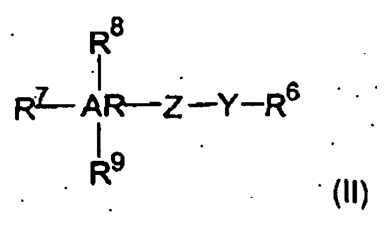 where
• AR is benzene, naphthalene, pyridine, pyrimidine, pyrazine, pyridazine, carbazole, pyrrole, pyrazole, furan, thiophene, 1,2,3-triazine, 1,3,5-triazine, quinoline, isoquinoline, indole, indoline, indolizine, indane, imidazole, 1,2,4-triazole, 1,2,3-triazole, tetrazole, benzimidazole, 1,3-thiazole, benzothiazole, indazole, benzoxazole, quinoxaline, quinazoline, quinolizine, cinnoline, acridine, julolidine, acenaphthene, fluorene, biphenyl, diphenylmethane, benzophenone, diphenyl ether, azobenzene, chromone, coumarin, diphenylamine, stilbene, where the N-heteroaromatics may also be quaternized,
• R⁶ is a hydrogen atom, a C₁-C₆-alkyl group, C₂-C₆-acyl group, C₂-C₄-alkenyl group, C₁-C₄-perfluoroalkyl group, an optionally substituted aryl or heteroaryl group,
• R⁷, R⁸ and R⁹, independently of one another, are a hydrogen atom, a halogen atom, a C₁-C₆-alkyl group, C₁-C₆-alkoxy group, C₁-C₆-aminoalkyl group, C₁-C₆-hydroxyalkyl group, a C₁-C₆-alkoxy-C₁-C₆-alkyloxy group, a C₂-C₆-acyl group, an acetyl group, carboxyl group, carboxylato group, carbamoyl group, sulpho group, sulphato group, sulphonamide group, sulphonamido group, C₂-C₆-alkenyl group, an aryl group, an aryl-C₁-C₆-alkyl group, a hydroxy group, a nitro group, a pyrrolidino group, a morpholino group, a piperidino group, an amino group or ammonio group or a 1-imidazol(in)io group, where the last three groups can be substituted by one or more C₁-C₆-alkyl groups, C₁-C₆-carboxyalkyl groups, C₁-C₆-hydroxyalkyl groups, C₂-C₆-alkenyl groups, C₁-C₆-alkoxy-C₁-C₆-alkyl groups, by optionally substituted benzyl groups, by sulpho-(C₁-C₄)-alkyl groups or heterocycle-(C₁-C₄)-alkyl groups,
where two of the radicals from R⁷, R⁸, R⁹ and -Z-Y-R⁶, together with the remainder of the radical, can also form a fused-on optionally substituted 5-, 6- or 7-membered ring, which can likewise carry a fused-on aromatic ring, where the system AR can, depending on the size of the ring, carry further substituents which, independently of one another, can be the same groups as R⁷, R⁸ and R⁹,
• Z is a direct bond, a carbonyl group, a carboxy-(C₁-C₄)-alkylene group, an optionally substituted C₂-C₆-alkenylene group, C₄-C₆-alkadienylene group, furylene group, thienylene group, arylene group, vinylenearylene group, vinylenefurylene group, vinylenethienylene group, where Z, together with the -Y-R⁶ group, can also form an optionally substituted 5-, 6- or 7-membered ring,
• Y is a group which is chosen from carbonyl, a group according to formula III and a group according to formula IV, 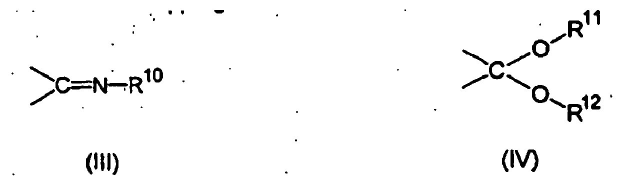 where
• R¹⁰ is a hydrogen atom, a hydroxy group, a C₁-C₄-alkoxy group, a C₁-C₆-alkyl group, a C₁-C₆-hydroxyalkyl group, a C₂-C₆-polyhydroxyalkyl group, a C₁-C₆-alkoxy- C₁-C₆-alkyl group,
• R¹¹ and R¹², independently of one another, are a C₁-C₆-alkyl group, an aryl group or form, together with the structural element O-C-O of the formula IV, a 5- or 6-membered ring.

8. Agent according to one of Claims 1 to 7, **characterized in that** component B is chosen from the group consisting of acetophenone, propiophenone, 2-hydroxyacetophenone, 3-hydroxyacetophenone, 4-hydroxyacetophenone, 2-hydroxypropiophenone, 3-hydroxypropiophenone, 4-hydroxypropiophenone, 2-hydroxybutyrophenone, 3-hydroxybutyrophenone, 4-hydroxybutyrophenone, 2,4-dihydroxyacetophenone, 2,5-dihydroxyacetophenone, 2,6-dihydroxyacetophenone, 2,3,4-trihydroxyacetophenone, 3,4,5-trihydroxyacetophenone, 2,4,6-trihydroxyacetophenone, 2,4,6-trimethoxyacetophenone, 3,4,5-trimethoxyacetophenone, 3,4,5-trimethoxyacetophenone diethyl ketal, 4-hydroxy-3-methoxyacetophenone, 3,5-dimethoxy-4-hydroxyacetophenone, 4-aminoacetophenone, 4-dimethylaminoacetophenone, 4-morpholinoacetophenone, 4-piperidinoacetophenone, 4-imidazolinoacetophenone, 2-hydroxy-5-bromoacetophenone, 4-hydroxy-3-nitroacetophenone, acetophenone-2-carboxylic acid, acetophenone-4-carboxylic acid, benzophenone, 4-hydroxybenzophenone, 2-aminobenzophenone, 4,4'-dihydroxybenzophenone, 2,4-dihydroxybenzophenone, 2,4,4'-trihydroxybenzophenone, 2,3,4-trihydroxybenzophenone, 2-hydroxy-1-acetonaphthone, 1-hydroxy-2-acetonaphthone, chromone, chromone-2-carboxylic acid, flavone, 3-hydroxyflavone, 3,5,7-trihydroxyflavone, 4',5,7-trihydroxyflavone, 5,6,7-trihydroxyflavone, quercetin, 1-indanone, 9-fluorenone, 3-hydroxyfluorenone, anthrone, 1,8-dihydroxyanthrone, vanillin, coniferyl aldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-3-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 4-hydroxy-3,5-dimethoxybenzaldehyde, 4-hydroxy-3,5-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxybenzaldehyde, 2,5-dihydroxybenzaldehyde, 2,6-dihydroxybenzaldehyde, 3,4-dihydroxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 4-hydroxy-2-methoxybenzaldehyde, 4-dimethylaminobenzaldehyde, 4-diethylaminobenzaldehyde, 4-dimethylamino-2-hydroxybenzaldehyde, 4-diethylamino-2-hydroxybenzaldehyde, 4-pyrrolidinobenzaldehyde, 4-morpholinobenzaldehyde, 2-morpholinobenzaldehyde, 4-piperidinobenzaldehyde, 2-methoxy-1-naphthaldehyde, 4-methoxy-1-naphthaldehyde, 2-hydroxy-1-naphthaldehyde, 2,4-dihydroxy-1-naphthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-hydroxy-1-naphthaldehyde, 4-dimethylamino-1-naphthaldehyde, 4-dimethylaminocinnamaldehyde, 2-dimethylaminobenzaldehyde, 2-chloro-4-dimethylaminobenzaldehyde, 4-dimethylamino-2-methylbenzaldehyde, 4-diethylaminocinnamaldehyde, 4-dibutylaminobenzaldehyde, 4-diphenylaminobenzaldehyde, 4-dimethylamino-2-methoxybenzaldehyde, 4-(1-imidazolyl)benzaldehyde, piperonal, 2,3,6,7-tetrahydro-1H,5H-benzo[ij]quinolizine-9-carboxaldehyde, 2,3,6,7-tetrahydro-8-hydroxy-1H,5H-benzo[ij]quinolizine-9-carboxaldehyde, N-ethylcarbazole-3-aldehyde, 2-formylmethylene-1,3,3-trimethylindoline (Fischers aldehyde or tribase aldehyde), 2-indolealdehyde, 3-indolealdehyde, 1-methylindole-3-aldehyde, 2-methylindole-3-aldehyde, 1-acetylindole-3-aldehyde, 3-acetylindole, 1-methyl-3-acetylindole, 2-(1',3',3'-trimethyl-2-indolinylidene)acetaldehyde, 1-methylpyrrole-2-aldehyde, 1-methyl-2-acetylpyrrole, 4-pyridinealdehyde, 2-pyridinealdehyde, 3-pyridinealdehyde, 4-acetylpyridine, 2-acetylpyridine, 3-acetylpyridine, pyridoxal, quinoline-3-aldehyde, quinoline-4-aldehyde, antipyrine-4-aldehyde, furfural, 5-nitrofurfural, 2-thenoyltrifluoroacetone, chromone-3-aldehyde, 3-(5'-nitro-2'-furyl)acrolein, 3-(2'-furyl)acrolein and imidazole-2-aldehyde, 1,3-diacetylbenzene, 1,4-diacetylbenzene, 1,3,5-triacetylbenzene, 2-benzoylacetophenone, 2-(4'-methoxybenzoyl)acetophenone, 2-(2'-furoyl)acetophenone, 2-(2'-pyridoyl)acetophenone and 2-(3'-pyridoyl)acetophenone, benzylidene acetone, 4-hydroxybenzylidene acetone, 2-hydroxybenzylidene acetone, 4-methoxybenzylidene acetone, 4-hydroxy-3-methoxybenzylidene acetone, 4-dimethylaminobenzylidene acetone, 3,4-methylenedioxybenzylidene acetone, 4-pyrrolidinobenzylidene acetone, 4-piperidinobenzylidene acetone, 4-morpholinobenzylidene acetone, 4-diethylaminobenzylidene acetone, 3-benzylidene-2,4-pentanedione, 3-(4'-hydroxybenzylidene)-2,4-pentanedione, 3-(4'-dimethylaminobenzylidene)-2,4-pentanedione, 2-benzylidenecyclohexanone, 2-(4'-hydroxybenzylidene)cyclohexanone, 2-(4'-dimethylaminobenzylidene)cyclohexanone, 2-benzylidene-1,3-cyclohexanedione, 2-(4'-hydroxybenzylidene)-1,3-cyclohexanedione, 3-(4'-dimethylaminobenzylidene)-1,3-cyclohexanedione, 2-benzylidene-5,5-dimethyl-1,3-cyclohexanedione, 2-(4'-hydroxybenzylidene)-5,5-dimethyl-1,3-cyclohexanedione, 2-(4'-hydroxy-3-methoxybenzylidene)-5,5-dimethyl-1,3-cyclohexanedione, 2-(4'-dimethylaminobenzylidene)-5,5-dimethyl-1,3-cyclohexanedione, 2-benzylidenecyclopentanone, 2'-(4-hydroxybenzylidene)cyclopentanone, 2-(4'-dimethylaminobenzylidene)cyclopentanone, 5-(4-dimethylaminophenyl)penta-2,4-dienal, 5-(4-diethylaminophenyl)penta-2,4-dienal, 5-(4-methoxyphenyl)penta-2,4-dienal, 5-(3,4-dimethoxyphenyl)penta-2,4-dienal, 5-(2,4-dimethoxyphenyl)penta-2,4-dienal, 5-(4-piperidinophenyl)penta-2,4-dienal, 5-(4-morpholinophenyl)penta-2,4-dienal, 5-(4-pyrrolidinophenyl)penta-2,4-dienal, 6-(4-dimethylaminophenyl)hexa-3,5-dien-2-one, 6-(4-diethylaminophenyl)hexa-3,5-dien-2-one, 6-(4-methoxyphenyl)hexa-3,5-dien-2-one, 6-(3,4-dimethoxyphenyl)hexa-3,5-dien-2-one, 6-(2,4-dimethoxyphenyl)hexa-3,5-dien-2-one, 6-(4-piperidinophenyl)hexa-3,5-dien-2-one, 6-(4-morpholinophenyl)hexa-3,5-dien-2-one, 6-(4-pyrrolidinophenyl)hexa-3,5-dien-2-one, 5-(4-dimethylamino-1-naphthyl)penta-3,5-dienal, 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 4-hydroxy-3-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 6-nitropiperonal, 2-nitropiperonal, 5-nitrovanillin, 2,5-dinitrosalicylaldehyde, 5-bromo-3-nitrosalicylaldehyde, 3-nitro-4-formylbenzenesulphonic acid, 4-nitro-1-naphthaldehyde, 2-nitrocinnamaldehyde, 3-nitrocinnamaldehyde, 4-nitrocinnamaldehyde, 9-methyl-3-carbazolealdehyde, 9-ethyl-3-carbazolealdehyde, 3-acetylcarbazole, 3,6-diacetyl-9-ethylcarbazole, 3-acetyl-9-methylcarbazole, 1,4-dimethyl-3-carbazolealdehyde, 1,4,9-trimethyl-3-carbazolealdehyde, 4-formyl-1-methylpyridinium, 2-formyl-1-methylpyridinium, 4-formyl-1-ethylpyridinium, 2-formyl-1-ethylpyridinium, 4-formyl-1-benzylpyridinium, 2-formyl-1-benzylpyridinium, 4-formyl-1,2-dimethylpyridinium, 4-formyl-1,3-dimethylpyridinium, 4-formyl-1-methylquinolinium, 2-formyl-1-methylquinolinium, 4-acetyl-1-methylpyridinium, 2-acetyl-1-methylpyridinium, 4-acetyl-1-methylquinolinium, 5-formyl-1-methylquinolinium, 6-formyl-1-methylquinolinium, 7-formyl-1-methylquinolinium, 8-formyl-1-methylquinolinium, 5-formyl-1-ethylquinolinium, 6-formyl-1-ethylquinolinium, 7-formyl-1-ethylquinolinium, 8-formyl-1-ethylquinolinium, 5-formyl-1-benzylquinolinium, 6-formyl-1-benzylquinolinium, 7-formyl-1-benzylquinolinium, 8-formyl-1-benzylquinolinium, 5-formyl-1-allylquinolinium, 6-formyl-1-allylquinolinium, 7-formyl-1-allylquinolinium and 8-formyl-1-allylquinolinium, 5-acetyl-1-methylquinolinium, 6-acetyl-1-methylquinolinium, 7-acetyl-1-methylquinolinium, 8-acetyl-1-methylquinolinium, 5-acetyl-1-ethylquinolinium, 6-acetyl-1-ethylquinolinium, 7-acetyl-1-ethylquinolinium, 8-acetyl-1-ethylquinolinium, 5-acetyl-1-benzylquinolinium, 6-acetyl-1-benzylquinolinium, 7-acetyl-1-benzylquinolinium, 8-acetyl-1-benzylquinolinium, 5-acetyl-1-allylquinolinium, 6-acetyl-1-allylquinolinium, 7-acetyl-1-allylquinolinium and 8-acetyl-1-allylquinolinium, 9-formyl-10-methylacridinium, 4-(2'-formylvinyl)-1-methylpyridinium, 1,3-dimethyl-2-(4'-formylphenyl)benzimidazolium, 1,3-dimethyl-2-(4'-formylphenyl)imidazolium, 2-(4'-formylphenyl)-3-methylbenzothiazolium, 2-(4'-acetylphenyl)-3-methylbenzothiazolium, 2-(4'-formylphenyl)-3-methylbenzoxazolium, 2-(5'-formyl-2'-furyl)-3-methylbenzothiazolium, 2-(5'-formyl-2'-furyl)-3-methylbenzothiazolium, 2-(5'-formyl-2'-thienyl)-3-methylbenzothiazolium, 2-(3'-formylphenyl)-3-methylbenzothiazolium, 2-(4'-formyl-1-naphthyl)-3-methylbenzothiazolium, 5-chloro-2-(4'-formylphenyl)-3-methylbenzothiazolium, 2-(4'-formylphenyl)-3,5-dimethylbenzothiazolium benzenesulphonate, p-toluenesulphonate, methanesulphonate, perchlorate, sulphate, chloride, bromide, iodide, tetrachlorozincate, methylsulphate, trifluoromethanesulphonate, tetrafluoroborate, isatin, 1-methylisatin, 1-allylisatin, 1-hydroxymethylisatin, 5-chloroisatin, 5-methoxyisatin, 5-nitroisatin, 6-nitroisatin, 5-sulphoisatin, 5-carboxyisatin, quinisatin, 1-methylquinisatin, and any mixtures of the above compounds.

9. Agent according to one of Claims 1 to 8, **characterized in that** component B is chosen from the group consisting of vanillin, coniferylaldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-3-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 4-hydroxy-3,5-dimethoxybenzaldehyde, 4-hydroxy-3,5-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyde, 2,4-dihydroxybenzaldehyde, 2,5-dihydroxybenzaldehyde, 2,6-dihydroxybenzaldehyde, 3,4-dihydroxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 4-hydroxy-2-methoxybenzaldehyde, 4-dimethylaminobenzaldehyde, 4-diethylaminobenzaldehyde, 4-dimethylamino-2-hydroxybenzaldehyde, 4-diethylamino-2-hydroxybenzaldehyde, 4-pyrrolidinobenzaldehyde, 4-morpholinobenzaldehyde, 2-morpholinobenzaldehyde, 4-piperidinobenzaldehyde, 2-methoxy-1-naphthaldehyde, 4-methoxy-1-naphthaldehyde, 2-hydroxy-1-naphthaldehyde, 2,4-dihydroxy-1-naphthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-hydroxy-1-naphthaldehyde, 4-dimethylamino-1-naphthaldehyde, 4-dimethylaminocinnamaldehyde, 2-dimethylaminobenzaldehyde, 2-chloro-4-dimethylaminobenzaldehyde, 4-dimethylamino-2-methylbenzaldehyde, 4-diethylaminocinnamaldehyde, 4-dibutylaminobenzaldehyde, 4-diphenylaminobenzaldehyde, 4-dimethylamino-2-methoxybenzaldehyde, 4-(1-imidazolyl)benzaldehyde and piperonal.

10. Agent according to one of Claims 1 to 9, **characterized in that** it comprises at least one compound as component C, chosen from (a) CH-acidic compounds and (b) compounds with primary or secondary amino or hydroxy group, chosen from aromatic hydroxy compounds, primary or secondary aromatic amines and nitrogen-containing heterocyclic compounds.

11. Agent according to Claim 10, **characterized in that** the CH-acidic compounds of component C are chosen from the group consisting of 1,2,3,3-tetramethyl-3H-indolium iodide, 1,2,3,3-tetramethyl-3H-indolium p-toluenesulphonate, 1,2,3,3-tetramethyl-3H-indolium methanesulphonate, 1,3,3-trimethyl-2-methyleneindoline (Fischer's base), 2,3-dimethylbenzothiazolium iodide, 2,3-dimethylbenzothiazolium p-toluenesulphonate, 2,3-dimethylnaphtho[1,2-d]thiazolium p-toluenesulphonate, 3-ethyl-2-methylnaphtho[1,2-d]thiazolium p-toluenesulphonate, rhodanine, rhodanine-3-acetic acid, 1,4-dimethylquinolinium iodide, 1,2-dimethylquinolinium iodide, barbituric acid, thiobarbituric acid, 1,3-dimethylthiobarbituric acid, 1,3-diethylthiobarbituric acid, 1,3-diethylbarbituric acid, oxindole, 3-indoxyl acetate, 2-coumaranone, 5-hydroxy-2-coumaranone, 6-hydroxy-2-coumaranone, 3-methyl-1-phenylpyrazolin-5-one, indane-1,2-dione, indane-1,3-dione, indan-1-one, benzoylacetonitrile, 3-dicyanomethyleneindan-1-one, 2-amino-4-imino-1,3-thiazoline hydrochloride, 5,5-dimethylcyclohexane-1,3-dione, 2H-1,4-benzoxazin-4H-3-one, 3-ethyl-2-methylbenzoxazolium iodide, 3-ethyl-2-methylbenzothiazolium iodide, 1-ethyl-4-methylquinolinium iodide, 1-ethyl-2-methylquinolinium iodide, 1,2,3-trimethylquinoxalinium iodide, 3-ethyl-2-methylbenzoxazolium p-toluenesulphonate, 3-ethyl-2-methylbenzothiazolium p-toluenesulphonate, 1-ethyl-4-methylquinolinium p-toluenesulphonate, 1-ethyl-2-methylquinolinium p-toluenesulphonate and 1,2,3-trimethylquinoxalinium p-toluenesulphonate.

12. Agent according to Claim 10, **characterized in that** the primary and secondary aromatic amines of component C are chosen from the group consisting of N,N-dimethyl-p-phenylenediamine, N,N-diethyl-p-phenylenediamine, N-(2-hydroxyethyl)-N-ethyl-p-phenylenediamine, N,N-bis(2-hydroxyethyl)-p-phenylenediamine, N-(2-methoxyethyl)-p-phenylenediamine, 2,3-dichloro-p-phenylenediamine, 2,4-dichloro-p-phenylenediamine, 2,5-dichloro-p-phenylenediamine, 2-chloro-p-phenylenediamine, 2,5-dihydroxy-4-morpholinoaniline, 2-aminophenol, 3-aminophenol, 4-aminophenol, 2-aminomethyl-4-aminophenol, 2-hydroxymethyl-4-aminophenol, o-phenylenediamine, m-phenylenediamine, p-phenylenediamine, 2,5-diaminotoluene, 2,5-diaminophenol, 2,5-diaminoanisole, 2,5-diaminophenetol, 4-amino-3-methylphenol, 2-(2,5-diaminophenyl)ethanol, 2,4-diaminophenoxyethanol, 2-(2,5-diaminophenoxy)ethanol, 3-amino-4-(2-hydroxyethyloxy)phenol, 3,4-methylenedioxyphenol, 3,4-methylenedioxyaniline, 3-amino-2,4-dichlorophenol, 4-methylaminophenol, 2-methyl-5-aminophenol, 3-methyl-4-aminophenol, 2-methyl-5-(2-hydroxyethylamino)phenol, 3-amino-2-chloro-6-methylphenol, 2-methyl-5-amino-4-chlorophenol, 5-(2-hydroxyethylamino)-4-methoxy-2-methylphenol, 4-amino-2-hydroxymethylphenol, 2-(diethylaminomethyl)-4-aminophenol, 4-amino-1-hydroxy-2-(2-hydroxyethylaminomethyl)benzene, 1-hydroxy-2-amino-5-methylbenzene, 1-hydroxy-2-amino-6-methylbenzene, 2-amino-5-acetamidophenol, 1,3-dimethyl-2,5-diaminobenzene, 5-(3-hydroxypropylamino)-2-methylphenol, 5-amino-4-methoxy-2-methylphenol, N,N-dimethyl-3-aminophenol, N-cyclopentyl-3-aminophenol, 5-amino-4-fluoro-2-methylphenol, 2,4-diamino-5-fluorotoluene, 2,4-diamino-5-(2-hydroxyethoxy)toluene, 2,4-diamino-5-methylphenetol, 3,5-diamino-2-methoxy-1-methylbenzene, 2-amino-4-(2-hydroxyethylamino)anisole, 2,6-bis(2-hydroxyethylamino)-1-methylbenzene, 1,3-diamino-2,4-dimethoxybenzene, 3,5-diamino-2-methoxytoluene, 2-aminobenzoic acid, 3-aminobenzoic acid, 4-aminobenzoic acid, 2-aminophenylacetic acid, 3-aminophenylacetic acid, 4-aminophenylacetic acid, 2,3-diaminobenzoic acid, 2,4-diaminobenzoic acid, 2,5-diaminobenzoic acid, 3,4-diaminobenzoic acid, 3,5-diaminobenzoic acid, 4-aminosalicylic acid, 5-aminosalicylic acid, 3-amino-4-hydroxybenzoic acid, 4-amino-3-hydroxybenzoic acid, 2-aminobenzenesulphonic acid, 3-aminobenzenesulphonic acid, 4-aminobenzenesulphonic acid, 3-amino-4-hydroxybenzenesulphonic acid, 4-amino-3-hydroxynaphthalene-1-sulphonic acid, 6-amino-7-hydroxynaphthalene-2-sulphonic acid, 7-amino-4-hydroxynaphthalene-2-sulphonic acid, 4-amino-5-hydroxynaphthalene-2,7-disulphonic acid, 3-amino-2-naphthoic acid, 3-aminophthalic acid, 5-aminoisophthalic acid, 1,3,5-triaminobenzene, 1,2,4-triaminobenzene, 1,2,4,5-tetraaminobenzene, 2,4,5-triaminophenol, pentaaminobenzene, hexaaminobenzene, 2,4,6-triaminoresorcinol, 4,5-diaminopyrocatechin, 4,6-diaminopyrogallol, 1-(2-hydroxy-5-aminobenzyl)-2-imidazolidinone, 4-amino-2-((4-[(5-amino-2-hydroxyphenyl)methyl]-piperazinyl)methyl)phenol, 3,5-diamino-4-hydroxypyrocatechin, 1,4-bis(4-aminophenyl)-1,4-diazacycloheptane, aromatic nitriles, such as 2-amino-4-hydroxybenzonitrile, 4-amino-2-hydroxybenzonitrile, 4-aminobenzonitrile, 2,4-diaminobenzonitrile, amino compounds containing nitro groups, such as 3-amino-6-methylamino-2-nitropyridine, picramic acid, [8-[(4-amino-2-nitrophenyl)azo]-7-hydroxynaphth-2-yl]trimethylammonium chloride, [8-((4-amino-3-nitrophenyl)azo)-7-hydroxynaphth-2-yl]-trimethylammonium chloride (Basic Brown 17), 1-hydroxy-2-amino-4,6-dinitrobenzene, 1-amino-2-nitro-4-[bis(2-hydroxyethyl)amino]benzene, 1-amino-2-[(2-hydroxyethyl)amino]-5-nitrobenzene (HC Yellow No. 5), 1-amino-2-nitro-4-[(2-hydroxyethyl)amino]benzene (HC Red No. 7), 2-chloro-5-nitro-N-2-hydroxyethyl-1,4-phenylenediamine, 1-[(2-hydroxyethyl)amino]-2-nitro-4-aminobenzene (HC Red No. 3), 4-amino-3-nitrophenol, 4-amino-2-nitrophenol, 6-nitro-o-toluidine, 1-amino-3-methyl-4-[(2-hydroxyethyl)amino]-6-nitrobenzene (HC Violet No. 1), 1-amino-2-nitro-4-[(2,3-dihydroxypropyl)amino]-5-chlorobenzene (HC Red No. 10), 2-(4-amino-2-nitroanilino)benzoic acid, 6-nitro-2,5-diaminopyridine, 2-amino-6-chloro-4-nitrophenol, 1-amino-2-(3-nitrophenylazo)-7-phenylazo-8-naphthol-3,6-disulphonic acid disodium salt (Acid Blue No. 29), 1-amino-2-(2-hydroxy-4-nitrophenylazo)-8-naphthol-3,6-disulphonic acid disodium salt (palatine chrome green), 1-amino-2-(3-chloro-2-hydroxy-5-nitrophenylazo)-8-naphthol-3,6-disulphonic acid disodium salt (Gallion), 4-amino-4'-nitrostilbene-2,2'-disulphonic acid disodium salt, 2,4-diamino-3',5'-dinitro-2'-hydroxy-5-methylazobenzene (Mordant Brown 4), 4'-amino-4-nitrodiphenylamine-2-sulphonic acid, 4'-amino-3'-nitrobenzophenone-2-carboxylic acid, 1-amino-4-nitro-2-(2-nitrobenzylideneamino)benzene, 2-[2-(diethylamino)ethylamino]-5-nitroaniline, 3-amino-4-hydroxy-5-nitrobenzenesulphonic acid, 3-amino-3'-nitrobiphenyl, 3-amino-4-nitroacenaphthene, 2-amino-l-nitronaphthalene, 5-amino-6-nitrobenzo-1,3-dioxole, anilines, in particular anilines containing nitro groups, such as 4-nitroaniline, 2-nitroaniline, 1,4-diamino-2-nitrobenzene, 1,2-diamino-4-nitrobenzene, 1-amino-2-methyl-6-nitrobenzene, 4-nitro-1,3-phenylenediamine, 2-nitro-4-amino-1-(2-hydroxyethylamino)-benzene, 2-nitro-1-amino-4-[bis(2-hydroxyethyl) amino] benzene, 4-amino-2-nitrodiphenylamine-2'-carboxylic acid, 1-amino-5-chloro-4-(2-hydroxyethylamino)-2-nitrobenzene, aromatic anilines and phenols with a further aromatic radical as shown in formula VI 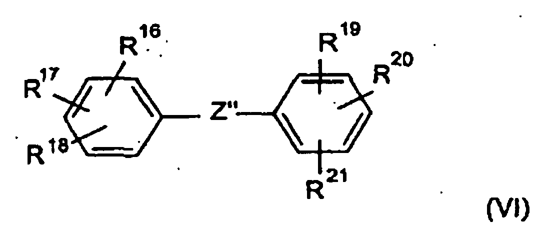 in which
• R¹⁶ is a hydroxy or an amino group which may be substituted by C₁-C₆-alkyl, C₁-C₆-hydroxyalkyl, C₁-C₆-alkoxy or C₁-C₆-alkoxy-C₁-C₆-alkyl groups,
• R¹⁷, R¹⁸, R¹⁹, R²⁰ and R²¹, independently of one another, are a hydrogen atom, a hydroxy or an amino group which may be substituted by C₁-C₆-alkyl, C₁-C₆-hydroxyalkyl, C₁-C₆-alkoxy, C₁-C₆-aminoalkyl or C₁-C₆-alkoxy-C₁-C₆-alkyl groups, and
• Z" is a direct bond, a saturated or unsaturated carbon chain optionally substituted by hydroxy groups and having 1 to 4 carbon atoms, a carbonyl group, sulphonyl group or imino group, an oxygen atom or sulphur atom, or a group with the formula (VII)
**-Q'-(CH₂-Q-4-CH₂-Q")ₒ-** **(VII)**
in which
• Q is a direct bond, a CH₂ group or CHOH group,
• Q' and Q", independently of one another, are an oxygen atom, an NR²² group, in which R²² is a hydrogen atom, a C₁-C₆-alkyl group or C₁-C₆-hydroxyalkyl group, where also the two groups, together with the remaining molecule, can form a 5-, 6- or 7-membered ring, the group O-(CH₂)ₚ-NH or NH-(CH₂)ₚ'-O, in which p and p' are 2 or 3, and
• o is a number from 1 to 4,
such as, for example, 4,4'-diaminostilbene and its hydrochloride, 4,4'-diaminostilbene-2,2'-disulphonic acid mono- or di-Na salt, 4-amino-4'-dimethylaminostilbene and its hydrochloride, 4,4'-diaminodiphenylmethane, 4,4'-diaminodiphenyl sulphide, 4,4'-diaminodiphenyl sulphoxide, 4,4'-diaminodiphenylamine, 4,4'-diaminodiphenylamine-2-sulphonic acid, 4,4'-diaminobenzophenone, 4,4'-diaminodiphenyl ether, 3,3',4,4'-tetraaminodiphenyl, 3,3',4,4'-tetraaminobenzophenone, 1,3-bis(2,4-diaminophenoxy)propane, 1,8-bis(2,5-diaminophenoxy)-3,6-dioxaoctane, 1,3-bis(4-aminophenylamino)propane, 1,3-bis(4-aminophenylamino)-2-propanol, 1,3-bis[N-(4-aminophenyl)-2-hydroxyethylamino]-2-propanol, N,N-bis[2-(4-aminophenoxy)ethyl]methylamine, N-phenyl-1,4-phenylenediamine and bis(5-amino-2-hydroxyphenyl)methane.

13. Agent according to Claim 10, **characterized in that** the aromatic hydroxyl compounds of component C are chosen from the group consisting of 2-, 4-, 5-methylresorcinol, 2,5-dimethylresorcinol, resorcinol, 3-methoxyphenol, pyrocatechin, hydroquinone, pyrogallol, phloroglucine, hydroxyhydroquinone, 2-, 3-, 4-methoxy-, 3-dimethylamino-, 2-(2-hydroxyethyl)-, 3,4-methylenedioxyphenol, 2,4-, 3,4-dihydroxybenzoic acid, -phenylacetic acid, gallic acid, 2,4,6-trihydroxybenzoic acid, -acetophenone, 2-, 4-chlororesorcinol, 1-naphthol, 1,5-, 2,3-, 2,7-dihydroxynaphthalene, 6-dimethylamino-4-hydroxy-2-naphthalenesulphonic acid and 3,6-dihydroxy-2,7-naphthalenesulphonic acid.

14. Agent according to Claim 10, **characterized in that** the nitrogen-containing heterocyclic compounds of component C are chosen from the group consisting of 2-aminopyridine, 3-aminopyridine, 4-aminopyridine, 2-amino-3-hydroxypyridine, 2,6-diaminopyridine, 2,5-diaminopyridine, 2-(aminoethylamino)-5-aminopyridine, 2,3-diaminopyridine, 2-dimethylamino-5-aminopyridine, 2-methylamino-3-amino-6-methoxypyridine, 2,3-diamino-6-methoxypyridine, 2,6-dimethoxy-3,5-diaminopyridine, 2,4,5-triaminopyridine, 2,6-dihydroxy-3,4-dimethylpyridine, N-[2-(2,4-diaminophenyl)aminoethyl]-N-(5-amino-2-pyridyl)amine, N-[2-(4-aminophenyl)aminoethyl]-N-(5-amino-2-pyridyl)amine, 2,4-dihydroxy-5,6-diaminopyrimidine, 4,5,6-triaminopyrimidine, 4-hydroxy-2,5,6-triaminopyrimidine, 2-hydroxy-4,5,6-triaminopyrimidine, 2,4,5,6-tetraaminopyrimidine, 2-methylamino-4,5,6-triaminopyrimidine, 2,4-diaminopyrimidine, 4,5-diaminopyrimidine, 2-amino-4-methoxy-6-methylpyrimidine, 3,5-diaminopyrazole, 3,5-diamino-1,2,4-triazole, 3-aminopyrazole, 3-amino-5-hydroxypyrazole, 1-phenyl-4,5-diaminopyrazole, 1-(2-hydroxyethyl)-4,5-diaminopyrazole, 1-phenyl-3-methyl-4,5-diaminopyrazole, 4-amino-2,3-dimethyl-1-phenyl-3-pyrazolin-5-one (4-aminoantipyrin), 1-phenyl-3-methylpyrazol-5-one, 2-aminoquinoline, 3-aminoquinoline, 8-aminoquinoline, 4-aminoquinaldine, 2-aminonicotinic acid, 6-aminonicotinic acid, 5-aminoisoquinoline, 5-aminoindazole, 6-aminoindazole, 5-aminobenzimidazole, 7-aminobenzimidazole, 5-aminobenzothiazole, 7-aminobenzothiazole, 2,5-dihydroxy-4-morpholinoaniline, and indole and indoline derivatives, such as 4-aminoindole, 5-aminoindole, 6-aminoindole, 7-aminoindole, 5,6-dihydroxyindole, 5,6-dihydroxyindoline 4-hydroxyindoline and hydroxypyrimidine derivatives and the physiologically compatible salts of the abovementioned compounds.

15. Agent according to one of Claims 1 to 14, **characterized in that** the compounds of the formula I, the compounds of component B and the compounds of component C are each present in an amount of from 0.03 to 65 mmol, in particular from 1 to 40 mmol, based on 100 g of the total colorant.

16. Agent according to one of Claims 1 to 15, **characterized in that** it comprises at least one reaction product RP according to formula VIII, 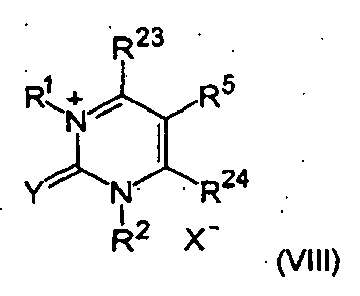 in which
• R¹, R², R⁵, Y and X⁻ are defined as in Claim 1,
• R²³ and R²⁴, independently of one another, are a hydrogen atom, a C₁-C₆-alkyl group, a group according to formula IX, 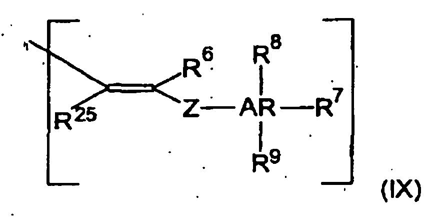 in which
• R⁶, R⁷, R⁸, R⁹, AR and Z are defined as in Claim 7,
• R²⁵ is a hydrogen atom or a C₁-C₅-alkyl group,
with the proviso that at least one of the radicals R²³ and R²⁴ is a group according to formula IX.

17. Agent according to Claim 16, **characterized in that** AR according to formula IX is benzene or naphthalene.

18. Agent according to one of Claims 16 or 17, **characterized in that** Z according to formula IX is a direct bond or vinylene.

19. Agent according to one of Claims 16 to 18, **characterized in that** R²⁵ according to formula IX is a hydrogen atom.

20. Agent according to one of Claims 16 to 19, **characterized in that** R⁷, R⁸ and R⁹ according to formula IX, independently of one another, are a hydrogen atom, a halogen atom, a C₁-C₆-alkyl group, a hydroxy group, a C₁-C₆-alkoxy group, an amino group, a C₁-C₆-dialkylamino group, a di (C₂-C₆-hydroxyalkyl) amino group, a di(C₁-C₆-alkoxy-C₁-C₆-alkyl)amino group, a C₁-C₆-hydroxyalkyloxy group, a sulphonyl group, a carboxyl group, a sulphonamide group, a carbamoyl group, a C₂-C₆-acyl group, an acetyl group, a sulphonic acid group, a sulphonamido group or a nitro group.

21. Agent according to one of Claims 16 to 20, **characterized in that** the reaction product RP is present in an amount of from 0.03 to 65 mmol, in particular from 1 to 40 mmol, based on 100 g of the total colorant.

22. Agent according to one of Claims 1 to 21, **characterized in that** it comprises colour enhancers chosen from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methyimidazole, arginine, histidine, pyrrolidine, proline, pyrrolidone, pyrrolidone-5-carboxylic acid, pyrazole, 1,2,4-triazole, piperazidine or any mixtures thereof.

23. Agent according to one of Claims 1 to 22, **characterized in that** it comprises at least one direct dye, preferably in an amount of from 0.01 to 20% by weight, based on the total colorant.

24. Agent according to one of Claims 1 to 23, **characterized in that** it comprises at least one developer component and optionally at least one coupler component as oxidation dye precursor.

25. Agent according to one of Claims 1 to 24, **characterized in that** ammonium or metal salts chosen from the group of formates, carbonates, halides, sulphates, butyrates, valerates, caproates, acetates, lactates, glycolates, tartrates, citrates, gluconates, propionates, phosphates and phosphonates of alkali metals, such as potassium, sodium or lithium, alkaline earth metals, such as magnesium, calcium, strontium or barium, or of aluminium, manganese, iron, cobalt, copper or zinc, are added.

26. Agent according to one of Claims 1 to 25, **characterized in that** it comprises oxidizing agents, in particular H₂O₂, in an amount of from 0.01 to 6% by weight, based on the application solution.

27. Agent according to one of Claims 1 to 26, **characterized in that** it comprises anionic, zwitterionic or nonionic surfactants.

28. Use of at least one compound according to formula I as in claim 1, in combination with at least one compound of component B as in Claim 1, as a colouring component in hair colorants.

29. Method of dyeing keratinous fibres, in particular human hair, in which a colorant, as in Claim 1, and customary cosmetic ingredients are applied to the keratinous fibres, left on the fibres for a certain time, usually about 15-30 minutes, and then rinsed out again or washed out using a shampoo.

30. Method according to Claim 29,
**characterized in that**, in a two-step method, before or after application of the compound according to formula I, component B is applied to the keratinous fibres, the mixture obtained on the hair is left on the fibres for a certain time, usually about 15-30 minutes, and then rinsed out again or washed out using a shampoo.

31. Method according to one of Claims 29 or 30,
**characterized in that**, before a colorant as in Claim 1 is applied, the keratinous fibres have been bleached in the course of a pretreatment with a bleaching agent or have been coloured with an oxidation colorant.

32. Use of at least one 1,2-dihydropyrimidinium derivative according to formula I and/or its enamine form, 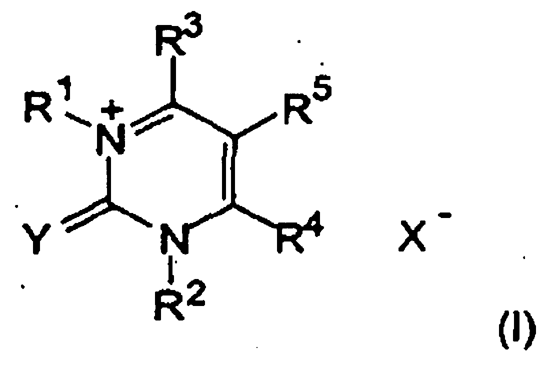 where R¹, R², R³, R⁴, R⁵, Y and X⁻ are as defined in Claim 1 in combination with reactive carbonyl compounds (component B) for the nuancing of oxidation colorations or keratinous fibres, in particular human hair.

33. Use of at least one 1,2-dihydropyrimidinium derivative according to formula I and/or its enamine form, 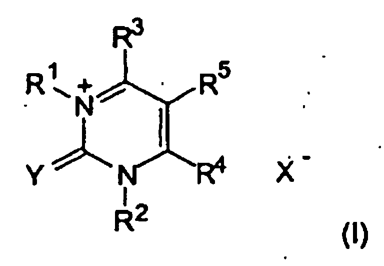 where R¹, R², R³, R⁴, R⁵, Y and X⁻ are as defined in Claim 1
in combination with at least one reactive carbonyl compound (component B) for freshening up the colour of keratinous fibres coloured using oxidative colorants.

## Revendications

1. Agent pour teindre des fibres kératiniques, en particulier les cheveux humains, contenant au moins un dérivé 1,2-dihydro-pyrimidinium selon la formule I et/ou sa forme énamine, 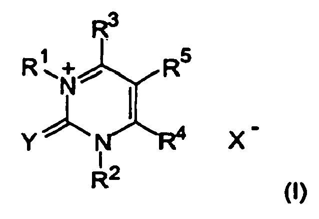 dans laquelle
• R¹ et R² représentent indépendamment l'un de l'autre un groupe alkyle linéaire ou cyclique en C₁ à C₆, un groupe alcényle en C₂ à C₆, un groupe aryle éventuellement substitué, un groupe hétéroaryle éventuellement substitué, un groupe aryl-(alkyle en C₁ à C₆), un groupe hydroxyalkyle en C₁ à C₆, un groupe polyhydroxyalkyle en C₂ à C₆, un groupe (alcoxy en C₁ à C₆)-(alkyle en C₁ à C₆), un groupe R^{I}R^{II}N-(CH₂)ₘ-, dans lequel R^{I} et R^{II} représentent indépendamment l'un de l'autre un atome d'hydrogène, un groupe alkyle en C₁ à C₄, un groupe hydroxyalkyle en C₁ à C₄, ou un groupe aryl-(alkyle en C₁ à C₆), R^{I} et R^{II} pouvant former conjointement avec l'atome d'azote un cycle de 5, 6 ou 7 maillons et m représente un nombre 2, 3, 4, 5 ou 6,
• R³ et R⁴ représentent indépendamment l'un de l'autre un atome d'hydrogène ou un groupe alkyle en C₁ à C₆, au moins l'un des radicaux R³ et R⁴ désignant un groupe alkyle en C₁ à C₆,
• R⁵ représente un atome d'hydrogène, un groupe alkyle en C₁ à C₆, un groupe hydroxyalkyle en C₁ à C₆, un groupe polyhydroxyalkyle en C₂ à C₆, un groupe alcoxy en C₁ à C₆, un groupe hydroxyalcoxy en C₁ à C₆, un groupe R^{III} R^{IV}N-(CH₂)_{q}-, R^{III} et R^{IV} représentant indépendamment l'un de l'autre un atome d'hydrogène, un groupe alkyle en C₁ à C₆, un groupe hydroxyalkyle en C₁ à C₆, ou un groupe aryl(alkyle en C₁ à C₆) et q représente un nombre 1, 2, 3, 4, 5 ou 6, le radical R⁵ pouvant former conjointement avec l'un des radicaux R³ ou R⁴ un cycle aromatique ou aliphatique de 5 ou 6 maillons, qui peut être substitué éventuellement par un atome d'halogène, un groupe alkyle en C₁ à C₆, un groupe hydroxyalkyle en C₁ à C₆, un groupe polyhydroxyalkyle en C₂ à C₆, un groupe alcoxy en C₁ à C₆, un groupe hydroxyalcoxy en C₁ à C₆, un groupe nitro, un groupe hydroxy, un groupe R^{V}R^{VI}N-(CH₂)ₛ-, dans lequel R^{V} et R^{VI} représentent indépendamment l'un de l'autre un atome d'hydrogène, un groupe alkyle en C₁ à C₆, un groupe hydroxyalkyle en C₁ à C₆, ou un groupe aryl-(alkyle en C₁ à C₆) et s représente un nombre 0, 1, 2, 3, 4, 5 ou 6,
• Y représente un atome d'oxygène, un atome de soufre ou un groupe NR^{VII}, dans lequel R^{VII} représente un atome d'hydrogène, un groupe aryle, un groupe hétéroaryle, un groupe alkyle en C₁ à C₆, ou un groupe aryl-(alkyle en C₁ à C₆),
• X⁻ représente un ion halogénure, benzènesulfonate, p-toluènesulfonate, (alcane en C₁ à C₄)-sulfonate, trifluoro-méthanesulfonate, perchlorate, 0,5 sulfate, hydrogénosulfate, tétrafluoroborate, hexafluorophosphate, ou tétrachlorozincate,
et au moins un composé ayant un groupe carbonyle réactif (composant B).

2. Agent selon la revendication 1, **caractérisé en ce que** Y représente un atome d'oxygène ou un atome de soufre.

3. Agent selon l'une quelconque des revendications 1 ou 2, **caractérisé en ce que** Y représente un atome d'oxygène.

4. Agent selon l'une quelconque des revendications 1 à 3, **caractérisé en ce qu'**au moins un radical R^{III} ou R^{IV} représente un groupe méthyle.

5. Agent selon l'une quelconque des revendications 1 à 4, **caractérisé en ce que** X⁻ représente un ion chlorure, bromure, iodure, hydrogénosulfate ou p-toluènesulfonate.

6. Agent selon l'une quelconque des revendications 1 à 5, **caractérisé en ce que** les composés selon la formule I sont choisis dans le groupe formé par le chlorure de 1,2-dihydro-1,3,4,6-tétraméthyl-2-oxo-pyrimidinium, le chlorure de 1,2-dihydro-1,3-diéthyl-4,6-diméthyl-2-oxo-pyrimidinium, le chlorure de 1,2-dihydro-1,3-dipropyl-4,6-diméthyl-2-oxopyrimidinium, le chlorure de 1,2-dihydro-1,3-di(2-hydroxyéthyl)-4,6-diméthyl-2-oxopyrimidinium, le chlorure de 1,2-dihydro-1,3-diphényl-4,6-diméthyl-2-oxo-pyrimidinium, l'hydrogénosulfate de 1,2-dihydro-1,3,4,6-tétraméthyl-2-oxo-pyrimidinium, l'hydrogénosulfate de 1,2-dihydro-1,3-diéthyl-4,6-diméthyl-2-oxo-pyrimidinium, l'hydrogénosulfate de 1,2-dihydro1,3-dipropyl-4,6-diméthyl-2-oxo-pyrimidinium, l'hydrogénosulfate de 1,2-dihydro-1,3-di(2-hydroxyéthyl)-4,6-diméthyl-2-oxo-pyrimidinium, l'hydrogénosulfate de 1,2-dihydro-1,3-diphényl-4,6-diméthyl-2-oxo-pyrimidinium, le chlorure de 1,2-dihydro-1,3,4-triméthyl-2-oxo-pyrimidinium, l'hydrogénosulfate de 1,2-dihydro-1,3,4-triméthyl-2-oxo-pyrimidinium, le chlorure de 1,2-dihydro-1,3-diéthyl-4-méthyl-2-oxo-pyrimidinium, l'hydrogénosulfate de 1,2-dihydro-1,3-diéthyl-4-méthyl-2-oxo-pyrimidinium, le chlorure de 1,2-dihydro-1,3-dipropyl-4-méthyl-2-oxo-pyrimidinium, l'hydrogénosulfate de 1,2-dihydro-1,3-dipropyl-4-méthyl-2-oxo-pyrimidinium, le chlorure de 1,2-dihydro-1,3-di(2-hydroxyéthyl)-4-méthyl-2-oxo-pyrimidinium, l'hydrogénosulfate de 1,2-dihydro-1,3-di(2-hydroxyéthyl)-4-méthyl-2-oxopyrimidinium, le chlorure de 1,2-dihydro-1,3-diphényl-4-méthyl-2-oxopyrimidinium, l'hydrogénosulfate de 1,2-dihydro-1,3-diphényl-4-méthyl-2-oxopyrimidinium, le chlorure de 1,2-dihydro-1,3,4,5,6-pentaméthyl-2-oxopyrimidinium, le chlorure de 1,2-dihydro-1,3,4,6-tétraméthyl-2-thioxopyrimidinium, le chlorure de 1,2-dihydro-1,3-diéthyl-4,6-diméthyl-2-thioxopyrimidinium, le chlorure de 1,2-dihydro-1,3-dipropyl-4,6-diméthyl-2-thioxopyrimidinium, le chlorure de 1,2-dihydro-1,3-di(2-hydroxyéthyl)-4,6-diméthyl-2-thioxopyrimidinium, le chlorure de 1,2-dihydro-1,3-diphényl-4,6-diméthyl-2-thioxo-pyrimidinium, l'hydrogénosulfate de 1,2-dihydro-1,3,4,6-tétraméthyl-2-thioxo-pyrimidinium, l'hydrogénosulfate de 1,2-dihydro-1,3-dipropyl-4,6-diméthyl-2-thioxo-pyrimidinium, l'hydrogénosulfate de 1,2-dihydro-1,3-di(2-hydroxyéthyl)-4,6-diméthyl-2-thioxo-pyrimidinium, l'hydrogénosulfate de 1,2-dihydro-1,3-diphényl-4,6-diméthyl-2-thioxo-pyrimidinium, le chlorure de 1,2-dihydro-1,3,4-triméthyl-2-thioxo-pyrimidinium, l'hydrogénosulfate de 1,2-dihydro-1,3,4-triméthyl-2-thioxo-pyrimidinium, le chlorure de 1,2-dihydro-1,3-diéthyl-4-méthyl-2-thioxopyrimidinium, l'hydrogénosulfate de 1,2-dihydro-1,3-diéthyl-4-méthyl-2-thioxo-pyrimidinium, le chlorure de 1,2-dihydro-1,3-dipropyl-4-méthyl-2-thioxo-pyrimidinium, l'hydrogénosulfate de 1,2-dihydro-1,3-dipropyl-4-méthyl-2-thioxo-pyrimidinium, le chlorure de 1,2-dihydro-1,3-di(2-hydroxyéthyl)-4-méthyl-2-thioxo-pyrimidinium, l'hydrogénosulfate de 1,2-dihydro-1,3-di(2-hydroxyéthyl)-4-méthyl-2-thioxo-pyrimidinium, le chlorure de 1,2-dihydro-1,3-diphényl-4-méthyl-2-thioxo-pyrimidinium, l'hydrogénosulfate de 1,2-dihydro-1,3-diphényl-4-méthyl-2-thioxo-pyrimidinium, le chlorure de 1,2-dihydro-3,4-diméthyl-2-oxoquinazolinium, le p-toluène-sulfonate de 1,2-dihydro-3,4-diméthyl-2-oxo-quinazolinium, le chlorure de 1,2-dihydro-3,4-diméthyl-2-thioxo-quinazolinium, le p-toluène-sulfonate de 1,2-dihydro-3,4-diméthyl-2-thioxo-quinazolinium, l'hydrogénosulfate de 1,2-dihydro-1,3,4-triméthyl-2-thioxo-quinazolinium, l'hydrogénosulfate de 1,3,4-triméthyl-2-oxo-2,3,5,6,7,8-hexahydroquinazolinium, l'hydrogénosulfate de 1,3,4-triméthyl-2-thioxo-2,3,5,6,7,8-hexahydroquinazolinium, l'hydrogénosulfate de 1,3,4-triméthyl-2-oxo-3,5,6,7-tétrahydro-2H-cyclopenta[a]pyrimidinium et l'hydrogénosulfate de 1,3,4-triméthyl-2-thioxo-3,5,6,7-tétrahydro-2H-cyclopenta[a]pyrimidinium.

7. Agent selon l'une quelconque des revendications 1 à 6, **caractérisé en ce que** le composant B est choisi parmi les composés selon la formule II 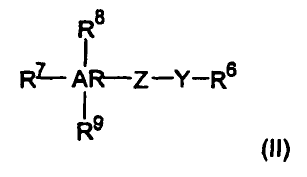 dans laquelle
• AR représente un groupe benzène, naphtalène, pyridine, pyrimidine, pyrazine, pyridazine, carbazole, pyrrole, pyrazole, furane, thiophène, 1,2,3-triazine, 1,3,5-triazine, quinoléine, isoquinoléine, indole, indoline, indolizine, indane, imidazole, 1,2,4-triazole, 1,2,3-triazole, tétrazole, benzimidazole, 1,3-thiazole, benzothiazole, indazole, benzoxazole, quinoxaline, quinazoline, quinolizine, cinnoline, acridine, julolidine, acénaphtène, fluorène, biphényle, diphénylméthane, benzophénone, diphényléther, azobenzène, chromone, coumarine, diphénylamine, stilbène, des substances N-hétéroaromatiques pouvant être également quaternisées,
• R⁶ représente un atome d'hydrogène, un groupe alkyle en C₁ à C₆, acyle en C₂ à C₆, alcényle en C₂ à C₄, perfluoroalkyle en C₁ à C₄, un groupe aryle ou hétéroaryle éventuellement substitué,
• R⁷, R⁸ et R⁹ représentent indépendamment l'un de l'autre un atome d'hydrogène, un atome d'halogène, un groupe alkyle en C₁ à C₆, alcoxy en C₁ à C₆, aminoalkyle en C₁ à C₆, hydroxyalkle en C₁ à C₆, un groupe (alcoxy en C₁ à C₆)-(alkyle en C₁ à C₆)oxy, un groupe acyle en C₂ à C₆, un groupe acétyle, carboxyle, carboxylato, carbamoyle, sulfo, sulfato, sulfonamide, sulfonamido, alcényle en C₂ à C₆, aryle, aryl-(alkyle en C₁ à C₆), un groupe hydroxy, nitro, pyrrolidino, morpholino, pipéridino, amino ou ammonio, ou 1-imidazol(in)io, les trois derniers groupes pouvant. être substitués par un ou plusieurs groupes alkyle en C₁ à C₆, carboxyalkyle en C₁ à C₆, hydroxyalkyle en C₁ à C₆, alcényle en C₂ à C₆, (alcoxy en C₁ à C₆)-(alkyle en C₁ à C₆), par des groupes benzyle éventuellement substitués, par des groupes sulfo-(alkyle en C₁ à C₄) ou hétérocycle-(alkyle en C₁ à C₄),
• également deux des radicaux parmi R⁷, R⁸, R⁹ et -Z-Y-R⁶ conjointement avec le restant de la molécule peuvent former un cycle condensé de 5, 6 ou 7 maillons, éventuellement substitué, qui peut porter également un noyau aromatique condensé, le système AR pouvant porter en fonction de la taille du cycle, d'autres substituants qui peuvent représenter indépendamment l'un de l'autre les mêmes groupes que R⁷, R⁸ et R⁹,
• Z représente une liaison directe, un groupe carbonyle, carboxy-(alkylène en C₁ à C₄), un groupe alcénylène en C₂ à C₆, alcadiénylène en C₄ à C₆, furylène, thiénylène, arylène, vinylène-arylène, vinylène-furylène, vinylène-thiénylène, éventuellement substitué, Z pouvant former conjointement avec le groupe -Y-R⁶ également un cycle de 5, 6 ou 7 maillons éventuellement substitué,
• Y représente un groupe qui est choisi parmi un groupe carbonyle, un groupe selon la formule III et un groupe selon la formule IV 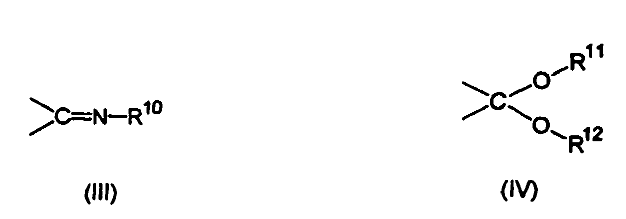
dans lesquelles
• R¹⁰ représente un atome d'hydrogène, un groupe hydroxy, un groupe alcoxy en C₁ à C₄, un groupe alkyle en C₁ à C₆, un groupe hydroxyalkyle en C₁ à C₆, un groupe polyhydroxyalkyle en C₂ à C₆, un groupe (alcoxy en C₁ à C₆)-(alkyle en C₁ à C₆),
• R¹¹ et R¹² représentent indépendamment l'un de l'autre un groupe alkyle en C₁ à C₆, un groupe aryle, ou forment conjointement avec l'élément structural O-C-O de la formule IV, un cycle de 5 ou 6 maillons.

8. Agent selon l'une quelconque des revendications 1 à 7, **caractérisé en ce que** le composant B est choisi dans le groupe formé par l'acétophénone, la propiophénone, la 2-hydroxyacétophénone, la 3-hydroxyacétophénone, la 4-hydroxyacétophénone, la 2-hydroxypropiophénone, la 3-hydroxypropiophénone, la 4-hydroxypropiophénone, la 2-hydroxybutyrophénone, la 3-hydroxybutyrophénone, la 4-hydroxybutyrophénone, la 2,4-dihydroxyacétophénone, la 2,5-dihydroxyacétophénone, la 2,6-dihydroxyacétophénone, la 2,3,4-trihydroxyacétophénone, la 3,4,5trihydroxyacétophénone, la 2,4,6-trihydroxyacétophénone, la 2,4,6-triméthoxyacétophénone, la 3,4,5-triméthoxyacétophénone, le 3,4,5-triméthoxy-acétophénone-diéthylcétal, la 4-hydroxy-3-méthoxy-acétophénone, la 3,5-diméthoxy-4-hydroxyacétophénone, la 4-aminoacétophénone, la 4-diméthylaminoacétophénone, la 4-morpholinoacétophénone, la 4-piperidinoacétophénone, la 4-imidazolinoacétophénone, la 2-hydroxy-5-bromoacétophénone, la 4-hydroxy-3-nitroacétophénone, l'acide acétophénone-2-carboxylique, l'acide acétophénone-4-carboxylique, la benzophénone, la 4-hydroxybenzophénone, la 2-aminobenzophénone, la 4,4'-dihydroxy-benzophénone, la 2,4-dihydroxy-benzophénone, la 2,4,4'-trihydroxybenzophénone, la 2,3,4-trihydroxybenzophénone, la 2-hydroxy-1-acétonaphtone, la 1-hydroxy-2-acétonaphtone, la chromone, l'acide chromone-2-carboxylique, la flavone, la 3-hydroxyflavone, la 3,5,7-trihydroxyflavone, la 4',5,7-trihydroxyflavone, la 5,6,7-trihydroxyflavone, la quercétine, la 1-indanone, la 9-fluorénone, la 3-hydroxyfluorénone, l'anthrone, la 1,8-dihydroxyanthrone, la vanilline, le coniférylaldéhyde, le 2-méthoxybenzaldéhyde, le 3-méthoxybenzaldéhyde, le 4-méthoxybenzaldéhyde, le 2-éthoxybenzaldéhyde, le 3-éthoxybenzaldéhyde, le 4-éthoxybenzaldéhyde, le 4-hydroxy-2,3-diméthoxybenzaldéhyde, le 4-hydroxy-2,5diméthoxy-benzaldéhyde, le 4-hydroxy-2,6-diméthoxy-benzaldéhyde, le 4-hydroxy-2méthyl-benzaldéhyde, le 4-hydroxy-3-méthyl-benzaldéhyde, le 4-hydroxy-2,3-diméthylbenzaldéhyde, le 4-hydroxy-2,5-diméthyl-benzaldéhyde, le 4-hydroxy-2,6-diméthylbenzaldéhyde, le 4-hydroxy-3,5-diméthoxy-benzaldéhyde, le 4-hydroxy-3,5-diméthylbenzaldéhyde, le 3,5-diéthoxy-4-hydroxy-benzaldéhyde, le 2,6-diéthoxy-4-hydroxy-benzaldéhyde, le 3-hydroxy-4-méthoxy-benzaldéhyde, le 2-hydroxy-4-méthoxybenzaldéhyde, le 2-éthoxy-4-hydroxy-benzaldéhyde, le 3-éthoxy-4-hydroxy-benzaldéhyde, le 4-éthoxy-2-hydroxy-benzaldéhyde, le 4-éthoxy-3-hydroxy-benzaldéhyde, le 2,3-diméthoxybenzalidéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,5-diméthoxybenzaldéhyde, le 2,3,4-triméthoxybenzaldéhyde, le 2,3,5-triméthoxybenzaldéhyde, le 2,3,6-triméthoxybenzaldéhyde, le 2,4,6-triméthoxybenzaldéhyde, le 2,4,5-triméthoxybenzaldéhyde, le 2,5,6-triméthoxybenzaldéhyde, le 2-hydroxybenzaldéhyde, le 3-hydroxybenzaldéhyde, le 4-hydroxybenzaldéhyde, le 2,3-dihydroxybenzaldéhyde, le 2,4-dihydroxybenzaldéhyde, le 2,5-dihydroxybenzaldéhyde, le 2,6-dihydroxybenzaldéhyde, le 3,4-dihydroxybenzaldéhyde, le 3,5-dihydroxybenzaldéhyde, le 2,3,4-trihydroxybenzaldéhyde, le 2,3,5-trihydroxybenzaldéhyde, le 2,3,6-trihydroxybenzaldéhyde, le 2,4,6-trihydroxybenzaldéhyde, le 2,4,5-trihydroxybenzaldéhyde, le 2,5,6-trihydroxybenzaldéhyde, le 4-hydroxy-2-méthoxybenzaldéhyde, le 4-diméthylaminobenzaldéhyde, le 4-diéthylamino-benzaldéhyde, le 4-diméthylamino-2-hydroxybenzaldéhyde, le 4-diéthylamino-2-hydroxybenzaldéhyde, le 4-pyrrolidinobenzaldéhyde, le 4-morpholinobenzaldéhyde, le 2-morpholinobenzaldéhyde, le 4-piperidinobenzaldéhyde, le 2-méthoxy-1-naphtaldéhyde, le 4-méthoxy-1-naphtaldéhyde, le 2-hydroxy-1-naphtaldéhyde, le 2,4-dihydroxy-1-naphtaldéhyde, le 4-hydroxy-3-méthoxy-1-naphtaldéhyde, le 2-hydroxy-4-méthoxy-1-naphtaldéhyde, le 3-hydroxy-4-méthoxy-1-naphtaldéhyde, le 2,4-diméthoxy-1-naphtaldéhyde, le 3,4-diméthoxy-1-naphtaldéhyde, le 4-hydroxy-1-naphtaldéhyde, le 4-diméthylamino-1-naphtaldéhyde, le 4-diméthylaminocinnamaldéhyde, le 2-diméthylaminobenzaldéhyde, le 2-chloro-4-diméthylaminobenzaldéhyde, le 4-diméthylamino-2-méthylbenzaldéhyde, le 4-diéthylamino-cinnamaldéhyde, le 4-dibutylamino-benzaldéhyde, le 4-diphenylamino-benzaldéhyde, le 4-diméthylamino-2-méthoxybenzaldéhyde, le 4-(1-imidazolyl)-benzaldéhyde, le pipéronal, le 2,3,6,7-tétrahydro1H,5H-benzo[ij]quinolizine-9-carboxaldéhyde, le 2,3,6,7-tétrahydro-8-hydroxy-1H,5Hbenzo[ij]quinolizine-9-carboxaldéhyde, le N-éthylcarbazol-3-aldéhyde, la 2-formylméthylène-1,3,3-triméthylindoline (aldéhyde de Fischer ou aldéhyde tribasique), le 2-indolaldéhyde, le 3-indolaldéhyde, le 1-méthylindol-3-aldéhyde, le 2-méthylindol-3-aldéhyde, le 1-acétylindol-3-aldéhyde, le 3-acétylindol, le 1-méthyl-3-acétylindol, le 2-(1',3',3'-triméthyl-2-indolinylidène)-acétaldéhyde, le 1-méthylpyrrol-2-aldéhyde, le 1-méthyl-2-acétylpyrrole, le 4-pyridinaldéhyde, le 2-pyridinaldéhyde, le 3-pyridinaldéhyde, la 4-acétylpyridine, la 2-acétylpyridine, la 3-acétylpyridine, le pyridoxal, le quinoléine-3-aldéhyde, le quinoléine-4-aldéhyde, l'antipyrine-4-aldéhyde, le furfural, le 5-nitrofurfural, le 2-thénoyl-trifluoro-acétone, le chromone-3-aldéhyde, la 3(5'-nitro-2'-furyl)-acroléine, la 3-(2'-furyl)-acroléine et l'imidazole-2-aldéhyde, le 1,3-diacétylbenzène, le 1,4-diacétylbenzène, le 1,3,5-triacétylbenzène, la 2-benzoyl-acétophénone, la 2-(4'-méthoxybenzoyl)-acétophénone, la 2-(2'-furoyl)-acétophénone, la 2-(2'-pyridoyl)-acétophénone et la 2-(3'-pyridoyl)-acétophénone,la benzylidène-acétone, la 4-hydroxybenzylidène-acétone, la 2-hydroxybenzylidène-acétone, la 4-méthoxybenzylidène-acétone, la 4-hydroxy-3-méthoxybenzylidène-acétone, la 4-diméthylaminobenzylidène-acétone, la 3,4-méthylenedioxybenzylidène-acétone, la 4-pyrrolidinobenzylidène-acétone, la 4-piperidinobenzylidène-acétone, la 4-morpholinobenzylidène-acétone, la 4-diéthylaminobenzylidène-acétone, la 3-benzylidène-2,4-pentanedione, la 3-(4'-hydroxybenzylidène)-2,4-pentanedione, la 3-(4'-diméthylaminobenzylidène)-2,4-pentanedione, la 2-benzylidènecyclohexanone, la 2-(4'-hydroxybenzylidène)-cyclohexanone, la 2-(4'-diméthylaminobenzylidène)-cyclohexanone, la 2-benzylidène-1,3-cyclohexanedione, la 2-(4'-hydroxybenzylidène)-1,3-cyclohexanedione, la 3-(4'-diméthylaminobenzylidène)-1,3-cyclohexanedione, la 2-benzylidène-5,5-diméthyl-1,3-cyclohexanedione, la 2-(4'-hydroxy-benzylidène)-5,5-diméthyl-1,3-cyclohexanedione, la 2-(4'-hydroxy-3-méthoxybenzylidène)-5,5-diméthyl-1,3-cyclohexanedione, la 2-(4'-diméthylaminobenzylidène)-5,5-diméthyl-1,3-cyclohexanedione, la 2-benzylidènecyclopentanone, la 2'-(4-hydroxybenzylidène)-cyclopentanone, la 2-(4'-diméthylaminobenzylidène)-cyclopentanone, le 5-(4-diméthylaminophenyl)penta-2,4-diénal, le 5-(4-diéthylaminophenyl)penta-2,4-diénal, le 5-(4-méthoxyphenyl)penta-2,4-diénal, le 5-(3,4-diméthoxyphenyl)penta-2,4-diénal, le 5-(2,4-diméthoxyphenyl)penta-2,4-diénal, le 5-(4-piperidinophenyl)penta-2,4-diénal, le 5-(4-morpholinophenyl)penta-2,4-diénal, le 5-(4-pyrrolidinophenyl)penta-2,4-diénal, la 6-(4-diméthylaminophenyl)hexa-3,5-dién-2-one, la 6-(4-diéthylaminophenyl)hexa-3,5-dién-2-one, la 6-(4-méthoxyphenyl)hexa-3,5-dién-2-one, la 6-(3,4-diméthoxyphenyl)hexa3,5-dién-2-one, la 6-(2,4-diméthoxyphenyl)hexa-3,5-dién-2-one, la 6-(4-piperidinophenyl)hexa-3,5-dién-2-one, la 6-(4-morpholinophenyl)hexa-3,5-dién-2-one, la 6-(4-pyrrolidinophenyl)hexa-3,5-dién-2-one, le 5-(4-diméthylamino-1-naphtyl)penta-3,5-diénal, le 2-nitrobenzaldéhyde, le 3-nitrobenzaldéhyde, le 4-nitrobenzaldéhyde, 4-méthyl-3-nitrobenzaldéhyde, le 3-hydroxy-4nitrobenzaldéhyde, le 4-hydroxy-3-nitrobenzaldéhyde, le 5-hydroxy-2-nitrobenzaldéhyde, le 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3-nitrobenzaldéhyde, le 2-chloro-6-nitrobenzaldéhyde, le 5-chloro-2-nitrobenzaldéhyde, le 4-chloro-2-nitrobenzaldéhyde, le 2,4-dinitrobenzaldéhyde, le 2,6-dinitrobenzaldéhyde, le 2-hydroxy-3-méthoxy-5nitrobenzaldéhyde, le 4,5-diméthoxy-2-nitrobenzaldéhyde, le 6-nitropipéronal, le 2-nitropipéronal, la 5-nitrovanilline, le 2,5-dinitrosalicylaldehyde, le 5-bromo-3-nitrosalicylaldéhyde, l'acide 3-nitro-4-formylbenzènesulfonique, le 4-nitro-1-naphtaldéhyde, le 2-nitrocinnamaldéhyde, le 3-nitrocinnamaldéhyde, le 4-nitrocinnamaldéhyde, le 9-méthyl-3-carbazolaldéhyde, le 9-éthyl-3-carbazolaldéhyde, le 3-acétyl-carbazole, le 3,6-diacétyl-9-éthylcarbazole, le 3-acétyl-9-méthylcarbazole, le 1,4-diméthyl-3-carbazolaldéhyde, le 1,4,9-triméthyl-3-carbazolaldéhyde, les benzènesulfonates, p-toluènesulfonates, méthanesulfonates, perchlorates, sulfates, chlorures, bromures, iodures, tétrachlorozincates, méthylsulfates, trifluorométhanesulfonates, tetrafluoroborates de 4-formyl-1-méthylpyridinium, de 2-formyl-1-méthylpyridinium, de 4-formyl-1-éthylpyridinium, de 2-formyl-1-éthylpyridinium, de 4-formyl-1-benzylpyridinium, de 2-formyl-1-benzylpyridinium, de 4-formyl-1,2-diméthylpyridinium, de 4-formyl-1,3diméthylpyridinium, de 4-formyl-1-méthylquinoléinium, de 2-formyl-1-méthylquinoléinium, de 4-acétyl-1-méthyl-pyridinium, de 2-acétyl-1-méthyl-pyridinium, de 4-acétyl-1-méthylquinoléinium, de 5-formyl-1-méthylquinoléinium, de 6-formyl-1-méthylquinoléinium, de 7-formyl-1-méthylquinoléinium, de 8-formyl-1-méthylquinoléinium, de 5-formyl-1éthyl-quinoléinium, de 6-formyl-1-éthylquinoléinium, de 7-formyl-1-éthylquinoléinium, de 8-formyl-1-éthylquinoléinium, de 5-formyl-1-benzylquinoléinium, de 6-formyl-1-benzylquinoléinium, de 7-formyl-1-benzylquinoléinium, de 8-formyl-1-benzylquinoléinium, de 5-formyl-1-allylquinoléinium, de 6-formyl-1-allylquinoléinium, de 7-formyl-1-allyl-quinoléinium et de 8-formyl-1-allylquinoléinium, de 5-acétyl-1-méthyl-quinoléinium, de 6-acétyl-1-méthylquinoléinium, de 7-acétyl-1-méthylquinoléinium, de 8-acétyl-1-méthylquinoléinium, de 5-acétyl-1-éthylquinoléinium, de 6-acétyl-1-éthylquinoléinium, de 7-acétyl-1-éthylquinoléinium, de 8-acétyl-1-éthylquinoléinium, de 5-acétyl-1-benzyl-quinoléinium, de 6-acétyl-1-benzylquinoléinium, de 7-acétyl-1-benzylquinoléinium, de 8-acétyl-1-benzylquinoléinium, de 5-acétyl-1-allylquinoléinium, de 6-acétyl-1-allylquinoléinium, de 7-acétyl-1-allylquinoléinium et de 8-acétyl-1-allylquinoléinium, de 9-formyl-10-méthylacridinium, de 4-(2'-formylvinyl)-1-méthylpyridinium, de 1,3-di-méthyl-2-(4'-formylphenyl)-benzimidazolium, de 1,3-diméthyl-2-(4'-formyl-phenyl)-imidazolium, de 2-(4'-formylphenyl)-3-méthylbenzothiazolium, de 2-(4'-acétyl-phenyl)-3-méthylbenzothiazolium, 2-(4'-formylphenyl)-3-méthyl-benzoxazolium, de 2-(5'-formyl-2'-furyl)-3-méthylbenzothiazolium, de 2-(5'-formyl-2'-furyl)-3-méthyl-benzothiazolium, de 2-(5'-formyl-2'-thienyl)-3-méthylbenzothiazolium, de 2-(3'-formyl-phenyl)-3-méthylbenzothiazolium, de 2-(4'-formyl-1-naphtyl)-3-méthyl-benzo-thiazolium, de 5-chloro-2-(4'-formylphenyl)-3-méthylbenzothiazolium, de 2-(4'-formylphenyl)-3,5-diméthylbenzothiazolium, l'isatine, la 1-méthylisatine, la 1-Allylisatine, la 1-hydroxyméthylisatine, la 5-chloroisatine, la 5-méthoxyisatine, la 5-nitroisatine, la 6-nitroisatine, la 5-sulfoisatine, la 5-carboxyisatine, la quinisatine, la 1-méthylquinisatine, ainsi que des mélanges quelconques des composés précédents.

9. Agent selon l'une quelconque des revendications 1 à 8, **caractérisé en ce que** le composant B est choisi dans le groupe formé par la vanilline, le coniférylaldéhyde, le 2-méthoxybenzaldéhyde, le 3-méthoxybenzaldéhyde, le 4-méthoxybenzaldéhyde, le 2-éthoxybenzaldéhyde, le 3-éthoxybenzaldéhyde, le 4-éthoxybenzaldéhyde, le 4-hydroxy-2,3-diméthoxy-benzaldéhyde, le 4-hydroxy-2,5diméthoxy-benzaldéhyde, le 4-hydroxy-2,6-diméthoxybenzaldéhyde, le 4-hydroxy-2-méthyl-benzaldéhyde, le 4-hydroxy-3-méthyl-benzaldéhyde, le 4-hydroxy-2,3-diméthyl-benzaldéhyde, le 4-hydroxy-2,5-diméthyl-benzaldéhyde, le 4-hydroxy-2,6-diméthyl-benzaldéhyde, le 4-hydroxy-3,5-diméthoxy-benzaldéhyde, le 4-hydroxy-3,5-diméthyl-benzaldéhyde, le 3,5-diéthoxy-4-hydroxy-benzaldéhyde, le 2,6-diéthoxy-4-hydroxybenzaldéhyde, le 3-hydroxy-4-méthoxy-benzaldéhyde, le 2-hydroxy-4-méthoxybenzaldéhyde, le 2-éthoxy-4-hydroxy-benzaldéhyde, le 3-éthoxy-4-hydroxy-benzaldéhyde, le 4-éthoxy-2-hydroxy-benzaldéhyde, le 4-éthoxy-3-hydroxy-benzaldéhyde, le 2,3-diméthoxybenzaldéhyde, le 2,4-diméthoxybenzaldéhyde, le 2,5-diméthoxybenzaldéhyde, le 2,6-diméthoxybenzaldéhyde, le 3,4-diméthoxybenzaldéhyde, le 3,5-diméthoxybenzaldéhyde, le 2,3,4-triméthoxybenzaldéhyde, le 2,3,5-triméthoxybenzaldéhyde, le 2,3,6-triméthoxybenzaldéhyde, le 2,4,6-triméthoxybenzaldéhyde, le 2,4,5-triméthoxybenzaldéhyde, le 2,5,6-triméthoxybenzaldéhyde, le 2-hydroxybenzaldéhyde, le 3-hydroxybenzaldéhyde, le 4-hydroxybenzaldéhyde, le 2,3-dihydroxybenzaldéhyde, le 2,4-dihydroxybenzaldéhyde, le 2,5dihydroxybenzaldéhyde, le 2,6-dihydroxybenzaldéhyde, le 3,4-dihydroxybenzaldéhyde, le 3,5dihydroxybenzaldéhyde, le 2,3,4-trihydroxybenzaldéhyde, le 2,3,5-trihydroxybenzaldéhyde, le 2,3,6-trihydroxybenzaldéhyde, le 2,4,6-trihydroxybenzaldéhyde, le 2,4,5-trihydroxybenzaldéhyde, le 2,5,6-trihydroxybenzaldéhyde, le 4-hydroxy-2-méthoxybenzaldéhyde, 4-diméthylaminobenzaldéhyde, le 4-diéthylamino-benzaldéhyde, le 4-diméthylamino-2-hydroxybenzaldéhyde, le 4-diéthylamino-2-hydroxybenzaldéhyde, le 4-pyrrolidinobenzaldéhyde, le 4-morpholinobenzaldéhyde, le 2-morpholinobenzaldéhyde, le 4-piperidinobenzaldéhyde, le 2-méthoxy-1-naphtaldéhyde, le 4-méthoxy-1-naphtaldéhyde, le 2-hydroxy-1-naphtaldéhyde, le 2,4-dihydroxy-1-napthaldéhyde, le 4-hydroxy-3-méthoxy1-naphtaldéhyde, le 2-hydroxy-4-méthoxy-1-naphtaldéhyde, le 3-hydroxy-4-méthoxy-1-naphtaldéhyde, le 2,4-diméthoxy-1-naphtaldéhyde, le 3,4-diméthoxy-1-naphtaldéhyde, le 4-hydroxy-1-naphtaldehyde, le 4-diméthylamino-1-naphtaldéhyde, le 4-diméthylaminocinnamaldéhyde, le 2-diméthylaminobenzaldéhyde, le 2-chloro-4-diméthylaminobenzaldéhyde, le 4-diméthylamino-2-méthylbenzaldéhyde, le 4-diéthylaminocinnamaldéhyde, le 4-dibutylamino-benzaldéhyde, le 4-diphenylaminobenzaldéhyde, le 4-diméthylamino-2-méthoxybenzaldéhyde, le 4-(1-imidazolyl)-benzaldéhyde et le pipéronal.

10. Agent selon l'une quelconque des revendications 1 à 9, **caractérisé en ce qu'**il contient au moins un composé en tant que composant C, choisi parmi (a) les composés à groupe CH acide, et (b) les composés ayant un groupe hydroxy ou amino primaire ou secondaire, choisis parmi les composés hydroxyaromatiques, les amines aromatiques primaires ou secondaires et les composés hétérocycliques azotés.

11. Agent selon la revendication 10, **caractérisé en ce que** les composés à groupe CH acide du composant C sont choisis dans le groupe formé par l'iodure de 1,2,3,3-tetraméthyl-3H-indolium, le p-toluènesulfonate de 1,2,3,3-tetraméthyl-3H-indolium, le méthanesulfonate de 1,2,3,3-tetraméthyl-3H-indolium, la 1,3,3-triméthyl-2-méthylèneindoline (base de Fischer), l'iodure de 2,3-diméthyl-benzothiazolium, le p-toluènesulfonate de 2,3-diméthyl-benzothiazolium, le p-toluènesulfonate de 2,3-diméthyl-naphto[1,2-d]thiazolium, le p-toluènesulfonate de 3-éthyl-2-méthyl-naphto[1,2-d]thiazolium, la rhodanine, l'acide rhodanine-3-acétique, l'iodure de 1,4-diméthylquinoléinium, l'iodure de 1,2-diméthylquinoléinium, l'acide barbiturique, l'acide thiobarbiturique, l'acide 1,3-diméthylthiobarbiturique, l'acide 1,3-diéthylthiobarbiturique, l'acide 1,3-diéthylbarbiturique, l'oxindol, l'acétate de 3-indoxyle, la 2-coumaranone, la 5-hydroxy-2-coumaranone, la 6-hydroxy-2-coumaranone, la 3-méthyl-1-phenyl-pyrazolin-5-one, l'indane-1,2-dione, l'indane-1,3-dione, l'indane-1-one, le benzoylacétonitrile, la 3-dicyanométhylène-indan-1-one, le chlorhydrate de 2-amino-4-imino-1,3-thiazoline, la 5,5-diméthylcyclohexane-1,3-dione, la 2H-1,4-benzoxazin-4H-3-one, l'iodure de 3-éthyl-2-méthylbenzoxazolium, l'iodure de 3-éthyl-2-méthyl-benzothiazolium, l'iodure de 1-éthyl-4-méthylquinoléinium, l'iodure de 1-éthyl-2-méthylquinoléinium, l'iodure de 1,2,3-triméthylquinoxalinium, le p-toluènesulfonate de 3-éthyl-2-méthyl-benzoxazolium, le p-toluènesulfonate de 3-éthyl-2-méthyl-benzothiazolium, le p-toluènesulfonate de 1-éthyl-4-méthyl-quinoléinium, le p-toluènesulfonate de 1-éthyl-2-méthylquinoléinium, et le p-toluènesulfonate de 1,2,3-triméthylquinoxalinium.

12. Agent selon la revendication 10, **caractérisé en ce que** les amines aromatiques primaires et secondaires du composant C sont choisies dans le groupe formé par la N,N-diméthyl-p-phénylènediamine, la N,N-diéthyl-p-phénylènediamine, la N-(2-hydroxyéthyl)-N-éthyl-p-phénylènediamine, la N,N-bis-(2-hydroxyéthyl)-p-phénylènediamine, la N-(2-méthoxyéthyl)-p-phénylènediamine, la 2,3-dichloro-p-phénylènediamine, la 2,4-dichloro-p-phénylènediamine, la 2,5-dichloro-p-phénylènediamine, la 2-chloro-p-phénylènediamine, la 2,5-dihydroxy-4-morpholinoaniline, le 2-aminophénol, le 3-aminophénol, le 4-aminophénol, le 2-aminométhyl-4-aminophénol, le 2-hydroxyméthyl-4-aminophénol, la o-phénylènediamine, la m-phénylènediamine, la p-phénylènediamine, le 2,5-diaminotoluène, le 2,5-diaminophénol, le 2,5-diaminoanisole, le 2,5-diaminophénéthol, le 4-amino-3-méthylphénol, le 2-(2,5-diaminophényl)-éthanol, le 2,4-diaminophénoxyéthanol, le 2-(2,5-diaminophénoxy)-éthanol, le 3-amino-4-(2-hydroxyéthyloxy)phénol, le 3,4-méthylènedioxyphénol, la 3,4-méthylènedioxyaniline, le 3-amino-2,4-dichlorophénol, le 4-méthylaminophénol, le 2-méthyl-5-aminophénol, le 3-méthyl-4-aminophénol, le 2-méthyl-5-(2-hydroxyéthylamino)phénol, le 3-amino-2-chloro-6-méthylphénol, le 2-méthyl-5-amino-4-chlorophénol, le 5-(2-hydroxyéthylamino)-4-méthoxy-2-méthylphénol, le 4-amino-2-hydroxyméthylphénol, le 2-(diéthylaminométhyl)-4-aminophénol, le 4-amino-1-hydroxy-2-(2-hydroxyéthylaminométhyl)-benzène, le 1-hydroxy-2-amino-5-méthyl-benzène, le 1-hydroxy-2-amino-6-méthyl-benzène, le 2-amino-5-acétamido-phénol, le 1,3-diméthyl-2,5-diaminobenzène, le 5-(3-hydroxypropylamino-)2-méthylphénol, le 5-amino-4-méthoxy-2-méthylphénol, le N,N-diméthyl-3-aminophénol, le N-cyclopentyl-3-aminophénol, le 5-amino-4-fluoro-2-méthylphénol, le 2,4-diamino-5-fluorotoluène, le 2,4-diamino-5-(2-hydroxyéthoxy)toluène, le 2,4-diamino-5-méthylphénetol, le 3,5-diamino-2-méthoxy-1-méthylbenzène, le 2-amino-4-(2-hydroxyéthylamino)-anisole, le 2,6-bis-(2-hydroxyéthylamino)-1-méthylbenzène, le 1,3-diamino-2,4-diméthoxybenzène, le 3,5-diamino-2-méthoxy-toluène, l'acide 2-aminobenzoïque, l'acide 3-aminobenzoïque, l'acide 4-aminobenzoïque, l'acide 2-aminophénylacétique, l'acide 3-aminophénylacétique, l'acide 4-aminophénylacétique, l'acide 2,3-diaminobenzoïque, l'acide 2,4-diaminobenzoïque, l'acide 2,5-diaminobenzoïque, l'acide 3,4-diaminobenzoïque, l'acide 3,5-diaminobenzoïque, l'acide 4-aminosalicylique, l'acide 5-aminosalicylique, l'acide 3-amino-4-hydroxy-benzoïque, l'acide 4-amino-3-hydroxy-benzoïque, l'acide 2-aminobenzènesulfonique, l'acide 3-aminobenzènesulfonique, l'acide 4-aminobenzènesulfonique, l'acide 3-amino-4-hydroxybenzènesulfonique, l'acide 4-amino-3-hydroxy-naphtalène-1-sulfonique, l'acide 6-amino-7-hydroxynaphtalène-2-sulfonique, l'acide 7-amino-4-hydroxynaphtalène-2-sulfonique, l'acide 4-amino-5-hydroxynaphtalène-2,7-disulfonique, l'acide 3-amino-2-naphtoïque, l'acide 3-aminophtalique, l'acide 5-aminoisophtalique, le 1,3,5-triaminobenzène, 1,2,4-triaminobenzène, le 1,2,4,5-tetraaminobenzène, le 2,4,5-triaminophénol, le pentaaminobenzène, le hexaaminobenzène, le 2,4,6-triaminoresorcinol, le 4,5-diaminopyrocatéchol, le 4,6-diaminopyrogallol, la 1-(2-hydroxy-5-aminobenzyl)-2-imidazolidinone, le 4-amino-2-((4-[(5-amino-2-hydroxyphényl)methyl]-piperazinyl)méthyl)phénol, le 3,5-diamino-4-hydroxypyrocatéchol, le 1,4-bis-(4-aminophényl)-1,4-diazacycloheptane, les nitriles aromatiques, comme le 2-amino-4-hydroxybenzonitrile, le 4-amino-2-hydroxybenzonitrile, le 4-aminobenzonitrile, le 2,4-diaminobenzonitrile, les composés amino contenant des groupes nitro, comme la 3-amino-6-méthylamino-2-nitro-pyridine, l'acide picramique, le chlorure de [8-[(4-amino-2-nitrophényl)-azo]-7-hydroxy-napht-2-yl]-triméthylammonium, le chlorure de [8-((4-amino-3-nitrophényl)-azo)-7-hydroxy-napht-2-yl]-triméthylammonium (Basic Brown 17), le 1-hydroxy-2-amino-4,6-dinitrobenzène, le 1-amino-2-nitro-4-[bis-(2-hydroxyéthyl)amino]-benzène, le 1--amino-2-[(2-hydroxyéthyl)amino]-5-nitrobenzène (HC Yellow N° 5), le 1-amino-2-nitro-4[(2-hydroxyéthyl)amino]-benzène (HC Red N° 7), la 2-chloro-5-nitro-N-2-hydroxyéthyl-1,4-phénylènediamine, le 1-(2-hydroxyéthyl)-amino-2-nitro-4-amino-benzène (HC Red N° 3), le 4-amino-3-nitrophénol, le 4-amino-2-nitrophénol, la 6-nitro-o-toluidine, le 1-amino-3-méthyl-4-[(2-hydroxyéthyl)amino]-6-nitrobenzène (HC Violet N° 1), le 1-amino-2-nitro-4[(2,3-dihydroxypropyl)amino]-5-chloro-benzène (HC Red N° 10), l'acide 2-(4-amino-2-nitroanilino)-benzoïque, la 6-nitro-2,5-diaminopyridine, le 2-amino-6-chloro-4-nitrophénol, l'acide 1-amino-2-(3-nitrophénylazo)-7-phényl-azo-8-naphtol-3,6-disulfonique, sel disodique (Acid blue N° 29), l'acide 1-amino-2-(2-hydroxy-4-nitrophénylazo)-8-naphtol-3,6-disulfonique, sel disodique (Palatinchrome green), l'acide 1-amino-2-(3-chloro-2-hydroxy-5-nitro-phénylazo)-8-naphtol-3,6-disulfonique, sel disodique (Gallion), l'acide 4-amino-4'-nitrostilbène-2,2'-disulfonique, sel disodique, le 2,4-diamino-3',5'-dinitro-2'-hydroxy-5-méthylazobenzène (Mordant brown 4), l'acide 4'-amino-4-nitrodiphénylamine-2-sulfonique, l'acide 4'-amino-3'-nitrobenzophénone-2-carboxylique, le 1-amino-4-nitro-2-(2-nitrobenzylidèneamino)-benzène, la 2-[2-(diéthylamino)éthylamino]-5-nitroaniline, l'acide 3-amino-4-hydroxy-5-nitrobenzènesulfonique, le 3-amino-3'-nitrobiphényle, le 3-amino-4-nitro-acénaphtène, le 2-amino-1-nitronaphtalène, le 5-amino-6-nitrobenzo-1,3-dioxol, l'aniline, en particulier les anilines contenant des groupes nitro, comme la 4-nitroaniline, la 2-nitroaniline, le 1,4-diamino-2-nitrobenzène, le 1,2-diamino-4-nitrobenzène, le 1-amino-2-méthyl-6-nitrobenzène, la 4-nitro-1,3-phénylènediamine, le 2-nitro-4-amino-1-(2-hydroxyéthylamino)benzène, le 2-nitro-1-amino-4-[bis-(2-hydroxyéthyl)-amino]-benzène, l'acide 4-amino-2-nitrodiphénylamine-2'-carboxylique, le 1-amino-5-chloro-4-(2-hydroxyéthylamino)-2-nitrobenzène, les anilines aromatiques ou phénols avec un radical aromatique supplémentaire, tels qu'ils sont représentés dans la formule VI 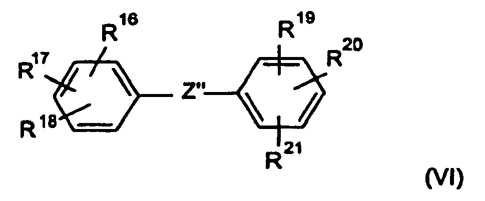 dans laquelle
• R¹⁶ représente un groupe hydroxy ou amino, qui peut être substitué par des groupes alkyle en C₁ à C₆, hydroxyalkyle en C₁ à C₆, alcoxy en C₁ à C₆, ou (alcoxy en C₁ à C₆)-(alkyle en C₁ à C₆),
• R¹⁷, R¹⁸, R¹⁹, R²⁰ et R²¹ représentent indépendamment l'un de l'autre un atome d'hydrogène, un groupe hydroxy ou amino, qui peut être substitué par les groupes alkyle en C₁ à C₆, hydroxyalkyle en C₁ à C₆, alcoxy en C₁ à C₆, aminoalkyle en C₁ à C₆, ou (alcoxy en C₁ à C₆)-(alkyle en C₁ à C₆), et
• Z" représente une liaison directe, une chaîne carbonée saturée ou insaturée, substituée éventuellement par des groupes hydroxy ayant de 1 à 4 atomes de carbone, un groupe carbonyle, sulfonyle, ou imino, un atome d'oxygène ou de soufre, ou un groupe de formule VII
**-Q'-(CH₂-Q-CH₂-Q")ₒ-** **(VII)**
dans laquelle
• Q représente une liaison directe, un groupe CH₂ ou CHOH,
• Q' et Q" représentent indépendamment l'un de l'autre un atome d'oxygène, un groupe NR²², dans lequel R²² représente un atome d'hydrogène, un groupe alkyle en C₁ à C₆, ou un groupe hydroxyalkyle en C₁ à C₆, les deux groupes pouvant également former conjointement avec le restant de la molécule un cycle de 5, 6, ou 7 maillons, le groupe O-(CH₂)ₚ-NH ou NH-(CH₂)_{p'}-O, dans lesquels p et p' valent 2 ou 3, et
• o désigne un nombre de 1 à 4,
comme par exemple le 4,4'-diaminostilbène et son chlorhydrate, le sel monosodique ou disodique de l'acide 4,4'-diaminostilbène-2,2'-disulfonique, le 4-amino-4'-diméthylaminostilbène et son chlorhydrate, le 4,4'-diaminodiphénylméthane, le sulfure de 4,4'-diaminodiphényle, le sulfoxyde de 4,4'-diaminodiphényle, la 4,4'-diaminodiphénylamine, l'acide 4,4'-diaminodiphénylamine-2-sulfonique, la 4,4'-diaminobenzophénone, le 4,4'-diaminodiphényléther, le 3,3',4,4'-tétraaminodiphényle, la 3,3',4,4'-tétraaminobenzophénone, le 1,3-bis-(2,4-diaminophénoxy)-propane, le 1,8-bis-(2,5-diaminophénoxy)-3,6-dioxaoctane, le 1,3-bis-(4-aminophénylamino)propane, le 1,3-bis-(4-aminophénylamino)-2-propanol, le 1,3-bis-[N-(4-aminophényl)-2-hydroxyéthylamino]-2-propanol, la N,N-bis-[2-(4-aminophénoxy)-éthyl]-méthylamine, la N-phényl-1,4-phénylènediamine, et le bis-(5-amino-2-hydroxyphényl)-méthane.

13. Agent selon la revendication 10, **caractérisé en ce que** les composés hydroxyaromatiques du composant C sont choisis dans le groupe formé par le 2-, le 4-, le 5-méthylrésorcinol, le 2,5-diméthylrésorcinol, le résorcinol, le 3-méthoxyphénol, le pyrocatéchol, l'hydroquinone, le pyrogallol, la phloroglucine, l'hydroxyhydroquinone, le 2-, le 3-, le 4-méthoxy-, le 3-diméthylamino-, le 2-(2-hydroxyéthyl)-, le 3,4-méthylène-dioxyphénol, l'acide 2,4-, l'acide 3,4-dihydroxy-benzoïque, -phénylacétique, l'acide gallique, l'acide 2,4,6-trihydroxybenzoïque, la 2,4,6-trihydroxyacétophénone, le 2-, le 4-chlororésorcinol, le 1-naphtol, le 1,5-, 2,3-, le 2,7-dihydroxynaphtalène, l'acide 6-diméthylamino-4-hydroxy-2-naphtalènesulfonique et l'acide 3,6-dihydroxy-2,7-naphtalènesulfonique.

14. Agent selon la revendication 10, **caractérisé en ce que** les composés hétérocycliques azotés du composant C sont choisi dans le groupe formé par la 2-aminopyridine, la 3-aminopyridine, la 4-aminopyridine, la 2-amino-3-hydroxypyridine, la 2,6-diaminopyridine, la 2,5-diaminopyridine, la 2-(aminoéthylamino)-5-aminopyridine, la 2,3-diaminopyridine, la 2-diméthylamino-5-aminopyridine, la 2-méthylamino-3-amino-6-méthoxypyridine, la 2,3-diamino-6-méthoxypyridine, la 2,6-diméthoxy-3,5-diaminopyridine, la 2,4,5-triaminopyridine, la 2,6-dihydroxy-3,4-diméthylpyridine, la N-[2-(2,4-diaminophényl)aminoéthyl]-N-(5-amino-2-pyridyl)-amine, la N-[2-(4-amino-phényl)-aminoéthyl]-N-(5-amino-2-pyridyl)-amine, la 2,4-dihydroxy-5,6-diaminopyrimidine, la 4,5,6-triaminopyrimidine, la 4-hydroxy-2,5,6-triaminopyrimidine, la 2-hydroxy-4,5,6-triaminopyrimidine, la 2,4,5,6-tétraaminopyrimidine, la 2-méthylamino-4,5,6-triaminopyrimidine, la 2,4-diaminopyrimidine, la 4,5-diaminopyrimidine, la 2-amino-4-méthoxy-6méthyl-pyrimidine, le 3,5-diaminopyrazole, le 3,5-diamino-1,2,4-triazole, le 3-aminopyrazole, le 3-amino-5-hydroxypyrazole, le 1-phényl-4,5-diaminopyrazole, le 1-(2-hydroxyéthyl)-4,5-diaminopyrazole, le 1-phényl-3-méthyl-4,5-diaminopyrazole, la 4-amino-2,3-diméthyl-1-phényl-3-pyrazolin-5-one (4-amino-antipyrine), la 1-phényl-3-méthylpyrazol-5-one, la 2-aminoquinoléine, la 3-aminoquinoléine, la 8-aminoquinoléine, la 4-aminoquinaldine, l'acide 2-aminonicotinique, l'acide 6-aminonicotinique, la 5-aminoisoquinoléine, le 5-aminoindazole, le 6-aminoindazole, le 5-aminobenzimidazole, le 7-aminobenzimidazole, le 5-aminobenzothiazole, le 7-aminobenzothiazole, la 2,5-dihydroxy-4-morpholino-aniline ainsi que les dérivés d'indol et d'indoline, comme le 4-aminoindol, le 5-aminoindol, le 6-aminoindol, le 7-aminoindol, le 5,6-dihydroxyindol, la 5,6-dihydroxyindoline, la 4-hydroxyindoline, et les dérivés d'hydroxypyrimidine et les sels acceptables sur le plan physiologique des composés précités.

15. Agent selon l'une quelconque des revendications 1 à 14, **caractérisé en ce que** les composés de formule I, les composés du composant B et les composés du composant C sont contenus respectivement en une quantité de 0,03 à 65 mmoles, en particulier de 1 à 40 mmoles, par rapport à 100 g de la teinture totale.

16. Agent selon l'une quelconque des revendications 1 à 15, **caractérisé en ce que**, il contient au moins un produit de réaction RP selon la formule VIII 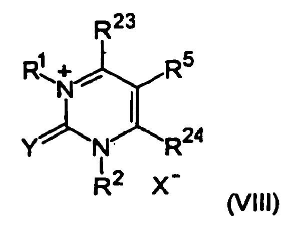 dans laquelle
• R¹, R², R⁵, Y et X⁻ sont définis selon la revendication 1,
• R²³ et R²⁴ représentent indépendamment l'un de l'autre un atome d'hydrogène, un groupe alkyle en C₁ à C₆, un groupe selon la formule IX, 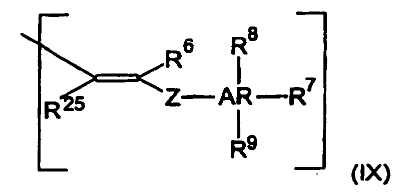 dans laquelle
• R⁶, R⁷, R⁸, R⁹, AR et Z sont définis selon la revendication 7,
• R²⁵ représente un atome d'hydrogène ou un groupe alkyle en C₁ à C₅,
à la condition qu'au moins l'un des radicaux R²³ et R²⁴ représente un groupe selon la formule IX.

17. Agent selon la revendication 16, **caractérisé en ce que** AR selon la formule IX représente un groupe benzène ou naphtalène.

18. Agent selon l'une quelconque des revendications 16 ou 17, **caractérisé en ce que** Z selon la formule IX représente une liaison directe ou un groupe vinylène.

19. Agent selon l'une quelconque des revendications 16 à 18, **caractérisé en ce que** R²⁵ selon la formule IX représente un atome d'hydrogène.

20. Agent selon l'une quelconque des revendications 16 à 19, **caractérisé en ce que** R⁷, R⁸, et R⁹ selon la formule IX représentent indépendamment l'un de l'autre un atome d'hydrogène, un atome d'halogène, un groupe alkyle en C₁ à C₆, un groupe hydroxy, un groupe alcoxy en C₁ à C₆, un groupe amino, un groupe dialkylamino en C₁ à C₆, un groupe di(hydroxyalkyle en C₂ à C₆)amino, un groupe di((alcoxy en C₁ à C₆)-(alkyle en C₁ à C₆))amino, un groupe hydroxyalkyloxy en C₁ à C₆, un groupe sulfonyle, un groupe carboxyle, un groupe sulfonamide, un groupe carbamoyle, un groupe acyle en C₂ à C₆, un groupe acétyle, un groupe d'acide sulfonique, un groupe sulfonamido, ou un groupe nitro.

21. Agent selon l'une quelconque des revendications 16 à 20, **caractérisé en ce que**, le produit de réaction RP est contenu en une quantité de 0,03 à 65 mmoles, en particulier de 1 à 40 mmoles, par rapport à 100 g de la teinture totale.

22. Agent selon l'une quelconque des revendications 1 à 21, **caractérisé en ce qu'**il contient des renforçateurs de couleur choisis dans le groupe formé par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'arginine, l'histidine, la pyrrolidine, la proline, la pyrrolidone, l'acide pyrrolidone-5-carboxylique, le pyrazole, le 1,2,4-triazole, la pipérazidine ou leurs mélanges quelconques.

23. Agent selon l'une quelconque des revendications 1 à 22, **caractérisé en ce qu'**il contient au moins un colorant montant directement sur les fibres, de préférence en une quantité de 0,01 à 20% en poids, par rapport à la teinture totale.

24. Agent selon l'une quelconque des revendications 1 à 23, **caractérisé en ce qu'**il contient au moins un composant révélateur et éventuellement au moins un composant de couplage en tant que précurseur de colorant par oxydation.

25. Agent selon l'une quelconque des revendications 1 à 24, **caractérisé en ce que** des sels d'ammonium ou métalliques choisis dans le groupe formé par les formiates, les carbonates, les halogénures, les sulfates, les butyrates, les valériates, les caproates, les acétates, les lactates, les glycolates, les tartrates, les citrates, les gluconates, les propionates, les phosphates et les phosphonates de métaux alcalins, comme le potassium, le sodium ou le lithium, de métaux alcalinoterreux comme le magnésium, le calcium, le strontium ou le baryum ou d'aluminium, de manganèse, de fer, de cobalt, de cuivre ou de zinc, sont ajoutés.

26. Agent selon l'une quelconque des revendications 1 à 25, **caractérisé en ce qu'**il contient un agent d'oxydation, en particulier H₂O₂, en une quantité de 0,01 à 6% en poids, par rapport à la solution d'application.

27. Agent selon l'une quelconque des revendications 1 à 26, **caractérisé en ce qu'**il contient des tensioactifs anioniques, zwitterioniques ou non ioniques.

28. Utilisation d'au moins un composé selon la formule I selon la revendication 1, en combinaison avec au moins un composé du composant B selon la revendication 1, en tant que composant colorant dans des teintures capillaires.

29. Procédé pour teindre des fibres kératiniques, en particulier les cheveux humains, dans lequel une teinture, selon la revendication 1, ainsi que des ingrédients cosmétiques courants, sont appliqués sur les fibres kératiniques, laissés pendant quelque temps, habituellement pendant environ 15 à 30 minutes, sur la fibre, puis éliminés de nouveau par rinçage ou par lavage avec un shampoing.

30. Procédé selon la revendication 29, **caractérisé en ce que** dans un procédé en deux étapes, le composant B est appliqué sur les fibres kératiniques avant ou après application du composé selon la formule I, le mélange obtenu est laissé sur le cheveu pendant quelque temps, habituellement pendant environ 15 à 30 minutes, puis éliminé de nouveau par rinçage ou par lavage avec un shampoing.

31. Procédé selon l'une quelconque des revendications 29 ou 30, **caractérisé en ce que** les fibres kératiniques avant application d'une teinture selon la revendication 1, ont été blondies avec un agent blondissant dans le cadre d'un prétraitement, ou colorées avec une teinture par oxydation.

32. Utilisation d'au moins un dérivé 1,2-dihydro-pyrimidinium selon la formule I et/ou sa forme énamine, 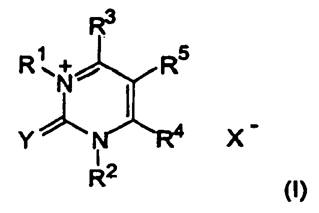 dans laquelle R¹, R², R³, R⁴, R⁵, Y et X⁻ sont définis comme dans la revendication 1,
en combinaison avec des composés carbonyle réactifs (composant B), pour nuancer des colorations par oxydation de fibres kératiniques, en particulier de cheveux humains.

33. Utilisation d'au moins un dérivé 1,2-dihydro-pyrimidinium selon la formule I et/ou sa forme énamine, 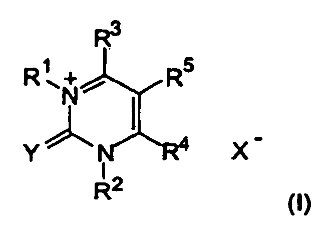 dans laquelle R¹, R², R³, R⁴, R⁵, Y et X- sont définis comme dans la revendication 1
en combinaison avec au moins un composé carbonyle réactif (composant B), pour rafraîchir les couleurs de fibres kératiniques teintes avec des teintures par oxydation.
